# DEMANDE DE BREVET EUROPEEN

(11) **EP 2 801 568 A1**
(43) Date de publication de la demande: **12.11.2014**
(21) Numéro de dépôt: 14179708.4
(22) Date de dépôt: 28.06.2010
(51) Int. Cl.: C07D 209/10, C07D 213/30, C07D 215/14, C07D 261/20, C07D 471/04, C07D 295/26, A61K 31/4965, A61P 35/00, A61P 25/00, A61P 29/00

(54) **Composés benzène-sulfonamides, leur procédé de synthèse et leur utilisation en médecine ainsi qu'en cosmétique**

(30) Priorité: 30.06.2009 FR 0954460
(62) Demande divisionnaire de: 10745348.2
(71) Demandeur: Galderma Research & Development, 06410 Biot (FR)
(72) Inventeur: Clary, Laurence, F-06480 La Colle Sur Loup (FR); Chambon, Sandrine, F-06110 Le Cannet (FR); Chantalat, Laurent, F-06130 Grasse (FR); Rosignoli, Carine, F-06250 Mougins le Haut (FR); Roye, Olivier, F-83440 Fayence (FR); Pascal, Jean-Claude, F-06100 Nice (FR); Schuppli, Marlène, F-06650 Le Rouret (FR)
(74) Mandataire: Jansen, Cornelis Marinus

(57) **Abrégé**

La présente invention se rapporte à de nouveaux composés benzène-sulfonamides de la structure répondant à la formule générale (I) et à leur utilisation dans des compositions pharmaceutiques destinées à un usage en médecine humaine ou vétérinaire pour le traitement de maladies inflammatoires ou désordres impliquant une production de TNFα. Les composés peuvent aussi être utilisés dans des compositions cosmétiques.

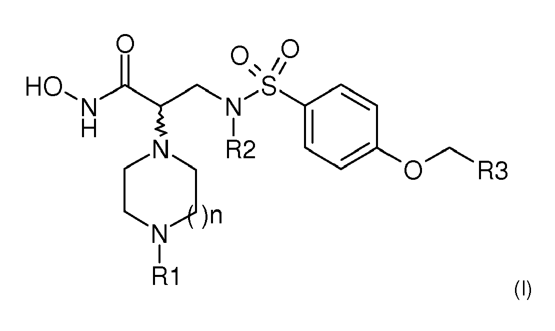

## Description

### DOMAINE TECHNIQUE

La présente invention se rapporte à de nouveaux composés benzène-sulfonamides répondant à la formule générale (I) suivante : 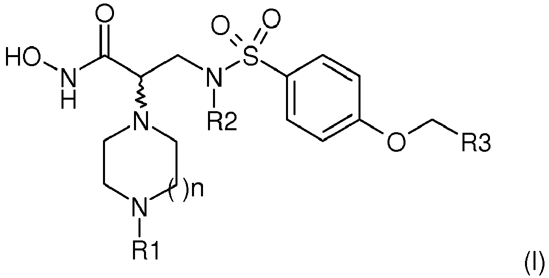 ainsi qu'à leur procédé de synthèse et à leur utilisation dans des compositions pharmaceutiques destinées à un usage en médecine humaine ou vétérinaire.
Les composés de la présente invention agissent comme inhibiteurs de l'enzyme de conversion du TNFα encore appelée TACE. Ils sont de ce fait utiles dans le traitement des maladies pour lesquelles diminuer la production de TNFα est d'un grand intérêt.
La présente invention se rapporte également à l'utilisation des composés répondant à la formule générale (I) dans des compositions cosmétiques.

### ETAT ANTERIEUR DE LA TECHNIQUE

Les adamalysines (« ADAM » ou A Disintegrin and Metalloproteinase) sont une sous famille des enzymes métalloendopeptidases à zinc. Leur ectodomaine comporte un domaine protéasique dont l'activation dépend du zinc, un domaine disintégrine et un domaine riche en cystéines. A ce jour, au moins 30 ADAMs différentes ont été identifiées dont la première caractérisée a été ADAM17 encore appelée TACE (TNFα converting enzyme) [Gueydan C et al. Med.Sci 1997, 13, 83-88; Black R.A et al. Nature 1997, 385 :729-733; Moss et al. Nature 1997, 385 :733-736]. L'ARNm de TACE est présent dans de nombreux tissus et plus particulièrement au niveau des monocytes, des macrophages, des lymphocytes T mais aussi au niveau des kératinocytes par exemple.
TACE est responsable de la coupure du pro-TNFα, protéine membranaire de 26 kDa, pour conduire à la libération du TNFα soluble, protéine de 17kDa, biologiquement active [Schlondorff et al. Biochem J. 2000, 347, 131-138]. Le TNFα soluble libéré par la cellule est capable d'agir sur des sites très éloignés du site de synthèse.

Le TNFα est impliqué dans un grand nombre de processus biologiques pro-inflammatoires [Aggarwal et al, Eur. Cytokine Netw., 1996, 7 : 93-124]. Plusieurs études pharmacologiques et cliniques ont montré de façon évidente que bloquer les effets du TNFα avec des anticorps spécifiques ou des biologiques anti-TNFα (Etanercept, Adalimumab, Infliximab) était bénéfique dans le traitement de maladies autoimmunes comme l'arthrite rhumatoide [Feldman et al. Lancet, 1994, 344, 1105), le diabète mélitus non insulino dépendant [Lohmander L.S et al. Arthritis Rheum, 1993, 36, 1214-1222], la maladie de Crohn's [MacDonald et al. Clin. Exp. Immunol. 1990, 81, 301].
Le TNFα joue également un rôle fondamental au cours du phénomène inflammatoire déclenché dans les lésions de psoriasis. Les taux sériques de TNFα sont élevés chez les patients psoriasiques [Mussi A et al. J. Biol. Regul. Homeost Agents, 1997, 11, 115-118]; les taux de TNFα sont également élevés dans les plaques même de psoriasis [Bonifati C. et al. Clin. Exp. Dermatol., 1994, 19, 383-387]. Les cellules clés de la physiopathologie du psoriasis sont les kératinocytes, les cellules dendritiques et certains lymphocytes T. L'interaction entre ces familles de cellules conduit à une cascade inflammatoire menant aux lésions caractéristiques du psoriasis avec libération de TNFα [Kupper TS, N. Engl. J. Med, 2003, 349, 1987-1990]. Des études cliniques pour le traitement de psoriasis en plaques modéré à sévère par les anti-TNFα biologiques (Etanercept, Adalimumab, Infliximab) ont démontré leur efficacité à la fois sur les lésions psoriasiques et sur la qualité de vie des patients [Ortonne JP, Annales de dermatologie et de vénéreologie, 2005, 132 (8-9 pt2), 4S6-9 et 2005, 132, 9S01-9S70].

Ainsi, les composés qui inhibent la production de TNFα sont d'un grand intérêt pour le traitement des maladies inflammatoires et des maladies impliquant une libération de TNFα.

### EXPOSE DE L'INVENTION

Notre invention décrit donc de nouvelles molécules qui inhibent l'enzyme TACE (TNFα converting enzyme) et de ce fait, inhibent la sécrétion de TNFα soluble (forme active du TNFα) par les cellules. Ces nouvelles molécules sont donc des principes actifs potentiels pour le traitement des pathologies faisant intervenir une diminution ou une inhibition de la production de TNFα.
A titre d'illustration, et de façon non limitative, ces pathologies sont par exemple le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'attaque ischémique, le rejet de greffe, le cancer, l'athérosclérose, l'obésité, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD), l'inflammation occulaire, les maladies inflammatoires de la peau, le psoriasis, la dermatite atopique et le rhumatisme psoriasique.

Ces molécules sont également des principes actifs potentiels pour le traitement de pathologies neurologiques à caractère inflammatoire pour lesquelles diminuer la production de TNFα serait d'un grand intérêt. Ces pathologies listées ci-après de façon non limitative sont par exemple la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'oedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale ;

Une grande variété d'inhibiteurs de TACE est déjà connue comme indiqué ci-dessous. Toutefois, un grand nombre de ces inhibiteurs n'agit pas sélectivement sur l'enzyme TACE par rapport à d'autres enzymes de la famille des ADAMs et/ou de matrix métalloprotéinases (MMPs).

Or, l'inhibition non sélective de ces familles d'enzymes induit des effets secondaires indésirables observés *In Vivo.* Par exemple, l'inhibition de MMP-1 (collagenase-1) a été associée aux problèmes de toxicité musculo-squelettique.
Comme inhibiteur non sélectif, on peut citer également l'Apratastat, inhibiteur connu et testé cliniquement en phase 2 pour le traitement de l'arthrite rhumatoïde (Curr Opin Investig Drugs. 2006 Nov;7(11),1014-1019). Cet inhibiteur n'est pas sélectif de l'enzyme TACE par rapport à certaines MMPs (WO 00/44709; page 251, table 10, exemple 61).

D'autres inhibiteurs de TACE également connus et faisant partie de la même famille que l'Apratastat, à savoir celle de dérivés benzène sulfonamides cycliques, ont été décrits dans WO 00/44709 et WO 97/18194. D'autres brevets (WO 96/00214, WO 97/22587) revendiquent des inhibiteurs de MMPs et/ou TACE pour lesquels la partie benzène sulfonamide est séparée de la fonction acide hydroxamique par un seul atome de carbone. Des publications décrivant ce type d'inhibiteurs de MMPs de façon plus large sont aussi celle de MacPherson et al. J. Med. Chem. 1997,40, 2525 et celle de Tamura et al. J. Med. Chem. 1998, 41, 640. D'autres exemples d'inhibiteurs de MMPs/TACE pour lesquels la fonction sulfonamide est séparée de l'acide hydroxamique par un enchainement de deux atomes de carbones formant un cycle sont décrits dans les brevets WO 98/16503, WO 98/16506, WO 98/16514 et WO 98/16520. D'autres exemples d'inhibiteurs de MMPs pour lesquels la fonction sulfonamide est séparée de l'acide hydroxamique par un enchainement de deux atomes de carbones sont également décrits dans WO 2008/045671.

Or, la demanderesse a maintenant découvert de manière inattendue et surprenante que de nouveaux composés de formule générale (I) présentent une très bonne activité inhibitrice de TACE et en particulier, inhibent l'enzyme TACE de façon sélective par rapport à d'autres ADAMs et MMPs.

Ainsi, la présente invention concerne des composés de formule générale (I) suivante : 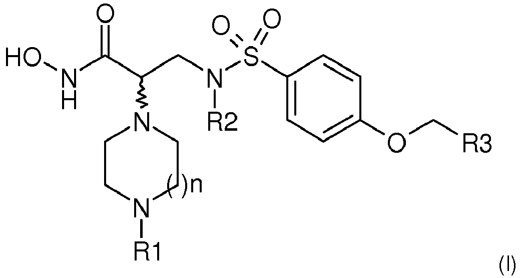 dans laquelle :
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical-C(O)-R₄, un radical -SO₂-R₄, un radical C(O)OR4, R₄ ayant les significations données ci-après;
R₂ est un atome d'hydrogène ou un radical alkyle inférieur.
R₃ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué, un radical hétérocyclique, un radical hétérocyclique substitué, un radical cycloalkyle, un radical cycloalkyle substitué, un radical hétéroaryle, un radical hétéroaryle substitué, un radical hétéroaralkyle, un radical hétéroaralkyle substitué;
R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
n peut prendre les valeurs de 0, 1, 2 ou 3;
ainsi que les sels d'addition des composés de formule générale (I) avec un acide pharmaceutiquement acceptable, les sels d'addition des composés de formule générale (I) avec une base pharmaceutiquement acceptable et les énantiomères des composés de formule générale (I).

Parmi les sels d'addition des composés de formule générale (I) avec un acide pharmaceutiquement acceptable, on peut citer de préférence les sels avec un acide organique ou avec un acide inorganique.
Les acides inorganiques appropriés sont par exemple les acides halohydriques comme l'acide chlorhydrique, l'acide bromhydrique, l'acide sulfurique, l'acide nitrique et l'acide phosphorique.

Les acides organiques appropriés sont par exemple l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide methanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.

Parmi les sels d'addition des composés de formule générale (I) avec une base pharmaceutiquement acceptable, on peut citer de préférence les sels avec une base organique ou avec une base inorganique.
Les bases inorganiques sont par exemple l'hydroxyde de potassium, l'hydroxyde de sodium, l'hydroxyde de lithium ou l'hydroxyde de calcium.
Les bases organiques appropriés comprennent les amines et les amino-acides. Parmi les amines, on peut citer par exemple les amines primaires, secondaires ou tertiaires aliphatiques ou aromatiques comme la methylamine, l'ethylamine, l'éthanolamine, la propylamine, l'isopropylamine, les 4 isomères de la butylamine, la diméthylamine, diethylamine, la diethanolamine, la dipropylamine, la diisopropylamine, la di n-butylamine, la pyrrolidine, la piperidine, la morpholine, la diethanol phenylamine, la trimethylamine, la triethylamine, la tripropylamine, la quinuclidine, la pyridine, la quinoline ou l'isoquinoline.
Parmi les amino-acides, on peut citer par exemple la lysine, l'arginine et l'ornithine.

Selon la présente invention, on désigne par radical alkyle inférieur une chaîne hydrocarbonée saturée, linéaire ou ramifiée, comprenant de 1 à 4 atomes de carbone.

Selon la présente invention, on désigne par radical alkyle une chaîne hydrocarbonée saturée, linéaire ou ramifiée, comprenant de 1 à 10 atomes de carbone.

Selon la présente invention, on désigne par radical alkenyle une chaîne hydrocarbonée insaturée, linéaire ou ramifiée comprenant de 2 à 10 atomes de carbone et comprenant une ou plusieurs doubles liaisons.

Selon la présente invention, on désigne par radical alkynyle une chaîne hydrocarbonée insaturée, linéaire ou ramifiée comprenant de 2 à 10 atomes de carbone et comprenant une ou plusieurs triples liaisons.

Selon la présente invention, on désigne par radical alkyle substitué une chaîne hydrocarbonée saturée, linéaire ou ramifiée, comprenant de 1 à 10 atomes de carbone et substitué par un ou plusieurs radicaux choisis parmi un atome d'halogène, un radical alkoxyle, un radical hydroxyle.

Selon la présente invention, on désigne par radical alkenyle substitué une chaîne hydrocarbonée insaturée, linéaire ou ramifiée, comprenant de 2 à 10 atomes de carbone, comprenant une ou plusieurs doubles liaisons et substituée par un ou plusieurs radicaux choisis parmi un atome d'halogène, un radical alkoxyle et un radical hydroxyle.

Selon la présente invention, on désigne par radical alkynyle substitué une chaîne hydrocarbonée insaturée, linéaire ou ramifiée, comprenant de 2 à 10 atomes de carbone, comprenant une ou plusieurs triples liaisons et substituée par un ou plusieurs radicaux choisis parmi un atome d'halogène, un radical alkoxyle et un radical hydroxyle.

Selon la présente invention, on désigne par cycloalkyle une chaine hydrocarbonée, saturée, cyclique comprenant de 3 à 7 atomes de carbones.

Selon la présente invention, on désigne par cycloalkyle substitué une chaine hydrocarbonée, saturée, cyclique comprenant de 3 à 7 atomes de carbones et substituée par un ou plusieurs radicaux choisis parmi un atome d'halogène, un radical alkoxyle et un radical hydroxyle.

Selon la présente invention, on désigne par radical aryle, un cycle hydrocarboné aromatique ou deux cycles fusionnés hydrocarbonés aromatiques.
Les radicaux aryles préférés sont choisis parmi les radicaux phenyle et naphtyle.

Selon la présente invention, on désigne par radical aryle substitué, un cycle ou deux cycles fusionnés hydrocarboné(s) aromatique(s) substitué(s) par un ou plusieurs groupes d'atomes choisis parmi un alkyle, un alkoxyle, un aryle, un halogène, un hydroxyle, un cyano, un trifluorométhyle et un nitro.

Selon la présente invention, on désigne par radical aralkyle, un alkyle substitué par un aryle.

Selon la présente invention, on désigne par radical aralkyle substitué, un alkyle substitué par un aryle substitué.

Selon la présente invention, on désigne par radical hétérocyclique une chaîne hydrocarbonée cyclique ou polycyclique, saturée ou insaturée, comprenant un ou plusieurs hétéroatomes choisis parmi O, S et N.

Selon la présente invention, on désigne par radical hétérocyclique substitué, un radical hétérocyclique substitué par un ou plusieurs groupes d'atomes choisis parmi un alkyle, un alkoxyle, un halogène, un hydroxyle, un cyano, un trifluorométhyle et un nitro.

Selon la présente invention, on désigne par radical hétéroaryle, un radical hétérocyclique aromatique c'est-à-dire une chaîne hydrocarbonée cyclique ou polycyclique, aromatique, comprenant un ou plusieurs hétéroatomes choisis parmi O, S et N.

Selon la présente invention, on désigne par radical hétéroaryle substitué, un radical hétéroaryle substitué par un ou plusieurs groupes d'atomes choisis par exemple parmi un alkyle, un alkoxy, un aryle, un aryle substitué, un halogène, un hydroxy, un cyano, un trifluorométhyle et un nitro.

Selon la présente invention, on désigne par radical hétéroaralkyle, un radical alkyle substitué par un radical hétéroaryle.

Selon la présente invention, on désigne par radical hétéroaralkyle substitué, un radical hétéroaralkyle substitué par un ou plusieurs groupes d'atomes choisis parmi un alkyle, un alkoxyle, un halogène, un hydroxyle, un cyano, un trifluorométhyle et un nitro.

Selon la présente invention, on désigne par radical alkoxyle un atome d'oxygène substitué par un radical alkyle.

Selon la présente invention, on désigne par atome d'halogène un atome de fluor, de chlore, de brome ou d'iode.

Parmi les composés de formule générale (I) entrant dans le cadre de la présente invention, on peut notamment citer les composés suivants :
1) 3-[(4-but-2-ynyloxy-benzènesulfonyl)-méthyl-amino]-N-hydroxy-2-(4-méthanesulfonyl-pipérazin-1-yl)-propionamide
2) ((S)-3-(4-but-2-ynyloxy-benzènesulfonylamino)-N-hydroxy-2-(4-méthanesulfonyl-pipérazin-1-yl)-propionamide
3) ((S)-3-(4-benzyloxy-benzènesulfonylamino)-N-hydroxy-2-(4-méthanesulfonyl-pipérazin-1-yl)-propionamide
4) ((S)-3-[(4-benzyloxy-benzènesulfonyl)-méthyl-amino]-N-hydroxy-2-(4-méthanesulfonyl-pipérazin-1-yl)-propionamide
5) ((S)-N-hydroxy-2-(4-méthanesulfonyl-pipérazin-1-yl)-3-[4-(2-méthyl-quinolin-4-ylméthoxy)-benzènesulfonylamino]-propionamide
6) ((S)-N-hydroxy-2-(4-méthanesulfonyl-pipérazin-1-yl)-3-[4-(naphthalen-1-ylméthoxy)-benzènesulfonylamino]-propionamide
7) ((S)-N-hydroxy-2-(4-méthanesulfonyl-pipérazin-1-yl)-3-(4-propoxy-benzènesulfonylamino)-propionamide
8) ((S)-3-[4-(3-cyano-benzyloxy)-benzènesulfonylamino]-N-hydroxy-2-(4-méthanesulfonyl-pipérazin-1-yl)-propionamide
9) ((S)-3-[4-(4-cyano-benzyloxy)-benzènesulfonylamino]-N-hydroxy-2-(4-méthanesulfonyl-pipérazin-1-yl)-propionamide
10) 4-{(S)-1-hydroxycarbamoyl-2-[4-(2-méthyl-quinolin-4-ylméthoxy)-benzènesulfonylamino]-éthyl}-pipérazine-1-carboxylate de benzyle
11) (S)-N-hydroxy-2-(4-méthanesulfonyl-pipérazin-1-yl)-3-[4-(2-phényl-pyridin-4-ylméthoxy)-benzène sulfonylamino]-propionamide
12) ((R)-N-hydroxy-2-(4-méthanesulfonyl-pipérazin-1-yl)-3-[4-(2-méthyl-quinolin-4-ylméthoxy)-benzène sulfonylamino]-propionamide
13) ((S)-N-hydroxy-3-[4-(2-méthyl-quinolin-4-ylméthoxy)-benzènesulfonylamino]-2-pipérazin-1-yl-propionamide
14) Chlorhydrate de (S)-N-hydroxy-2-(4-méthanesulfonyl-pipérazin-1-yl)-3-[4-(2-méthyl-quinolin-4-ylméthoxy)-benzène sulfonylamino]-propionamide
15) Di trifluoro acétate de 3-{4-[(S)-2-hydroxycarbamoyl-2-(4-méthanesulfonyl-pipérazin-1-yl)-éthylsulfamoyl]-phénoxyméthyl}-2-méthyl-indole-1-carboxylate de terbutyle
16) ((S)-N-hydroxy-2-(4-méthanesulfonyl-pipérazin-1-yl)-3-[4-(quinolin-4-ylméthoxy)-benzènesulfonylamino]-propionamide
17) ((S)-2-(4-benzyl-pipérazin-1-yl)-N-hydroxy-3-[4-(2-méthyl-quinolin-4-ylméthoxy)-benzènesulfonylamino]-propionamide
18) ((S)-2-[4-(4-fluoro-benzyl)-pipérazin-1-yl]-N-hydroxy-3-[4-(2-méthyl-quinolin-4-ylméthoxy)-benzène sulfonylamino]-propionamide
19) ((S)-2-(4-éthyl-pipérazin-1-yl)-N-hydroxy-3-[4-(2-méthyl-quinolin-4-ylméthoxy)-benzènesulfonylamino]-propionamide
20) ((S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide
21) ((S)-N-hydroxy-2-[4-(4-méthyl-benzyl)-pipérazin-1-yl]-3-[4-(2-méthyl-quinolin-4-ylméthoxy)-benzène sulfonylamino]-propionamide
22) ((S)-3-[4-(benzo-isoxazol-3-ylméthoxy)-benzènesulfonylamino]-N-hydroxy-2-(4-méthanesulfonyl-pipérazin-1-yl)-propionamide
23) ((S)-N-hydroxy-2-(4-isobutyryl-pipérazin-1-yl)-3-[4-(2-méthyl-quinolin-4-ylméthoxy)-benzènesulfonylamino]-propionamide
24) ((S)-N-hydroxy-2-[4-(2-méthyl-propane-1-sulfonyl)-pipérazin-1-yl]-3-[4-(2-méthyl-quinolin-4-ylméthoxy)-benzènesulfonylamino]-propionamide
25) ((S)-N-hydroxy-2-(4-méthanesulfonyl-pipérazin-1-yl)-3-[4-(2-trifluorométhyl-pyrazolo[1,5-a]pyridin-3-ylméthoxy)-benzènesulfonylamino]-propionamide
26) ((S)-N-hydroxy-3-[4-(2-méthyl-quinolin-4-ylméthoxy)-benzènesulfonylamino]-2-[4-(propane-2-sulfonyl)-pipérazin-1-yl]-propionamide
27) ((S)-2-(4-benzyl-pipérazin-1-yl)-N-hydroxy -3-[4-(2-trifluorométhyl-pyrazolo[1,5-a]pyridin-3-ylméthoxy)-benzènesulfonylamino]-propionamide
28) ((S)-2-(4-acétyl-pipérazin-1-yl)-N-hydroxy-3-[4-(2-méthyl-quinolin-4-ylméthoxy)-benzènesulfonylamino]-propionamide
29) ((S)-N-hydroxy-2-(4-méthanesulfonyl-pipérazin-1-yl)-3-{propyl-[4-(quinolin-4-ylméthoxy)-benzènesulfonyl]-amino}-propionamide
30) ((S)-2-(4-benzènesulfonyl-pipérazin-1-yl)-N-hydroxy-3-[4-(pyrazolo[1,5-a]pyridin-3-ylméthoxy)-benzènesulfonylamino]-propionamide
31) (S)-2-(4-benzyl-pipérazin-1-yl)-N-hydroxy-3-[4-(1-méthyl-pipéridin-4-ylméthoxy)-benzènesulfonylamino]-propionamide
32) ((S)-2-[4-(4-fluoro-benzoyl)-pipérazin-1-yl]-N-hydroxy-3-[4-(3-m-tolyl-propoxy)-benzènesulfonylamino]-propionamide
33) ((S)-N-hydroxy-3-[4-(2-méthyl-naphthalen-1-ylméthoxy)-benzènesulfonylamino]-2-(4-propionyl-pipérazin-1-yl)-propionamide
34) ((S)-N-hydroxy-3-[4-(4-méthyl-pentyloxy)-benzènesulfonylamino]-2-(4-phénylacétyl-pipérazin-1-yl)-propionamide
35) ((S)-N-hydroxy-2-(4-méthanesulfonyl-pipérazin-1-yl)-3-[4-(2-méthyl-pyridin-4-ylméthoxy)-benzènesulfonylamino]-propionamide
36) ((S)-2-(3-acétyl-imidazolidin-1-yl)-N-hydroxy-3-[4-(2-méthyl-quinolin-4-ylméthoxy)-benzènesulfonylamino]-propionamide
37) ((S)-3-[4-(3,5-diméthyl-benzyloxy)-benzènesulfonylamino]-N-hydroxy-2-imidazolidin-1-yl-propionamide
38) ((S)-N-hydroxy-2-(4-méthanesulfonyl-[1,4]diazepan-1-yl)-3-[4-(2-méthyl-quinolin-4-ylméthoxy)-benzènesulfonylamino]-propionamide
39) ((S)-2-(4-benzyl-[1,4]diazepan-1-yl)-N-hydroxy-3-[4-(2-méthyl-quinolin-4-ylméthoxy)-benzènesulfonylamino]-propionamide
40) ((S)-2-[1,4]diazocan-1-yl-N-hydroxy-3-[4-(2-méthyl-quinolin-4-ylméthoxy)-benzènesulfonylamino]-propionamide.
41) ((S)-N-hydroxy-3-[4-(2-méthyl-benzofuran-3-ylméthoxy)-benzènesulfonylamino]-2-[4-(propane-2-sulfonyl)-pipérazin-1-yl]-propionamide
42)((S)-2-(4-benzyl-pipérazin-1-yl)-N-hydroxy-3-[4-(2-isopropyl-1H-indol-3-ylméthoxy)-benzènesulfonylamino]-propionamide

Les composés de formule générale (I) sont préparés selon le schéma réactionnel de la figure 1 présenté ci-dessous.

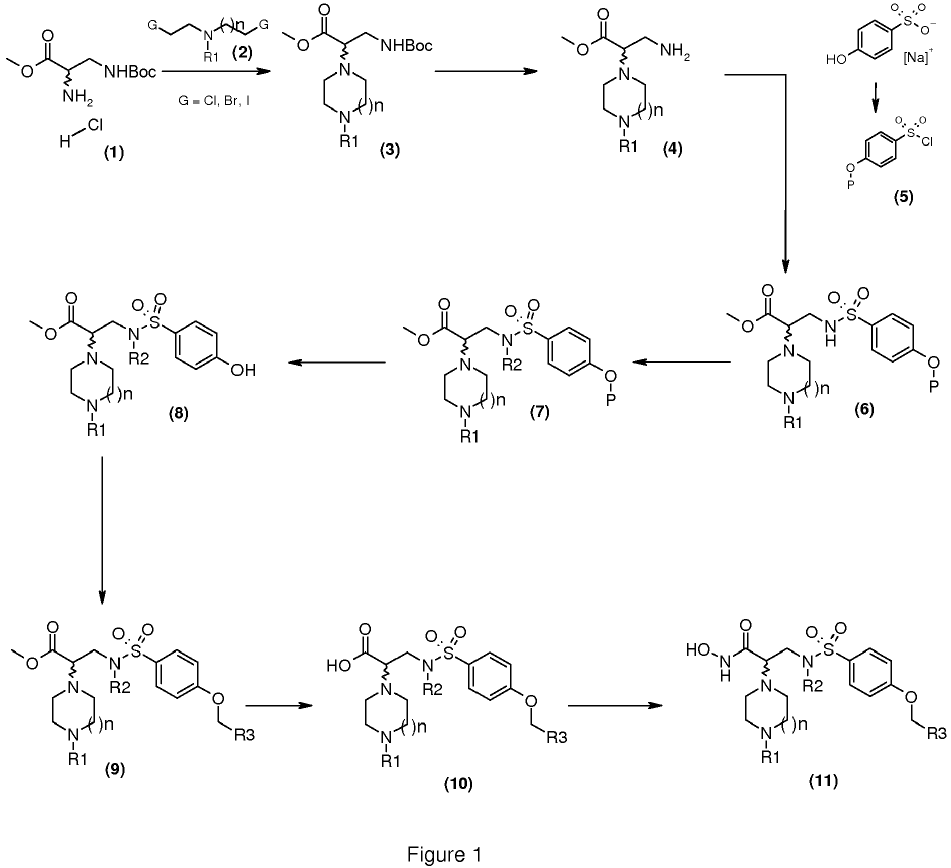

Selon la figure 1, les composés **(3)** sont obtenus par réaction entre l'acide aminé **(1)** H-DAP(Boc)-OMe.HCl ou H-(D)-DAP(Boc)-OMe.HCl et le composé **(2)** (commercial ou préalablement préparé) en présence d'une base tertiaire organique comme la diisopropyléthylamine ou la triéthylamine à une température comprise entre 60°C et 120°C. Les composés **(4)** sont obtenus par déprotection de la fonction amine des composés **(3)** selon des méthodes classiques comme par exemple l'utilisation d'une solution d'acide chlorhydrique dans l'isopropanol.
Une réaction entre le composé **(4)** et le chlorure de 4-hydroxy-benzène sulfonyle O-protégé par un groupement benzyle par exemple (P = CH2-Ph) **(5)** en présence d'une base tertiaire comme par exemple la triéthylamine dans du dichlorométhane conduit au composé **(6).** Une N-alkylation de la fonction sulfonamide peut alors être réalisée par réaction avec un halogénure d'alkyle en présence d'une base comme par exemple du carbonate de potassium dans un solvant tel que du DMF pour conduire au dérivé **(7).** Le composé **(8)** est obtenu par déprotection selon les méthodes connues par l'homme de l'art pour déprotéger une fonction phénol. Le composé **(9)** est obtenu par alkylation de la fonction phénol du composé **(8)** par réaction avec un halogénure d'alkyle en présence d'une base comme par exemple du carbonate de césium dans de l'acétone ou par réaction de Mitsunobu avec un dérivé alcool primaire en présence de triphénylphosphine et de diisopropyl azodicarboxylate par exemple. Par réaction de saponification en présence d'une base comme l'hydroxyde de lithium en présence d'eau et de tétrahydrofuranne par exemple, le composé **(10)** est obtenu. Dans une dernière étape, le composé **(11)** est obtenu par couplage entre la O-(tert-butyldimethylsilyl)hydroxylamine par exemple et le dérivé **(10)** dans des conditions de couplage peptidique classiques, en utilisant par exemple comme agents de couplage le chlorhydrate de 1-(3-diméthylaminopropyl)-3-éthylcarbodiimide, l'hydroxybenzotriazole ou le TBTU, et comme base la triéthylamine ou la diisopropyléthylamine dans un solvant tel que le dichlorométhane ou le diméthylformamide. La déprotection de l'acide hydroxamique silylé intermédiairement formé se fait in situ ou par lavage avec une solution aqueuse légèrement acide pour conduire au composé **(11).**

Une autre alternative pour l'obtention du composé (11) est présentée dans la figure 2 ci-dessous.

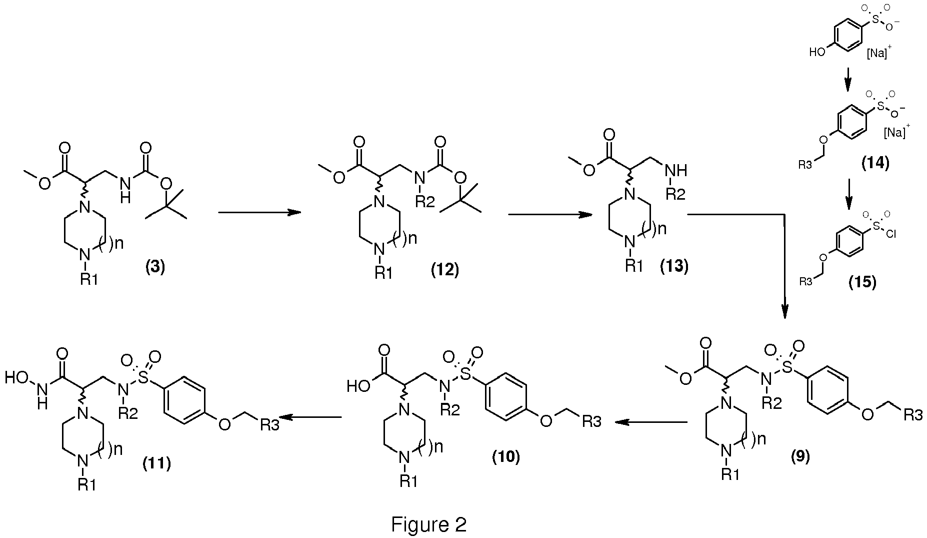

Selon le schéma de synthèse de la figure 2, le dérivé (3) peut éventuellement être alkylé en présence d'une base telle que l'hydrure de sodium et d'un halogénure d'alkyle dans du diméthylformamide par exemple pour conduire au composé (12) à partir duquel le composé (13) est obtenu selon les méthodes classiques de déprotection des amines comme par exemple l'utilisation d'une solution d'acide chlorhydrique dans l'isopropanol.
Le composé (14) est préalablement préparé à partir du sel de sodium de l'acide 4-hydroxy benzènesulfonique commercial par alkylation avec un halogénure d'alkyle en présence d'une base telle que l'hydroxyde de sodium par exemple dans un mélange de solvants comme l'isopropanol et l'eau par exemple. Le composé (15) est alors obtenu par réaction du dérivé (14) avec du chlorure d'oxalyle en présence de diméthylformamide dans du dichlorométhane par exemple.
Le dérivé **(9)** est obtenu par réaction entre les composés **(13)** et **(15)** en présence d'une base comme la triéthylamine dans du dichlorométhane par exemple,

Une voie de synthèse alternative pour l'obtention du composé (11) est également présentée dans la figure 3 ci-dessous.

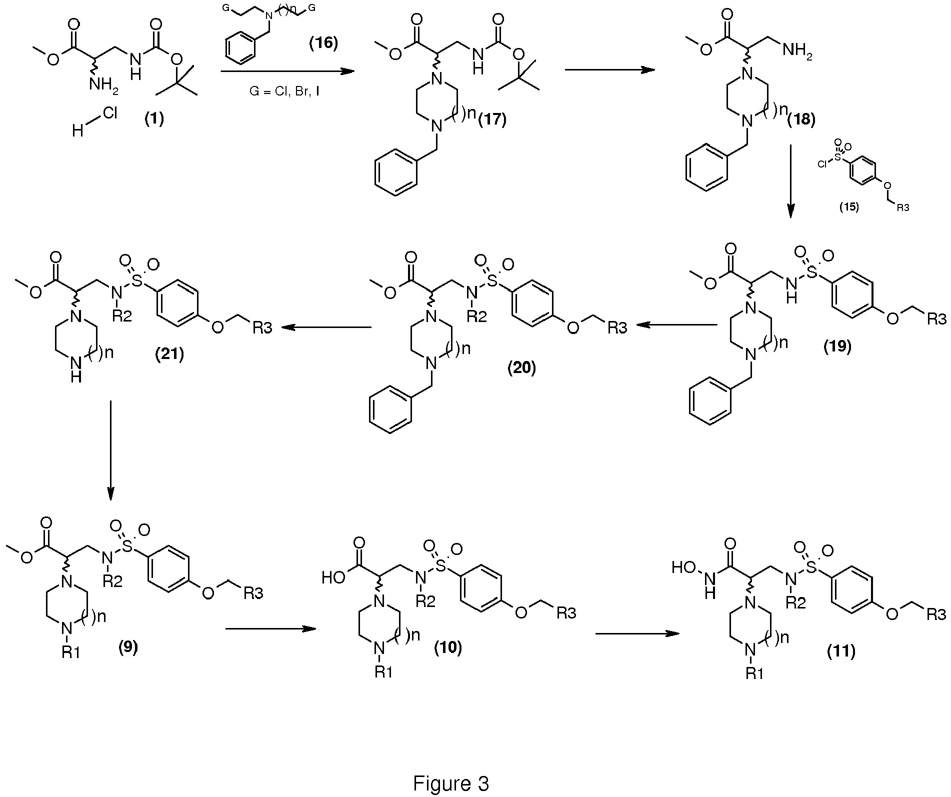

Selon la figure 3, le composé (17) est obtenu par réaction entre l'acide aminé (1) H-DAP(Boc)-OMe.HCl ou H-(D)-DAP(Boc)-OMe.HCl et le composé (16) (préalablement préparé par réaction de la bis (2-chloroéthyl)amine par exemple et du bromure de benzyle en présence de carbonate de potassium dans l'acétonitrile) en présence d'une base tertiaire organique comme la diisopropyléthylamine à une température de 120°C erviron. Après déprotection de la fonction amine, le composé (18) est condensé avec le chlorure de sulfonyle (15) pour conduire au dérivé (19). Une N-alkylation de la fonction sulfonamide peut alors être réalisée par réaction avec un halogénure d'alkyle en présence d'une base comme par exemple du carbonate de potassium dans un solvant tel que du DMF pour conduire au dérivé **(20).** Selon les conditions classiques d'hydrogénation du composé **(20)** en présence de palladium sur charbon dans un solvant tel que l'éthanol par exemple, le composé **(21)** est obtenu. Le composé **(9)** est obtenu selon les méthodes classiques de synthèse par exemple par réaction du composé **(21)** avec un chlorure d'acyle, ou un chlorure de sulfonyle en présence de triéthylamine, ou par réaction avec un halogénure d'alkyle en présence d'une base comme l'hydrure de sodium par exemple. Par réaction de saponification en présence d'une base comme l'hydroxyde de lithium en présence d'eau et de tétrahydrofuranne par exemple, le composé **(10)** est obtenu. Dans une dernière étape, le composé **(11)** est obtenu par couplage entre la O-(tert-butyldimethylsilyl)hydroxylamine par exemple et le dérivé **(10)** dans des conditions de couplage peptidique classiques, en utilisant par exemple comme agents de couplage le chlorhydrate de 1-(3-diméthylaminopropyl)-3-éthylcarbodiimide, l'hydroxybenzotriazole ou le TBTU, et comme base la triéthylamine ou la diisopropyléthylamine dans un solvant tel que le dichlorométhane ou le diméthylformamide. La déprotection de l'acide hydroxamique silylé intermédiairement formé se fait in situ ou par lavage avec une solution aqueuse acide pour conduire au composé **(11).**

Une voie de synthèse alternative pour les composés avec R1 représentant un radical -(CO)-R₄ est décrite dans la figure 4.

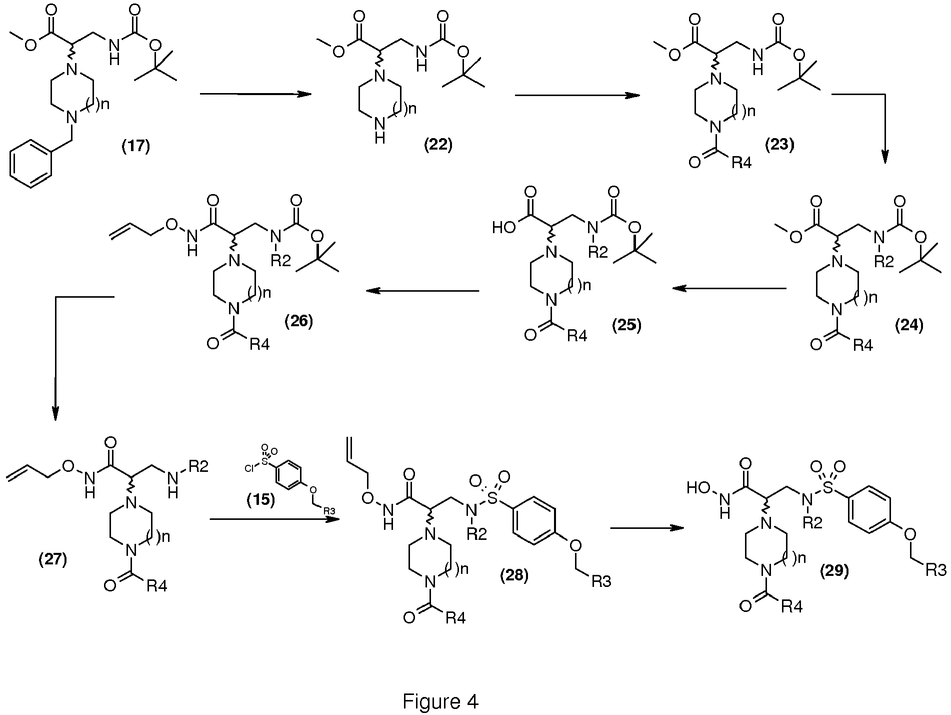

Après déprotection de la fonction amine du composé (17) suivant des conditions classiques d'hydrogénation en présence de palladium sur charbon dans un solvant tel que l'éthanol par exemple, le composé (22) est obtenu. Par réaction avec un chlorure d'acyle, R₄COCl en présence d'une base telle que la triéthylamine, le composé (23) est obtenu. Lorsque R₂ représente un radical alkyle inférieur, une N-alkylation du carbamate est alors réalisée par réaction avec un halogénure d'alkyle en présence d'une base comme par exemple du carbonate de potassium dans un solvant tel que du DMF pour conduire au dérivé **(24).** Par réaction de saponification en présence d'une base comme l'hydroxyde de lithium en présence d'eau et de tétrahydrofuranne par exemple, le composé **(25)** est préparé. Un couplage entre le chlorhydrate de O-allylhydroxylamine par exemple et le dérivé **(25)** permet d'obtenir le composé **(26)** dans des conditions de couplage peptidique classiques. Pour cela, on utilise par exemple le chlorhydrate de 1-(3-diméthylaminopropyl)-3-éthylcarbodiimide, l'hydroxybenzotriazole ou le TBTU comme agents de couplage, et la triéthylamine ou la diisopropyléthylamine comme base. La réaction s'effectue dans un solvant tel que le dichlorométhane ou le diméthylformamide. Après déprotection de la fonction amine du composé **(26)** selon des méthodes classiques, le composé **(27)** est obtenu. Il est condensé avec le chlorure de sulfonyle **(15)** pour conduire au composé **(28).** Dans une dernière étape, le composé **(29)** est obtenu par déprotection de la fonction hydroxylamine du composé **(28)** selon des méthodes classiques comme par exemple le traitement par du tétrakis(triphénylphosphine)palladium (0) et du carbonate de potassium dans du méthanol.

Selon la présente invention, les composés de formule générale (I) préférés sont ceux pour lesquels :
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical - C(O)-R₄, un radical -SO₂-R₄, un radical C(O)OR4, R₄ ayant les significations données ci-après;
R₂ est un atome d'hydrogène ou un radical alkyle inférieur.
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
n peut prendre les valeurs de 0, 1, 2;
ainsi que leurs sels d'addition avec un acide pharmaceutiquement acceptable, leurs sels d'addition avec une base pharmaceutiquement acceptable et les énantiomères desdits composés.

Selon la présente invention, les composés de formule générale (I) particulièrement préférés sont ceux pour lesquels :
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical -C(O)-R₄, un radical -SO₂-R₄, R₄ ayant les significations données ci-après;
R₂ est un atome d'hydrogène ou un radical alkyle inférieur ;
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué ;
R₄ est un radical alkyle, un radical alkyle substitué, un radical aryle, un radical aryle substitué, un radical aralkyle, un radical aralkyle substitué;
n peut prendre les valeurs de 1 ou 2;
ainsi que leurs sels d'addition avec un acide pharmaceutiquement acceptable, leurs sels d'addition avec une base pharmaceutiquement acceptable et les énantiomères desdits composés.

Selon la présente invention, les composés de formule générale (I) plus particulièrement préférés sont ceux pour lesquels :
R₁ représente un radical alkyle, un radical alkyle substitué, un radical aralkyle, un radical aralkyle substitué, un radical -C(O)-R₄, un radical -SO₂-R₄, R₄ ayant les significations données ci-après;
R₂ est un atome d'hydrogène,
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

Selon la présente invention, les composés de formule générale (I) encore plus particulièrement préférés sont ceux pour lesquels :
R₁ représente un radical alkyle, un radical alkyle substitué, un radical aralkyle, un radical aralkyle substitué, un radical -C(O)-R₄, un radical -SO₂-R₄, R₄ ayant les significations données ci-après;
R₂ est un atome d'hydrogène,
R₃ est un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

Selon la présente invention, les composés de formule générale (I) les plus particulièrement préférés sont ceux pour lesquels :
R₁ représente un radical alkyle, un radical alkyle substitué, un radical aralkyle, un radical aralkyle substitué, un radical -C(O)-R₄, un radical -SO₂-R₄, R₄ ayant les significations données ci-après;
R₂ est un atome d'hydrogène,
R₃ est un radical hétéroaryle, un radical hétéroaryle substitué,
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

Les composés selon l'invention présentent une très bonne activité inhibitrice de TACE et en particulier, ils inhibent l'enzyme TACE de façon sélective par rapport à d'autres ADAMs et MMPs. Cette activité inhibitrice de l'enzyme TACE est mesurée dans un test enzymatique et quantifiée par la mesure d'une IC₅₀ (concentration d'inhibiteur nécessaire pour obtenir 50% d'inhibition de l'enzyme TACE), comme décrit dans l'exemple 28. Les composés de la présente invention présentent une IC₅₀ pour TACE inférieure ou égale à 10µM et plus particulièrement inférieure ou égale à 1µM De façon avantageuse, les composés de la présente invention présentent une IC₅₀ pour TACE inférieure ou égale à 0,5µM.
De façon avantageuse, ces composés sont également très sélectifs de TACE par rapport aux autres ADAMs et MMPs (test décrit dans l'exemple 29): leur activité inhibitrice est au moins 10 fois plus importante pour TACE que pour d'autres ADAMs et MMPs (c'est-à-dire que la valeur de l'IC₅₀ pour TACE est au moins 10 fois plus petite que celle pour d'autres ADAMs et MMPs), et plus avantageusement au moins 100 fois plus importante.

TACE (TNFα-Converting Enzyme) catalyse la formation du TNF-alpha soluble à partir de la protéine précurseur (TNFα transmembranaire) liée aux membranes de certaines cellules. Le TNFα est une cytokine pro-inflammatoire qui est connue pour jouer un rôle dans de nombreuses pathologies à caractère inflammatoire.
L'invention vise donc l'utilisation d'au moins un composé de formule générale (I) tel que défini ci-dessus pour le traitement de pathologies ou de désordres liés à une libération de TNFα. Un inhibiteur de l'enzyme TACE de formule générale (I) diminue la production de TNFα. De ce fait, il est utile pour le traitement de pathologies liées à une libération de TNFα.
L'invention vise aussi l'utilisation d'au moins un composé de formule générale (I) tel que défini ci-dessus pour la préparation d'une composition pharmaceutique ou cosmétique dans laquelle ledit composé a une activité inhibitrice de l'enzyme TACE.
Elle vise donc l'utilisation d'au moins un composé de formule générale (I) tel que défini ci-dessus pour le traitement de pathologies ou de désordres qui sont améliorés par l'inhibition de l'enzyme TACE. L'invention concerne également une méthode de traitement thérapeutique (humain ou animal) ou cosmétique, consistant en l'administration ou l'application d'une composition pharmaceutique ou cosmétique comprenant un composé de formule générale (I) en tant qu'inhibiteur de TACE et de ce fait en tant qu'inhibiteur de la production de TNFα soluble.
Ainsi, l'invention concerne l'utilisation d'au moins un composé de formule générale (I) tel que défini ci-dessus pour le traitement de pathologies ou de désordres liés à une production de TNFα.

L'invention concerne également l'utilisation d'un composé de formule générale (I) tel que défini ci-dessus pour la préparation d'un médicament destiné au traitement de pathologies pour lesquelles diminuer la production de TNFα serait d'un grand intérêt.
En effet, les composés utilisés selon l'invention sont particulièrement appropriés au traitement et à la prévention des désordres / maladies tels que les maladies inflammatoires listées ci-après mais non limitatives comme le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'athérosclérose, l'obésité, l'attaque ischémique, le rejet de greffe, le cancer, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD), les maladies inflammatoires de la peau, le psoriasis, la dermatite atopique et le rhumatisme psoriasique.
Ces molécules sont également des principes actifs potentiels pour le traitement de pathologies neurologiques à caractère inflammatoire pour lesquelles diminuer la production de TNFα serait d'un grand intérêt. Ces pathologies listées ci-après de façon non limitative sont par exemple la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'cedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale ;

L'invention concerne l'utilisation d'un composé de formule générale (I) tel que défini ci-dessus pour la préparation d'un médicament destiné au traitement de pathologies à caractère inflammatoire dans lesquelles le TNFα est impliqué.

L'invention concerne l'utilisation d'un composé de formule générale (I) tel que défini ci-dessus pour la préparation d'un médicament destiné au traitement de maladies inflammatoires de la peau, du psoriasis, de la dermatite atopique ou du rhumatisme psoriasique;

La présente invention a aussi pour objet une composition pharmaceutique destinée notamment au traitement des affections susmentionnées, et qui est caractérisée par le fait qu'elle comprend, dans un support pharmaceutiquement acceptable et compatible avec le mode d'administration retenu pour cette composition, au moins un composé de formule générale (I). Ce composé de formule générale (I) peut également se présenter sous une de ses formes énantiomériques ou sous la forme d'un de ses sels pharmaceutiquement acceptables.

On va maintenant donner, à titre d'illustration et sans aucun caractère limitatif, plusieurs exemples de préparation de composés actifs de formule (I) selon l'invention, ainsi que des résultats d'activité biologiques de tels composés.

### EXEMPLES DE REALISATION

Les composés de formule générale (I) sont caractérisés par analyse RMN du proton sur un appareil Advanced 400MHz Bruker.

### Exemple 1: 3-[(4-but-2-ynyloxy-benzènesulfonyl)-méthyl-amino]-N-hydroxy-2-(4-méthanesulfonyl-pipérazin-1-yl)-propionamide.

### 1-1 : 2-(4-tert-butoxycarbonyl-pipérazin-1-yl)-malonate de diméthyle

19,5g (141 mmoles) de carbonate de potassium puis 19,5ml (134 mmoles) de diméthylbromomalonate sont additionnés à une solution de 25g (134 mmoles) de pipérazine 1-carboxylate de tertbutyle dans 300ml d'acétonitrile. Le milieu réactionnel est agité à température ambiante pendant 24h puis filtré pour éliminer les sels insolubles et concentré sous vide. Le résidu brut obtenu est purifié par chromatographie sur gel de silice élué avec un mélange heptane/acétate d'éthyle 70/30. 41g (97%) de 2-(4-tert-butoxycarbonyl-pipérazin-1-yl)-malonate de diméthyle sont obtenus sous forme d'une huile claire.

### 1-2 : 2-(4-tert-butoxycarbonyl-pipérazin-1-yl)-2-(1,3-dioxo-1,3-dihydro-isoindol-2-ylméthyl)-malonate de diméthyle:

3,5g (87 mmoles) d'hydrure de sodium sont additionnés par portions à une solution de 25g (87 mmoles) de 2-(4-tert-butoxycarbonyl-piperazin-1-yl)-malonate de diméthyle dans 250ml de tétrahydrofuranne refroidie à 2°C. Le milieu réactbnnel est agité à température ambiante pendant 30 minutes puis ramené à 2°C avant addition goutte à goutte de 21g (87 mmoles) de 2-bromométhyl-isoindole-1,3-dione dans 200ml de tétrahydrofuranne. Le milieu réactionnel est agité à température ambiante pendant 20h, traité par addition de 500ml d'eau puis extrait à l'acétate d'éthyle. La phase organique est séchée sur sulfate de magnésium, filtrée et concentrée sous vide.
Le produit brut obtenu est purifié par chromatographie sur gel de silice élué avec un mélange heptane/acétate d'éthyle 70/30. 27,5g (73%) de 2-(4-tert-Butoxycarbonyl-piperazin-1-yl)-2-(1,3-dioxo-1,3-dihydro-isoindol-2-ylmethyl)-malonate de diméthyle sont obtenus sous forme d'un solide blanc.

### 1-3: 2-aminométhyl-2-(4-tert-butoxycarbonyl-piperazin-1-yl)-malonate de diméthyle

Une solution de 2,9ml (64 mmoles) d'hydrazine hydrate dans 8ml de méthanol est additionnée à une solution de 27,5g (58 mmoles) de 2-(4-tert-butoxycarbonyl-pipérazin-1-yl)-2-(1,3-dioxo-1,3-dihydro-isoindol-2-ylméthyl)-malonate de diméthyle dans 300ml de méthanol préalablement refroidie à -5°C. Le milieu réactionnel est agité de -5°C à température ambiante pendant 3h. Après évaporation et addition de 300ml d'eau, le milieu réactionnel est extrait à l'acétate d'éthyle. Les phases organiques sont lavées avec une solution aqueuse saturée d'hydrogénocarbonate de sodium, séchées sur sulfate de magnésium, filtrées, évaporées. Le résidu obtenu est purifié par chromatographie sur gel de silice élué avec un mélange heptane / acétate d'éthyle 8/2 puis augmentation de la polarité jusqu'à un mélange acétate d'éthyle / méthanol 90/10. 10g (50%) de 2-aminométhyl-2-(4-tert-butoxycarbonyl-piperazin-1-yl)-malonate de diméthyle sont ainsi obtenus sous forme d'une huile claire.

### 1-4: 2-(4-tert-butoxycarbonyl-pipérazin-1-yl)-2-[(4-but-2-ynyloxybenzènesulfonylamino)-méthyl]-malonate de diméthyle

1,1ml (8 mmoles) de triéthylamine puis 1,8ml (7 mmoles) de chlorure de 4-but-2-ynyloxy-benzènesulfonyle sont additionnés sur une solution de 2,5g (7 mmoles) de 2-aminométhyl-2-(4-tert-butoxycarbonyl-piperazin-1-yl)-malonate de diméthyle dans 30ml de dichlorométhane. Le milieu réactionnel est agité à température ambiante pendant 2 heures puis concentré sous vide. Le produit brut obtenu est purifié par chromatographie sur gel de silice élué avec un mélange heptane/acétate d'éthyle 70/30. 2,1g (51%) de 2-(4-tert-butoxycarbonyl-pipérazin-1-yl)-2-[(4-but-2-ynyloxy-benzènesulfonylamino)-méthyl]-malonate de diméthyle sont obtenus sous forme d'un solide blanc.

### 1-5: 2-[(4-but-2-ynyloxy-benzènesulfonylamino)-méthyl]-2-piperazin-1-yl-malonate de diméthyle

2,8ml d'acide trifluoroacétique sont additionnés sur une solution de 2,1g (4 mmoles) de 2-(4-tert-butoxycarbonyl-piperazin-1-yl)-2-[(4-but-2-ynyloxybenzènesulfonylamino)-méthyl]-malonate de diméthyle dilué dans 30ml de dichlorométhane. Après agitation à température ambiante pendant 24h, une solution aqueuse saturée d'hydrogénocarbonate de sodium est additionnée jusqu'à pH=8 et le milieu réactionnel est extrait par du dichlorométhane. Les phases organiques sont rassemblées, lavées à l'eau, séchées sur sulfate de magnésium puis filtrées et évaporées. 1,7g (98%) de 2-[(4-but-2-ynyloxy-benzènesulfonylamino)-méthyl]-2-pipérazin-1-yl-malonate de diméthyle sont obtenus sous forme d'un solide blanc.

### 1-6: 2-[(4-but-2-ynyloxy-benzènesulfonylamino)-méthyl]-2-(4-méthanesulfonyl-pipérazin-1-yl)-malonate de diméthyle

0,6ml (4 mmoles) de triéthylamine puis 0,3ml (4 mmoles) de chlorure de méthanesulfonyle sont additionnés sur une solution de 1,6g (4 mmoles) de 2-[(4-but-2-ynyloxy-benzènesulfonylamino)-méthyl]-2-piperazin-1-yl-malonate de diméthyle dilué dans 30ml de dichlorométhane. Le milieu réactionnel est ensuite agité à température ambiante pendant 3h puis évaporé à sec. Le résidu brut est purifié par chromatographie sur gel de silice élué avec un mélange dichlorométhane/méthanol 99/1. 1,1g (58%) de 2-[(4-but-2-ynyloxy-benzènesulfonylamino)-méthyl]-2-(4-méthanesulfonyl-piperazin-1-yl)-malonate de diméthyle sont obtenus sous forme d'un solide blanc.

### 1-7: 2-{(4-but-2-ynyloxy-benzènesulfonyl)-méthyl-amino]-méthyl}-2-(4-méthanesulfonyl-pipérazin-1-yl) malonate de diméthyle

120mg (0,9 mmoles) de carbonate de potassium puis 56µl (0,9 mmoles) d'iodure de méthyle sont additionnés à une solution de 400mg (0,8 mmoles) de 2-[(4-but-2-ynyloxy-benzènesulfonylamino)-méthyl]-2-(4-méthanesulfonyl-pipérazin-1-yl)-malonate de diméthyle dans 10ml de diméthylformamide. Le milieu réactionnel est ensuite agité à température ambiante pendant 18h puis hydrolysé par addition d'eau et extrait à l'acétate d'éthyle. Les phases organiques sont lavées par de l'eau puis séchées sur sulfate de magnésium, filtrées et concentrées sous vide. Le produit brut obtenu est purifié par chromatographie sur gel de silice élué avec un mélange heptane/acétate d'éthyle 50/50. 410mg (100%) de 2-{[(4-but-2-ynyloxy-benzènesulfonyl)-méthyl-amino]-méthyl}-2-(4-méthanesulfonyl-pipérazin-1-yl)-malonate de diméthyle sont obtenus sous forme d'un solide blanc.

### 1-8: acide 3-[(4-but-2-ynyloxy-benzènesulfonyl)-méthyl-amino]-2-(4-méthanesulfonyl-piperazin-1-yl)-propanoïque

1,7ml (1,7 mmoles) d'une solution aqueuse d'hydroxyde de sodium de concentration 1M sont additionnés sur une solution de 270mg (0,5 mmoles) de 2-{[(4-but-2-ynyloxy-benzènesulfonyl)-méthyl-amino]-méthyl}-2-(4-méthanesulfonyl-pipérazin-1-yl) malonate de diméthyle dans 7ml de tétrahydrofuranne et 2ml de méthanol. Le milieu réactionnel est agité à 40°C pendant 15h puis ramené à pH=6 par l'addition d'une solution aqueuse d'acide chlorohydrique de concentration 1M. Après évaporation sous vide des solvants, le produit précipite. Le résidu obtenu est repris dans 5ml d'eau et agité pendant 30min jusqu'à précipitation. Le produit est filtré, rincé à l'eau puis séché sous vide. 200mg (87%) d'acide 3-[(4-but-2-ynyloxy-benzènesulfonyl)-méthyl-amino]-2-(4-méthanesulfonyl-pipérazin-1-yl)-propanoique sont obtenus sous forme d'un solide blanc.

### 1-9: 3-[(4-but-2-ynyloxy-benzènesulfonyl)-méthyl-amino]-N-hydroxy-2-(4-méthanesulfonyl-pipérazin-1-yl)-propionamide

63mg (0,5 mmoles) de 1-hydroxybenzotriazole puis 88mg (0,5 mmoles) de chlorhydrate de 1-éthyl-3-(3-diméthylaminopropyl)carbodiimide sont ajoutés à une solution de 200mg (0,4 mmoles) d' acide 3-[(4-but-2-ynyloxy-benzènesulfonyl)-méthyl-amino]-2-(4-méthanesulfonyl-piperazin-1-yl)-propanoïque dans 6ml de diméthylformamide. Le milieu réactionnel est agité 10min à température ambiante puis 68mg (0,5 mmoles) de O-tert-butyldiméthysilylhydroxylamine sont ajoutés . Le milieu réactionnel est ensuite agité à température ambiante pendant 24h, hydrolysé par l'addition de 2ml d'une solution aqueuse d'acide citrique 5% et agité pendant 30 minutes supplémentaires. Après extraction à l'acétate d'éthyle, la phase organique est lavée à l'eau, séchée sur sulfate de magnésium, filtrée et concentrée. Le résidu brut est purifié par chromatographie sur gel de silice élué avec un mélange dichlorométhane/méthanol 95/5. 100mg (50%) de 3-[(4-but-2-ynyloxy-benzènesulfonyl)-méthyl-amino]-N-hydroxy-2-(4-méthanesulfonyl-pipérazin-1-yl)-propionamide sont obtenus sous forme d'un solide blanc de point de fusion 86°C.
RMN ¹H (δ, DMSO) : 1,91 (s, 3H); 2,63-2,68 (m, 2H); 2,72 (s, 3H); 2,72-2,75 (m, 2H); 2,92 (s, 3H); 3,05-3,15 (m, 5H); 3,30-3,38 (m, 2H); 4,93 (s, 2H); 7,24 (d, J=6,8Hz, 2H); 7,79 (d, J=6,8Hz, 2H); 9,06 (s, 1 H); 10.77 (s, 1 H).

### Exemple 2: (S)-3-(4-but-2-ynyloxy-benzènesulfonylamino)-N-hydroxy-2-(4-méthanesulfonyl-pipérazin-1-yl)-propionamide.

### 2-1: Sel de sodium de l'acide 4-but-2-ynyloxy-benzènesulfonique

50g (370 mmoles) de 1-bromo-but-2-yne sont additionnés sur une solution de 43g (185 mmoles) de sel de sodium de l'acide 4-hydroxy-benzènesulfonique commercial et de 185ml (185 mmoles) d'une solution aqueuse d'hydroxyde de sodium de concentration 1M dans 800ml d'isopropanol. Le milieu réactionnel est chauffé à 70°C pendant 18h.
Après évaporation de l'isopropanol, le produit obtenu est filtré, rincé à l'isopropanol et à l'éther diéthylique puis séché sous vide. 46g (100%) du sel de sodium de l'acide 4-but-2-ynyloxy-benzènesulfonique sont obtenus sous forme d'un solide blanc.

### 2.2: Chlorure de 4-but-2-ynyloxy-benzènesulfonyle

30g (107 mmoles) du sel de sodium de l'acide 4-but-2-ynyloxy-benzènesulfonique dans 120ml de diméthylformamide sont additionnés goutte à goutte à une solution de 28ml (321 mmoles) de chlorure d'oxalyle dans 120ml de dichlorométhane, préalablement refroidie à -10°C puis le milieu réactionnel est agité à température ambiante pendant 18h. 800ml de glace sont additionnés et le milieu est extrait à l'acétate d'éthyle. Les phases organiques sont rassemblées, lavées à l'eau, séchées de sulfate de magnésium, filtrées et concentrées sous vide. 22g (84%) de chlorure de 4-but-2-ynyloxy-benzènesulfonyle sous la forme d'un solide beige.

### 2.3: N,N-bis-(2-chloro-éthyl)-méthanesulfonamide

8,6ml (62 mmoles) de triéthylamine sont additionnés à une solution de 5g (28 mmoles) du chlorhydrate de bis-(2-chloroéthyl)-amine dans 60ml de dichlorométhane. Les sels de chlorure de triéthylammonium précipitent et sont filtrés. 2,4ml (31 mmoles) de chlorure de méthanesulfonyle sont alors additionnés au filtrat obtenu et le milieu réactionnel est agité à température ambiante pendant 3h. Après addition d'eau, le produit est extrait au dichlorométhane. La phase organique est lavée à l'eau, séchée sur sulfate de magnésium, filtrée et concentrée. 5,8g (94%) de N,N-bis-(2-chloro-éthyl)-méthanesulfonamide sont obtenus sous forme d'un solide beige.

### 2.4: (S)-3-tert-butoxycarbonylamino-2-(4-méthanesulfonyl-pipérazin-1-yl)-propanoate de méthyle

Dans un tube de Schlenk, une solution de 5g (20 mmoles) de chlorhydrate de (S)-2-amino-3-tert-butoxycarbonylamino-propanoate de méthyle et 4,3g (20 mmoles) de N,N-bis-(2-chloro-éthyl)-méthanesulfonamide dans 65ml de N,N-Diisopropyléthylamine est chauffée à 127°C sous forte agitation pendant 18h. Après addition d'eau, le produit est extrait à l'acétate d'éthyle. Les phases organiques sont rassemblées, lavées à l'eau, séchées sur sulfate de magnésium, filtrées et concentrées sous vide. Le produit brut obtenu est purifié par chromatographie sur gel de silice élué avec un mélange heptane / acétate d'éthyle 50/50. 3,3g (46%) de (S)-3-tert-butoxycarbonylamino-2-(4-méthanesulfonyl-piperazin-1-yl)-propanoate de méthyle sont obtenus sous forme d'un solide blanc.

### 2.5: Chlorhydrate de (S)-3-amino-2-(4-méthanesulfonyl-pipérazin-1-yl)-propanoate de méthyle

15ml d'une solution d'acide chlorhydrique dans l'isopropanol de concentration 5-6N sont additionnés goutte à goutte à une solution de 2,7g (7,4 mmoles) de (S)-3-tert-butoxycarbonylamino-2-(4-méthanesulfonyl-pipérazin-1-yl)-propanoate de méthyle dans 30ml de méthanol. Le milieu réactionnel est agité à 40°C pendant 2h, concentré sous vide puis repris dans 20ml de méthanol et 150ml d'éther diéthylique. Le produit précipite, est filtré sous vide, rincé à l'éther diéthylique puis séché sous vide. 2,3g (100%) du chlorhydrate de (S)-3-amino-2-(4-méthanesulfonyl-piperazin-1-yl)-propanoate de méthyle sont obtenus sous forme d'un solide blanc.

### 2.6: (S)-3-(4-but-2-ynyloxy-benzènesulfonylamino)-2-(4-méthanesulfonyl-pipérazin-1-yl)-propanoate méthyle

0,3ml (2 mmoles) de triéthylamine et 270mg (1 mmole) de chlorure de 4-but-2-ynyloxy-benzènesulfonyle (préparé comme décrit en 2.2) sont additionnés sur une solution de 300mg (1 mmole) du chlorhydrate de (S)-3-amino-2-(4-méthanesulfonyl-pipérazin-1-yl)-propanoate de méthyle (préparé comme décrit en 2.5) dans 8ml de dichlorométhane. Après agitation à température ambiante pendant 18h,de l'eau est additionné et le milieu réactionnel est extrait au dichlorométhane. Les phases organiques sont lavées à l'eau, séchées sur sulfate de magnésium, filtrées et concentrés.
Le produit brut obtenu est purifié par chromatographie sur gel de silice élué avec un mélange heptane / acétate d'éthyle 50/50. 400mg (85%) de (S)-3-(4-but-2-ynyloxy-benzènesulfonylamino)-2-(4-méthanesulfonyl-piperazin-1-yl)-propanoate de méthyle sous forme d'un solide blanc.

### 2.7: acide (S)-3-(4-but-2-ynyloxy-benzènesulfonylamino)-2-(4-méthanesulfonyl-piperazin-1-yl)-propanoïque

1,3ml (1,3 mmoles) d'une solution aqueuse d'hydroxyde de lithium de concentration 1M sont additionnés sur une solution de 400mg (0,8 mmoles) de (S)-3-(4-but-2-ynyloxy-benzènesulfonylamino)-2-(4-méthanesulfonyl-pipérazin-1-yl)-propanoate de méthyle dilué dans 10ml de tétrahydrofuranne préalablement refroidie à 0°C. Le milieu réactionnel est agité à température ambiante pendant 20h. Après évaporation à sec, 1,5ml d'une solution aqueuse d'acide acétique de concentration 1 M sont additionnés pour obtenir un pH= 6. Le produit précipite, est filtré, rincé à l'eau puis à l'éther diéthylique et séché sous vide. 340mg (89%) d'acide (S)-3-(4-but-2-ynyloxy-benzènesulfonylamino)-2-(4-méthanesulfonyl-pipérazin-1-yl)-propanoïque sont obtenus sous forme d'un solide blanc.

### 2.8: (S)-3-(4-but-2-ynyloxy-benzènesulfonylamino)-N-hydroxy-2-(4-méthanesulfonyl-piperazin-1-yl)-propionamide

120mg (0,9 mmoles) de 1-hydroxybenzotriazole et 170mg (0,9 mmoles) du chlorhydrate de 1-éthyl-3-(3-diméthylaminopropyl)carbodiimide sont ajoutés à une solution de 340mg (0,7 mmoles) d' acide (S)-3-(4-but-2-ynyloxy-benzènesulfonylamino)-2-(4-méthanesulfonyl-pipérazin-1-yl)-propanoïque dans 8ml de diméthylformamide. Le milieu réactionnel est agité 30min puis 120mg (0,8 mmoles) de O-tert-butyldiméthysilyl-hydroxylamine dans 3ml de diméthylformamide sont additionnés. Le milieu réactionnel est ensuite agité à température ambiante pendant 20h puis hydrolysé par 2ml d'eau et 2ml d'une solution aqueuse 5% d'acide citrique. Après agitation pendant 30min, une solution aqueuse saturée d'hydrogénocarbonate de sodium est ajoutée jusqu'à pH=8 puis le milieu réactionnel est extrait par de l'acétate d'éthyle. La phase organique est séchée sur sulfate de magnésium, filtrée et concentrée. Le résidu est repris dans du dichlorométhane, filtré puis séché sous vide. 80mg (23%) de (S)-3-(4-but-2-ynyloxy-benzènesulfonylamino)-N-hydroxy-2-(4-méthanesulfonyl-piperazin-1-yl)-propionamide sont obtenus sous forme d'un solide blanc de point de fusion 150°C.
RMN ¹H (δ, DMSO) : 1,86 (s, 3H); 2,55 (m, 4H); 2,83 (s, 3H); 2,85-2,88 (m, 1 H); 2,97-3,00 (m, 3H); 3,00-3,06 (m, 2H); 3,10-3,12 (t, J=4,8Hz, 1H); 4,86 (s, 2H); 7,15 (d, J=9,2Hz, 2H); 7,51 (s, 1H); 7,75 (d, J=9,2Hz, 2H); 8,94 (s, 1 H); 10,6 (s, 1 H).

### Exemple 3 : (S)-3-(4-benzyloxy-benzenesulfonylamino)-N-hydroxy-2-(4-methanesulfonyl-piperazin-1-yl)-propionamide.

### 3.1 : N,N-bis-(2-chloro-éthyl)-méthanesulfonamide

14,3ml (185 mmoles) de chlorure de méthane sulfonyle sont ajoutés lentement à une solution de 15g (84 mmoles) de chlorhydrate de bis(2-chloroéthylamine) commercial et 26ml (185 mmoles) de triéthylamine dans 200ml de dichlorométhane et 70ml de tétrahydrofuranne préalablement agitée pendant 15min puis filtrée pour enlever le chlorure de triéthylammonium. Le milieu réactionnel est ensuite agité à température ambiante pendant 18h, extrait au dichlorométhane, lavé à l'eau. La phase organique est séchée sur sulfate de magnésium, filtrée, évaporée. Le résidu obtenu est lavé à l'éther diisopropylique, filtré puis séché sous vide. 15,3g (82%) de N,N-bis-(2-chloro-éthyl)-méthanesulfonamide sont obtenus sous forme d'un solide.

### 3.2: (S)-3-tert-butoxycarbonylamino-2-(4-méthanesulfonyl-pipérazin-1-yl)-propanoate de méthyle

Une solution de 9,6g (44 mmoles) de *N,N-bis-(2-chloro-éthyl)-méthanesulfonamide* et de 11,1 g (44 mmoles) de chlorhydrate de 2-amino-3-tert-butoxy-propanoate de méthyle dans 90ml de diisopropyléthylamine est chauffée à 127°C pendant 18h. Le milieu réactionnel est évaporé à sec. 31 g de résidu brut sont obtenus et purifiés par chromatographie sur gel de silice élués avec un mélange heptane / acétate d'éthyle 9/1 puis augmentation de la polarité jusqu'à 4/6. 5,5g (35%) de (S)-3-tert-butoxycarbonylamino-2-(4-méthanesulfonyl-pipérazin-1-yl)-propanoate de méthyle sont obtenus.

### 3.3: dichlorhydrate de (S)-3-Amino-2-(4-methanesulfonyl-piperazin-1-yl)-propanoate de méthyle

Une solution de 4g (11 mmoles) de (S)-3-tert-butoxycarbonylamino-2-(4-méthanesulfonyl-pipérazin-1-yl)-propanoate de méthyle (préparé comme décrit dans l'exemple 2.4) dans 40ml de méthanol et 20ml d'une solution d'acide chlorhydrique dans l'isopropanol de concentration 5 ou 6M est agitée à 40°C pendant 18h puis concentrée sous vide. Le résidu obtenu est repris dans 200ml d'éther diéthylique, filtré puis séché sous vide. 3,5g (94%) du dichlorhydrate de (S)-3-amino-2-(4-méthanesulfonyl-piperazin-1-yl)-propanoate de méthyle sont obtenus sous forme d'un solide beige.

### 3.4: sel de sodium de l'acide 4-benzyloxy-benzènesulfonique

64ml (539 mmoles) de bromure de benzyle sont additionnés à une solution de 50g (215 mmoles) de sel de sodium de l'acide 4-hydroxy-benzènesulfonique dihydraté dans 700ml d'isopropanol et 250ml (250 mmoles) d'une solution aqueuse d'hydroxyde de sodium de concentration 1M. Le milieu réactionnel est chauffé à 70°C pendant 20h.Après concentration sous vide de l'isopropanol, le produit précipite et est filtré. 61g (100%) de sel de sodium de l'acide 4-benzyloxy-benzènesulfonique sont obtenus sous forme d'un solide blanc.

### 3.5: chlorure de 4-benzyloxy-benzènesulfonyle

Une solution de 55ml (639 mmoles) de chlorure d'oxalyle dans 250ml de dichlorométhane est additionnée goutte à goutte à une solution de 61g (213 mmoles) de sel de sodium de l'acide 4-benzyloxy-benzènesulfonique dans 200ml de diméthylformamide, en maintenant la température entre -20°C et -10°C. Après addition, le milieu réactionnel est lentement ramené à température ambiante puis agité pendant 18h, versé sur de la glace et extrait à l'acétate d'éthyle. La phase organique est lavée par de l'eau, par une solution aqueuse saturée de chlorure de sodium et concentrée sous vide. 54g (89%) de chlorure de 4-benzyloxy-benzènesulfonyle sont obtenus sous forme d'un solide blanc.

### 3.6: (S)-3-(4-benzyloxy-benzènesulfonylamino)-2-(4-méthanesulfonyl-pipérazin-1-yl)-propanoate de méthyle

1,1ml (7,8 mmoles) de triéthylamine puis 730mg (2,6 mmoles) de chlorure de 4-benzyloxy-benzènesulfonyle dans 8ml de dichlorométhane sont additionnés à une solution de 800mg (2,4 mmoles) de dichlorhydrate de (S)-3-amino-2-(4-méthanesulfonyl-pipérazin-1-yl)-propanoate de méthyle dans 20ml de dichlorométhane et le milieu réactionnel est agité à température ambiante pendant 3h . Après addition d'eau, le produit est extrait au dichlorométhane. La phase organique est lavée à l'eau , séchée sur sulfate de magnésium, filtrée et concentrée.
Le résidu obtenu est purifié par chromatographie sur gel de silice élué avec un mélange heptane/ acétate d'éthyle 8/2. 0,9g (75%) de (S)-3-(4-benzyloxy-benzènesulfonylamino)-2-(4-méthanesulfonyl-piperazin-1-yl)-propanoate de méthyle sont obtenus sous forme d'un solide blanc.

### 3.7 : acide (S)-3-(4-benzyloxy-benzènesulfonylamino)-2-(4-méthanesulfonyl-piperazin-1-yl)-propanoique

2,6ml (2,6 mmoles) d'une solution aqueuse d'hydroxyde de lithium de concentration 1M sont additionnés à une solution de 900mg (1,8 mmoles) de (S)-3-(4-benzyloxy-benzènesulfonylamino)-2-(4-méthanesulfonyl-pipérazin-1-yl)-propanoate de méthyle dans 20ml de tétrahydrofurane et 0,5ml d'eau. Le milieu réactionnel est agité à température ambiante pendant 18h puis le THF est évaporé sous vide. 2,8ml d'une solution aqueuse d'acide acétique de concentration 1M puis 30ml d'eau sont additionnés et le produit précipite. La suspension est agitée 30min à 100°C puis ramenée à température ambiante, filtrée et séchée sous vide. 750mg (86%) d' acide (S)-3-(4-benzyloxy-benzènesulfonylamino)-2-(4-méthanesulfonyl-pipérazin-1-yl)-propanoique sont obtenus sous forme d'un solide blanc.

### 3.8: (S)-3-(4-benzyloxy-benzènesulfonylamino)-N-hydroxy-2-(4-méthanesulfonyl-pipérazin-1-yl)-propionamide

224mg (1,7 mmoles) de 1-hydroxybenzotriazole et 318mg (1,7 mmoles) du chlorhydrate de 1-éthyl-3-(3-diméthylaminopropyl) carbodiimide sont additionnés successivement à 750mg (1,5 mmoles) d'acide (S)-3-(4-benzyloxy-benzènesulfonylamino)-2-(4-méthanesulfonyl-piperazin-1-yl)-propanoique dans 20ml de diméthylformamide. Après agitation à température ambiante pendant 20min, une solution de 244mg (1,7 mmoles) de O-tert-butyldiméthylsilylhydroxylamine dans 3ml de diméthylformamide est ajoutée. Le milieu réactionnel est ensuite agité à température ambiante pendant 18h puis 2ml d'une solution aqueuse saturée d'hydrogénocarbonate de sodium et enfin 2ml d'eau sont ajoutés. Après extraction à l'acétate d'éthyle, la phase organique est lavée avec une solution aqueuse saturée d'hydrogénocarbonate de sodium, séchée sur sulfate de magnésium, filtrée et concentrée. Le résidu brut obtenu est repris dans 15ml d'acétate d'éthyle , chauffé à 70°C puis ramené à température ambiante, filtré, séché sous vide.
300mg (34%) de (S)-3-(4-benzyloxy-benzènesulfonylamino)-N-hydroxy-2-(4-méthanesulfonyl-pipérazin-1-yl)-propionamide sont obtenus sous forme d'un solide blanc de point de fusion 165°C. RMN ¹H (δ, DMSO): 2,40-2,50 (m, 2H); 2,50-2,60 (m, 2H); 2,84 (s, 3H), 3,00-3,05 (m, 4H); 3,06-3,09 (m, 2H); 3,34 (s, 1H); 5,19 (s, 2H); 7,19 (d, J=8,4Hz, 2H); 7,30-7,34 (m, 1H); 7,35-7,47 (m, 5H); 7,73 (d, J=8,4Hz, 2H); 8,93 (s, 1 H); 10,65 (s, 1 H).

### Exemple 4 : (S)-3-[(4-benzyloxy-benzènesulfonyl)-méthyl-amino]-N-hydroxy-2-(4-méthanesulfonyl-pipérazin-1-yl)-propionamide.

### 4.1: (S)-3-[(4-benzyloxy-benzènesulfonyl)-méthyl-amino]-2-(4-méthanesulfonyl-pipérazin-1-yl)-propanoate de méthyle

300mg (1,9 mmoles) de carbonate de potassium puis 0,2ml (3,1 mmoles) d' iodure de méthyle sont additionnés sur une solution de 800mg (1,6 mmoles) de (S)-3-(4-benzyloxy-benzènesulfonylamino)-2-(4-methanesulfonyl-pipérazin-1-yl)-propanoate méthyle (préparé comme décrit en 3.6) dans 15ml de diméthylformamide. Le milieu réactionnel est ensuite agité à température ambiante pendant 20h, hydrolysé puis dilué par de l'acétate d'éthyle. Le produit est extrait à l'acétate d'éthyle. Les phases organiques sont lavées avec de l'eau, séchées sur sulfate de magnésium et filtrées.
Le filtrat est concentré sous vide pour donner 820mg (100%) de (S)-3-(4-benzyloxy-benzènesulfonylamino)-2-(4-méthanesulfonyl-pipérazin-1-yl)-propanoate méthyle sous la forme d'un solide blanc.

### 4.2: acide (S)-3-[(4-benzyloxy-benzènesulfonyl)-méthyl-amino]-2-(4-méthanesulfonyl-pipérazin-1-yl)-propanoique:

De manière analogue à l'exemple 3.7, à partir de 820mg (1,6 mmoles) de (S)-3-[(4-benzyloxy-benzènesulfonyl)-méthyl-amino]-2-(4-méthanesulfonyl-pipérazin-1-yl)- propanoate de méthyle, 720mg (90%) d' acide (S)-3-[(4-benzyloxy-benzènesulfonyl)-méthyl-amino]-2-(4-méthanesulfonyl-pipérazin-1-yl)-propanoique sont obtenus sous forme d'un solide blanc.

### 4.3: (S)-3-[(4-benzyloxy-benzènesulfonyl)-méthyl-amino]-N-hydroxy-2-(4-méthanesulfonyl-pipérazin-1-yl)-propionamide

De manière analogue à l'exemple 3.8, à partir de 720mg (1,4 mmoles) d' acide (S)-3-[(4-benzyloxy-benzènesulfonyl)-méthyl-amino]-2-(4-méthanesulfonyl-pipérazin-1-yl)-propanoique, 360mg (49%) de (S)-3-[(4-benzyloxy-benzènesulfonyl)-méthyl-amino]-N-hydroxy-2-(4-méthanesulfonyl-pipérazin-1-yl)-propionamide sont obtenus sous forme d'un solide blanc de point de fusion 110°C. RMN ¹H (δ, DMSO): 2,58-2,63 (m, 2H); 2,65 (s, 3H); 2,67-2,73 (m, 2H); 2,86 (s, 3H); 2,98-3,05 (m, 4H); 3,05-3,09 (m, 1H); 3,24-3,25 (m, 1H); 3,28-3,31 (m, 1H); 5,21 (s, 2H); 7,24 (d, J=8,9Hz, 2H); 7,34-7,44 (m, 3H); 7,48 (d, J=7,2Hz, 2H); 7,72 (d, J=8,9Hz, 2H); 8,99 (s, 1 H); 10,69 (s, 1 H).

### Exemple 5: (S)-N-hydroxy-2-(4-méthanesulfonyl-pipérazin-1-yl)-3-[4-(2-méthyl-quinolin-4-ylméthoxy)-benzènesulfonylamino]-propionamide.

### 5.1: (S)-3-(4-hydroxy-benzènesulfonylamino)-2-(4-méthanesulfonyl-pipérazin-1-yl)-propanoate de méthyle

Une solution de 2,0g (3,9 mmoles) de (S)-3-(4-benzyloxy-benzènesulfonylamino)-2-(4-méthanesulfonyl-pipérazin-1-yl)-propanoate de méthyle (préparé comme décrit dans l'exemple 3.6) dans 60ml d'éthanol, 30ml de dioxanne et 0,5ml d'acide acétique glacial est dégazée sous flux d'azote puis 200mg (10% massique) de palladium sur charbon 10% en suspension dans 3ml de dioxanne sont additionnés. Le milieu réactionnel est placé sous une atmosphère d'hydrogène et agité à température ambiante pendant 18h. Après filtration sur célite, le filtrat est hydrolysé puis le produit est extrait à l'acétate d'éthyle. La phase organique est lavée à l'eau puis par une solution aqueuse saturée de chlorure de sodium, séchée sur sulfate de magnésium, filtrée, concentrée sous vide. 1,65g (100%) de (S)-3-(4-hydroxy-benzènesulfonylamino)-2-(4-méthanesulfonyl-pipérazin-1-yl)-propanoate de méthyle sont obtenus sous forme d'un solide blanc.

### 5.2 : (S)-2-(4-méthanesulfonyl-pipérazin-1-yl)-3-[4-(2-méthyl-quinolin-4-ylméthoxy)-benzènesulfonylamino]-propanoate de méthyle

280mg (0,85 mmoles) de carbonate de césium suivis de 160mg (0,85 mmoles) de 4-chlorométhyl-2-méthyl-quinoline et de 15mg d' iodure de potassium sont additionnés sur une solution de 300mg (0,71 mmoles) de (S)-3-(4-Hydroxy-benzenesulfonylamino)-2-(4-méthanesulfonyl-pipérazin-1-yl)-propanoate de méthyle dans 10ml d'acétone. Le milieu réactionnel est agité à température ambiante pendant 18h, filtré, concentré sous vide. Le produit brut est purifié par chromatographie sur gel de silice élué avec un mélange heptane/acétate d'éthyle 40/60. 130mg (32%) de (S)-2-(4-méthanesulfonyl-pipérazin-1-yl)-3-[4-(2-méthyl-quinolin-4-ylméthoxy)-benzènesulfonylamino]-propanoate de méthyle sont obtenus sous la forme d'un solide blanc.

### 5.3 : acide (S)-2-(4-méthanesulfonyl-pipérazin-1-yl)-3-[4-(2-méthyl-quinolin-4-ylméthoxy)-benzènesulfonylamino]-propanoique

De manière analogue à l'exemple 3.7, à partir de 130mg (0,2 mmoles) de (S)-2-(4-méthanesulfonyl-pipéridin-1-yl)-3-[4-(2-méthyl-quinolin-4-ylméthoxy)-benzènesulfonylamino]-propanoate de méthyle, 120mg (99%) d' acide (S)-2-(4-méthanesulfonyl-pipéridin-1-yl)-3-[4-(2-méthyl-quinolin-4-ylméthoxy)-benzènesulfonylamino]-propanoique sont obtenus sous forme d'un solide blanc.

### 5.4: (S)-N-hydroxy-2-(4-méthanesulfonyl-pipérazin-1-yl)-3-[4-(2-méthyl-quinolin-4-ylméthoxy)-benzènesulfonylamino]-propionamide

De manière analogue à l'exemple 3.8, à partir de 123mg (0,2 mmoles) d'acide (S)-2-(4-méthanesulfonyl-piperazin-1-yl)-3-[4-(2-méthyl-quinolin-4-ylméthoxy)-benzènesulfonylamino]-propanoique, 90mg (69%) de (S)-N-hydroxy-2-(4-méthanesulfonyl-pipérazin-1-yl)-3-[4-(2-méthyl-quinolin-4-ylméthoxy)-benzènesulfonylamino]-propionamide sont obtenus sous forme d'un solide de point de fusion 185°C.
RMN ¹H (δ, DMSO) : 2,54-2,60 (m, 4H); 2,72 (s, 3H); 2,88 (s, 3H), 2,88-2,93 (m, 1H); 3,01-3,05 (m, 1 H); 3,06-3,12 (m, 4H); 3,13-3,16 (t, J=7Hz, 1 H); 5,76 (s, 2H); 7,38 (d, J=8Hz, 2H); 7,57 (s, 1 H); 7,61-7,66 (m, 2H); 7,78-7,85 (m, 3H); 8,02 (d, J=8,2Hz, 1H); 8,15 (d, J= 8,2Hz, 1H); 8,98 (s, 1H); 10,71 (s, 1 H).

### Exemple 6 : (S)-N-hydroxy-2-(4-méthanesulfonyl-pipérazin-1-yl)-3-[4-(naphthalen-1-ylméthoxy)-benzènesulfonylamino]-propionamide.

### 6.1 : (S)-2-(4-méthanesulfonyl-pipérazin-1-yl)-3-[4-(2-méthyl-quinolin-4-ylméthoxy)-benzènesulfonylamino]-propanoate de méthyle

De manière analogue à l'exemple 5.2, à partir de 160mg (0,9 mmoles) de 4-chlorométhyl-2-méthyl-quinoline et de 300mg (0,7 mmoles) de (S)-3-(4-hydroxy-benzènesulfonylamino)-2-(4-méthanesulfonyl-pipérazin-1-yl)-propanoate de méthyle (préparé comme décrit en 5.1), 130mg (32%) de (S)-2-(4-méthanesulfonyl-pipérazin-1-yl)-3-[4-(2-méthyl-quinolin-4-ylméthoxy)-benzènesulfonylamino]-propanoate de méthyle sont obtenus sous forme d'un solide blanc.

### 6.2 : acide (S)-2-(4-méthanesulfonyl-pipérazin-1-yl)-3-[4-(naphthalen-1-ylméthoxy)-benzènesulfonylamino]-propanoique

De manière analogue à l'exemple 3.7, à partir de 240mg (0,6 mmoles) de (S)-2-(4-méthanesulfonyl-pipérazin-1-yl)-3-[4-(naphthalen-1-ylméthoxy)-benzènesulfonylamino]-propanoate de méthyle, 210mg (91%) d' acide (S)-2-(4-méthanesulfonyl-pipérazin-1-yl)-3-[4-(naphthalen-1-ylméthoxy)-benzènesulfonylamino]-propanoique sont obtenus sous forme d'un solide blanc.

### 6.3 : (S)-N-hydroxy-2-(4-méthanesulfonyl-pipérazin-1-yl)-3-[4-(naphthalen-1-ylméthoxy)-benzènesulfonylamino]-propionamide

De manière analogue à l'exemple 3.8, à partir de 210mg (0,4 mmoles) d'acide (S)-2-(4-méthanesulfonyl-pipérazin-1-yl)-3-[4-(naphthalen-1-ylméthoxy)-benzènesulfonylamino]-propanoique, 70mg (33%) de (S)-N-hydroxy-2-(4-méthanesulfonyl-pipérazin-1-yl)-3-[4-(naphthalen-1-ylméthoxy)-benzènesulfonylamino]-propionamide sont obtenus sous forme d'un solide beige de point de fusion 148°C.
RMN ¹H (δ, DMSO): 2,45 (m, 2H); 2,60 (m, 2H); 2,85 (s, 3H); 2,90-3,05 (m, 4H); 3,06-3,15 (m, 2H); 3,35 (s, 1 H); 5,66 (s, 2H); 7,30 (d, J=8,4Hz, 2H); 7,50-7,60 (m, 4H); 7,70 (d, J=6,2Hz, 1H); 7,77 (d, J=8.2Hz, 2H); 7,95-8,05 (m, 2H); 8,10 (d, J=6,4Hz, 1 H); 8,94 (s, 1 H); 10,70 (s, 1 H).

### Exemple 7 : (S)-N-hydroxy-2-(4-méthanesulfonyl-pipérazin-1-yl)-3-(4-propoxy-benzènesulfonylamino)-propionamide.

### 7.1 : (S)-2-(4-méthanesulfonyl-pipérazin-1-yl)-3-(4-propoxy-benzènesulfonylamino)-propanoate de méthyle

De manière analogue à l'exemple 5.2, à partir de 0,1ml (1,3 mmoles) de 1-bromopropane et de 400mg (0,95 mmoles) de (S)-3-(4-hydroxy-benzènesulfonylamino)-2-(4-méthanesulfonyl-pipérazin-1-yl)-propanoate de méthyle (préparé comme décrit en 5.1), 220mg (50%) de (S)-2-(4-méthanesulfonyl-pipérazin-1-yl)-3-(4-propoxy-benzènesulfonylamino)-propanoate de méthyle sont obtenus sous forme d'une huile incolore.

### 7.2 : acide (S)-2-(4-méthanesulfonyl-pipérazin-1-yl)-3-(4-propoxy-benzènesulfonylamino)-propanoique

De manière analogue à l'exemple 3.7, à partir de 220mg (0,5 mmoles) de (S)-2-(4-méthanesulfonyl-pipérazin-1-yl)-3-(4-propoxy-benzènesulfonylamino)-propanoate de méthyle, 190mg (90%) d' acide (S)-2-(4-méthanesulfonyl-pipérazin-1-yl)-3-(4-propoxy-benzènesulfonylamino)-propanoique sont obtenus sous forme d'un solide blanc.

### 7.3 : (S)-N-hydroxy-2-(4-méthanesulfonyl-pipérazin-1-yl)-3-(4-propoxy-benzènesulfonylamino)-propionamide

De manière analogue à l'exemple 3.8, à partir de 190mg (0,4 mmoles) d'acide (S)-2-(4-méthanesulfonyl-pipérazin-1-yl)-3-(4-propoxy-benzènesulfonylamino)-propanoique, 30mg (16%) de (S)-N-hydroxy-2-(4-méthanesulfonyl-pipérazin-1-yl)-3-(4-propoxy-benzènesulfonylamino)-propionamide sont obtenus sous forme d'un solide blanc de point de fusion 137°C.
RMN ¹H (δ, DMSO) : 0,91 (t, J=7,3Hz, 3H); 1,63-1,73 (m, 2H); 2,45 (m, 2H); 2,55 (m, 2H); 2,77 (s, 3H); 2,82 (m, 1 H); 2,83-2,95 (m, 4H); 2,95-3,05 (m, 2H); 3,94 (t, J=6,4Hz, 2H); 7,03 (d, J=8,7Hz, 2H); 7,38 (m, 1 H); 7,65 (d, J=8,7Hz, 2H); 8,85 (s, 1 H); 10,58 (s, 1 H).

### Exemple 8 : (S)-3-[4-(3-cyano-benzyloxy)-benzènesulfonylamino]-N-hydroxy-2-(4-méthanesulfonyl-pipérazin-1-yl)-propionamide.

### 8.1 : (S)-3-[4-(3-cyano-benzyloxy)-benzènesulfonylamino]-2-(4-méthanesulfonyl-pipérazin-1-yl)-propanoate de méthyle

De manière analogue à l'exemple 5.2, à partir de 205mg (1 mmole) de 3-(bromométhyl)benzonitrile et de 400mg (0,95 mmoles) de (S)-3-(4-hydroxy-benzènesulfonylamino)-2-(4-méthanesulfonyl-pipérazin-1-yl)-propanoate de méthyle (préparé comme décrit dans l'exemple 5.1), 295mg (58%) de (S)-3-[4-(3-cyano-benzyloxy)-benzènesulfonylamino]-2-(4-méthanesulfonyl-pipérazin-1-yl)-propanoate de méthyle sont obtenus sous forme d'un solide blanc.

### 8.2 : acide (S)-3-[4-(3-cyano-benzyloxy)-benzènesulfonylamino]-2-(4-méthanesulfonyl-pipérazin-1-yl)-propanoique

De manière analogue à l'exemple 3.7, à partir de 295mg (0,5 mmoles) de (S)-3-[4-(3-cyano-benzyloxy)-benzènesulfonylamino]-2-(4-méthanesulfonyl-pipérazin-1-yl)-propanoate de méthyle, 270mg (94%) d'acide (S)-3-[4-(3-cyano-benzyloxy)-benzènesulfonylamino]-2-(4-méthanesulfonyl-pipérazin-1-yl)-propanoique sont obtenus sous forme d'un solide blanc.

### 8.3 : (S)-3-[4-(4-cyano-benzyloxy)-benzènesulfonylamino]-N-hydroxy-2-(4-méthanesulfonyl-pipérazin-1-yl)-propionamide

De manière analogue à l'exemple 3.8, à partir de 264mg (0,5 mmoles) d'acide (S)-3-[4-(3-cyano-benzyloxy)-benzènesulfonylamino]-2-(4-méthanesulfonyl-pipérazin-1-yl)-propanoique, 107mg (40%) de (S)-3-[4-(3-cyano-benzyloxy)-benzènesulfonylamino]-N-hydroxy-2-(4-méthanesulfonyl-pipérazin-1-yl)-propionamide sont obtenus sous forme d'une poudre beige de point de fusion 108°C.
RMN ¹H (δ, DMSO) : 2,55 (m, 4H); 2,84 (s, 3H); 2,95-3,05 (m, 4H); 3,10 (t, J=6,4Hz, 1 H); 3,34 (m; 2H); 5,26 (s, 2H); 7,22 (d, J=8,6Hz, 2H); 7,50 (s, 1 H); 7,64 (t, J=7,6Hz, 1 H); 7,76 (d, J=8,6Hz, 2H); 7,83 (t, J=8Hz, 2H); 7,96 (s, 1 H); 8,93 (s, 1 H); 10,66 (s, 1 H).

### Exemple 9 : (S)-3-[4-(4-cyano-benzyloxy)-benzènesulfonylamino]-N-hydroxy-2-(4-méthanesulfonyl-pipérazin-1-yl)-propionamide.

### 9.1 : (S)-3-[4-(4-cyano-benzyloxy)-benzènesulfonylamino]-2-(4-méthanesulfonyl-pipérazin-1-yl)-propanoate de méthyle

De manière analogue à l'exemple 5.2, à partir de 400mg (1 mmole) de (S)-3-(4-hydroxy-benzènesulfonylamino)-2-(4-méthanesulfonyl-pipérazin-1-yl)-propanoate de méthyle (préparé comme décrit en 5.1) et de 205mg (1,1 mmoles) de 4-(bromométhyl)benzonitrile, 229mg (45%) de (S)-3-[4-(4-cyano-benzyloxy)-benzènesulfonylamino]-2-(4-méthanesulfonyl-pipérazin-1-yl)-propanoate de méthyle sont obtenus sous forme d'un solide blanc.

### 9.2 : acide (S)-3-[4-(4-cyano-benzyloxy)-benzènesulfonylamino]-2-(4-méthanesulfonyl-pipérazin-1-yl)-propanoique

De manière analogue à l'exemple 3.7, à partir de 229mg (0,4 mmoles) de (S)-3-[4-(4-cyano-benzyloxy)-benzènesulfonylamino]-2-(4-méthanesulfonyl-pipérazin-1-yl)-propanoate de méthyle, 202mg (91%) d'acide (S)-3-[4-(4-cyano-benzyloxy)-benzènesulfonylamino]-2-(4-méthanesulfonyl-pipérazin-1-yl)-propanoique sont obtenus sous forme d'un solide blanc.

### 9.3 : (S)-3-[4-(4-cyano-benzyloxy)-benzènesulfonylamino]-N-hydroxy-2-(4-méthanesulfonyl-pipérazin-1-yl)-propionamide

De manière analogue à l'exemple 3.8, à partir d'acide 197mg (0,4 mmoles) de (S)-3-[4-(4-cyano-benzyloxy)-benzènesulfonylamino]-2-(4-méthanesulfonyl-piperazin-1-yl)-propanoique, 81mg (40%) de (S)-3-[4-(4-cyano-benzyloxy)-benzènesulfonylamino]-N-hydroxy-2-(4-méthanesulfonyl-pipérazin-1-yl)-propionamide sont obtenus sous forme d'une poudre beige de point de fusion 109°C.
RMN ¹H (δ, DMSO) : 2,50-2,60 (m, 4H); 2,84 (s, 3H); 2,96 -3,01 (m, 4H); 3,09 (t, J=7Hz, 1 H); 3,34 (s, 2H); 5,32 (s, 2H); 7,22 (d, J=8,8Hz, 2H); 7,50 (m, 1 H); 7,66 (d, J=8,1 Hz, 2H); 7,75 (d, J=8,8Hz, 2H); 7,89 (d, J=8,1 Hz, 2H); 8,93 (s, 1 H); 10,66 (s, 1 H).

### Exemple 10 : 4-{(S)-1-hydroxycarbamoyl-2-[4-(2-méthyl-quinolin-4-ylméthoxy)-benzène sulfonylamino]-éthyl}-pipérazine-1-carboxylate de benzyle.

### 10.1 : bis-(2-chloroéthyl)-carbamate de benzyle

13,2ml (92 mmoles) de chloroformate de benzyle sont ajoutés lentement à une solution, refroidie à 0°C, de 15g (84 mmoles) de chlorhydrate de bis(2-chloroéthylamine), 26ml (185 mmoles) de triéthylamine dans 200ml de dichlorométhane et 70ml de tétrahydrofuranne préalablement agitée pendant 15min puis filtrée pour enlever le chlorure de triéthylammonium. Le milieu réactionnel est agité à température ambiante pendant 18h. Après addition d'eau, le milieu réactionnel est extrait à l'acétate d'éthyle. La phase organique est séchée sur sulfate de magnésium, filtrée, évaporée. 20g de résidu brut sont obtenus et purifiés par chromatographie sur gel de silice élués avec un mélange heptane / acétate d'éthyle 8/2. 6g (26%) de bis-(2-chloroéthyl)-carbamate de benzyle sont ainsi obtenus.

### 10.2 : 4-((S)-2-tert-butoxycarbonylamino-1-méthoxycarbonyl-éthyl)-pipérazine-1-carboxylate de benzyle

Une solution de 5,5g (20 mmoles) de bis-(2-chloroéthyl)-carbamate de benzyle et 5,1g (20 mmoles) de chlorhydrate de 2-amino-3-tert-butoxy-propanoate de méthyle dans 40ml de diisopropyléthylamine est chauffée à 127°C pendant 18h. Après refroidissement, le milieu réactionnel est évaporé à sec. 17g de résidu brut sont obtenus et purifiés par chromatographie sur gel de silice élués avec un mélange heptane / acétate d'éthyle 9/1 jusqu'à 4/6. 1,6g (19%) de 4-((S)-2-tert-butoxycarbonylamino-1-méthoxycarbonyl-éthyl)-pipérazine-1-carboxylate de benzyle.

### 10.3 : dichlorhydrate de 4-(2-amino-1-méthoxycarbonyl-éthyl)-pipérazine-1-carboxylate de benzyle

Une solution de 1,45g (3,4 mmoles) de 4-((S)-2-tert-butoxycarbonylamino-1-méthoxycarbonyl-éthyl)-pipérazine-1-carboxylate de benzyle dans 3,5ml d'une solution d'acide chlorhydrique dans l'isopropanol de concentration 5-6N et 10ml de méthanol est chauffée à 40°C pendant 3h puis évaporée. Le résidu est repris dans l'éther diéthylique, filtré. 1,2g (90%) de dichlorhydrate de 4-(2-amino-1-méthoxycarbonyl-éthyl)-pipérazine-1-carboxylate de benzyle sont obtenus sous forme d'un solide.

### 10.4 : 4-[(S)-2-(4-hydroxy-benzènesulfonylamino)-1-méthoxycarbonyl-éthyl]-pipérazine-1-carboxylate de benzyle

2,1ml (15 mmoles) de triéthylamine puis 920mg (5 mmoles) de chlorure de 4-hydroxy-benzènesulfonyle dans 20ml de dichlorométhane sont additionnés goutte à goutte à une solution de 1,1g (3 mmoles) de dichlorhydrate de 4-(2-amino-1-méthoxycarbonyl-éthyl)-pipérazine-1-carboxylate de benzyle dans 30ml de dichlorométhane, préalablement refroidie à 0°C. Le milieu réactionnel est ensuite agité à température ambiante pendant 18h. Après addition d'eau, le milieu réactionnel est extrait par du dichlorométhane. La phase organique est à l'eau puis séchée sur sulfate de magnésium, filtrée et concentrée sous vide. Le résidu brut obtenu est purifié par chromatographie sur gel de silice élué avec un mélange heptane/acétate d'éthyle 50/50. 60mg (46%) de 4-[2-(4-hydroxy-benzènesulfonylamino)-1-méthoxycarbonyl-éthyl]-pipérazine-1-carboxylate de benzyle sont obtenus sous forme d'un solide blanc.

### 10.5 : 4-{(S)-1-méthoxycarbonyl-2-[4-(2-méthyl-quinolin-4-ylméthoxy)-benzènesulfonylamino]-éthyl}-pipérazine-1-carboxylate de benzyle

De manière analogue à l'exemple 5.2, à partir de 260mg (1,4 mmoles) de 4-chlorométhyl-2-méthyl-quinoline et de 600mg (1,3 mmoles) de 4-[(S)-2-(4-hydroxy-benzènesulfonylamino)-1-méthoxycarbonyl-éthyl]-pipérazine-1-carboxylate de benzyle, 320mg (40%) de 4-{(S)-1-méthoxycarbonyl-2-[4-(2-méthyl-quinolin-4-ylméthoxy)-benzènesulfonylamino]-éthyl}-pipérazine-1-carboxylate de benzyle sont obtenus sous forme d'un solide blanc.

### 10.6 : 4-{(S)-1-carboxy-2-[4-(2-méthyl-quinolin-4-ylméthoxy benzènesulfonylamino]-éthyl}-pipérazine-1-carboxylate de benzyle

De manière analogue à l'exemple 3.7, à partir de 160mg (0,25 mmoles) de 4-{(S)-1-méthoxycarbonyl-2-[4-(2-méthylquinolin-4-ylméthoxy)-benzènesulfonylamino]-éthyl}-pipérazine-1-carboxylate de benzyle, 135mg (87%) de 4-{(S)-1-carboxy-2-[4-(2-méthyl-quinolin-4-ylméthoxy benzènesulfonylamino]-éthyl}-pipérazine-1-carboxylate de benzyle sont obtenus sous forme d'un solide beige.

### 10.7 : 4-{(S)-1-hydroxycarbamoyl-2-[4-(2-méthyl-quinolin-4-ylméthoxy)-benzènesulfonylamino]-ethyl]-piperazine-1-carboxylate de benzyle

De manière analogue à l'exemple 3.8, à partir de 135mg (0,2 mmoles) de 4-{(S)-1-carboxy-2-[4-(2-méthyl-quinolin-4-ylméthoxy benzènesulfonylamino]-éthyl}-pipérazine-1-carboxylate de benzyle, 115mg (82%) de 4-{(S)-1-hydroxycarbamoyl-2-[4-(2-méthyl-quinolin-4-ylméthoxy)-benzènesulfonylamino]-éthyl}-pipérazine-1-carboxylate de benzyle sont obtenus sous forme d'un solide blanc de point de fusion 162°C.
RMN ¹H (δ, DMSO): 2,35-2,45 (m, 4H); 2,70 (s, 3H); 2,80-2,90 (m, 1H); 2,95-3,05 (m, 1H); 3-05-3,10 (m, 1 H); 3,25-3,40 (m, 4H); 5,05 (s, 2H); 5,74 (s, 2H); 7,29-7,40 (m, 7H); 7,55 (m, 1 H); 7,60-7,70 (m, 2H); 7,79 (d, J=8,8Hz, 3H); 8,01 (d, J=8Hz, 1 H); 8,14 (d, J=8Hz, 1 H); 8,91 (s, 1 H); 10,67 (s, 1 H).

### Exemple 11 : (S)-N-hydroxy-2-(4-méthanesulfonyl-pipérazin-1-yl)-3-[4-(2-phényl-pyridin-4-ylméthoxy)-benzènesulfonylamino]-propionamide.

### 11.1 (S)-2-(4-méthanesulfonyl-pipérazin-1-yl)-3-[4-(2-phényl-pyridin-4-ylméthoxy)-benzènesulfonylamino]-propanoate de méthyle

0,23ml (1,4 mmoles) de diéthylazodicarboxylate sont ajoutés lentement à une solution de 400mg (0,9 mmoles) de (S)-3-(4-hydroxy-benzènesulfonylamino)-2-(4-méthanesulfonyl-pipérazin-1-yl)-propanoate de méthyle (préparé comme décrit dans l'exemple 5.1), 193mg (1,0 mmole) de (2-phényl-pyridin-4-yl)-méthanol et 373mg (1,4 mmoles) de triphénylphosphine dans 4ml de tétrahydrofuranne. Le mélange réactionnel est agité une heure à température ambiante puis évaporé à sec. Le résidu obtenu est purifié par chromatographie sur gel de silice élué avec un mélange heptane/acétate d'éthyle 60/40. 318mg (57%) de (S)-2-(4-méthanesulfonyl-pipérazin-1-yl)-3-[4-(2-phényl-pyridin-4-ylméthoxy)-benzènesulfonylamino]-propanoate de méthyle sont obtenus sous forme d'une poudre blanche.

### 11.2 : acide (S)-2-(4-méthanesulfonyl-pipérazin-1-yl)-3-[4-(2-phényl-pyridin-4-ylméthoxy)-benzènesulfonylamino]-propanoique

De manière analogue à l'exemple 3.7, à partir de 317mg (0,5 mmoles) de (S)-2-(4-méthanesulfonyl-pipérazin-1-yl)-3-[4-(2-phényl-pyridin-4-ylméthoxy)-benzènesulfonylamino]-propanoate de méthyle, 298mg (96%) d'acide (S)-2-(4-méthanesulfonyl-pipérazin-1-yl)-3-[4-(2-phényl-pyridin-4-ylméthoxy)-benzènesulfonylamino]-propanoique sont obtenus sous forme d'un solide blanc.

### 11.3 : (S)-N-hydroxy-2-(4-méthanesulfonyl-piperazin-1-yl)-3-[4-(2-phenyl-pyridin-4-ylmethoxy)-benzenesulfonylamino]-propionamide

De manière analogue à l'exemple 3.8, à partir de 293mg (0,5 mmoles) d'acide (S)-2-(4-méthanesulfonyl-pipérazin-1-yl)-3-[4-(2-phényl-pyridin-4-ylméthoxy)-benzènesulfonylamino]-propanoique, 64mg (21%) de (S)-N-hydroxy-2-(4-méthanesulfonyl-pipérazin-1-yl)-3-[4-(2-phényl-pyridin-4-ylméthoxy)-benzènesulfonylamino]-propionamide sont obtenus sous forme d'une poudre blanche de point de fusion 100°C.
RMN ¹H (δ, DMSO): 2,52-2,59 (m, 4H); 2,84 (s, 3H); 2,85-2,90 (m, 1H); 2,90-3,00 (m, 1H); 3,00-3,08 (m, 4H); 3,10 (t, J=7,0Hz, 1H); 5,35 (s, 2H); 7,26 (d, J=8,9Hz, 2H); 7,42 (m, 1H); 7,45-7,55 (m, 4H); 7,78 (d, J=8,8Hz, 2H); 8,03 (s, 1H); 8,10 (d, J=7,0Hz, 2H); 8,69 (d, J=5,0Hz, 1H); 8,93 (s, 1H); 10,66 (s, 1 H).

### Exemple 12 : (R)-N-hydroxy-2-(4-méthanesulfonyl-pipérazin-1-yl)-3-[4-(2-méthyl-quinolin-4-ylméthoxy)-benzènesulfonylamino]-propionamide.

### 12.1 : (R)-3-tert-butoxycarbonylamino-2-(4-méthanesulfonyl-pipérazin-1-yl)-propanoate de méthyle

De manière analogue à l'exemple 3.2, à partir de 3,8g (17 mmoles) de N,N-bis-(2-chloro-éthyl)-méthanesulfonamide (préparé comme décrit en 3.1) et 4g (16 mmoles) de chlorhydrate de 2- (R) amino-3-tert-butoxy-propanoate de méthyle commercial, 2,6g (46%) de (R)-3-tert-butoxycarbonylamino-2-(4-méthanesulfonyl-pipérazin-1-yl)-propanoate de méthyle sont obtenus sous forme d'un solide jaune clair.

### 12.2 : dichlorhydrate de (R)-3-amino-2-(4-méthanesulfonyl-pipérazin-1-yl)-propanoate de méthyle

De manière analogue à l'exemple 3.3, à partir de 2,5g (7 mmoles) de (R)-3-tert-butoxycarbonylamino-2-(4-méthanesulfonyl-pipérazin-1-yl)-propanoate de méthyle, 2,3g (100%) du dichlorhydrate de (R)-3-amino-2-(4-méthanesulfonyl-piperazin-1-yl)-propanoate de méthyle sont obtenus.

### 12.3 : (R)-3-(4-benzyloxy-benzènesulfonylamino)-2-(4-méthanesulfonyl-pipérazin-1-yl)-propanoate de méthyle

De manière analogue à l'exemple 3.6, à partir de 2,4g (8,4 mmoles) de chlorure de 4-benzyloxy-benzènesulfonyle (préparé comme décrit dans l'exemple 3.5) et de 2,3g (7,6 mmoles) de dichlorhydrate de (R)-3-amino-2-(4-méthanesulfonyl-pipérazin-1-yl)-propanoate de méthyle, 3g (77%) de (R)-3-(4-benzyloxy-benzènesulfonylamino)-2-(4-méthanesulfonyl-pipérazin-1-yl)-propanoate de méthyle sont obtenus sous forme d'un solide.

### 12.4 : (R)-3-(4-hydroxy-benzènesulfonylamino)-2-(4-méthanesulfonyl-pipérazin-1-yl)-propanoate de méthyle

De manière analogue à l'exemple 5.1, à partir de 3g (5,9 mmoles) de (R)-3-(4-benzyloxy-benzènesulfonylamino)-2-(4-méthanesulfonyl-pipérazin-1-yl)-propanoate de méthyle, 2g (80%) de (R)-3-(4-hydroxy-benzènesulfonylamino)-2-(4-méthanesulfonyl-pipérazin-1-yl)-propanoate de méthyle sont obtenus sous forme d'un solide blanc.

### 12.5 : (R)-2-(4-méthanesulfonyl-pipérazin-1-yl)-3-[4-(2-méthyl-quinolin-4-ylméthoxy)-benzènesulfonylamino]-propanoate de méthyle

De manière analogue à l'exemple 5.2, à partir de 1g (2,4 mmoles) de (R)-3-(4-hydroxy-benzènesulfonylamino)-2-(4-méthanesulfonyl-pipérazin-1-yl)-propanoate de méthyle et de 500mg (2,6 mmoles) de 4-chlorométhyl-2-méthylquinoline, 740mg (53%) de (R) -2-(4-méthanesulfonyl-pipérazin-1-yl)-3-[4-(2-méthyl-quinolin-4-ylméthoxy)-benzènesulfonylamino]-propanoate de méthyle sont obtenus sous forme d'un solide.

### 12.6 : acide (R)-2-(4-méthanesulfonyl-pipérazin-1-yl)-3-[4-(2-méthyl-quinolin-4-ylméthoxy)-benzènesulfonylamino]-propanoique

De manière analogue à l'exemple 3.7, à partir de 740mg (1,3 mmoles) de (R) -2-(4-méthanesulfonyl-pipérazin-1-yl)-3-[4-(2-méthyl-quinolin-4-ylméthoxy)-benzènesulfonylamino]-propanoate de méthyle, 622mg (86%) d' acide (R) -2-(4-méthanesulfonyl-pipérazin-1-yl)-3-[4-(2-méthyl-quinolin-4-ylméthoxy)-benzènesulfonylamino]-propanoique sont obtenus.

### 12.7 : (R)-N-hydroxy-2-(4-méthanesulfonyl-pipérazin-1-yl)-3-[4-(2-méthyl-quinolin-4-ylméthoxy)-benzènesulfonylamino]-propionamide

De manière analogue à l'exemple 3.8, à partir de 620mg (1,1 mmoles) d'acide (R) -2-(4-méthanesulfonyl-pipérazin-1-yl)-3-[4-(2-méthyl-quinolin-4-ylméthoxy)-benzènesulfonylamino]-propanoique, 465mg (73%) de (R)-N-hydroxy-2-(4-méthanesulfonyl-pipérazin-1-yl)-3-[4-(2-méthyl-quinolin-4-ylméthoxy)-benzènesulfonylamino]-propionamide sont obtenus sous forme d'un solide blanc.
RMN ¹H (δ, DMSO): 2.53 (m, 4H); 2,68 (s, 3H); 2,84 (s, 3H); 2,85 (m, 2H); 2,95-3,05 (m, 4H); 3,10 (m, 1 H); 5,72 (s, 2H); 7,35 (d, J=8,8Hz, 2H); 7,52 (m, 1 H); 7,57-7,62 (m, 2H); 7,75-7,82 (m, 3H); 7,98 (d, J=8,4Hz, 1 H); 8,11 (d, J=8,16Hz, 1 H); 8,93 (s, 1H); 10,70 (s, 1H).

### Exemple 13 : (S)-N-Hydroxy-3-[4-(2-methyl-quinolin-4-ylmethoxy)-benzenesulfonylamino]-2-piperazin-1-yl-propionamide.

### 13.1 : 4-{(S)-1-hydroxycarbamoyl-2-[4-(2-méthyl-quinolin-4-ylméthoxy)-benzènesulfonylamino]-éthyl]-pipérazine-1-carboxylate de benzyle:

De manière analogue à l'exemple 3.8, à partir de 135mg (0,2 mmoles) de 4-{(S)-1-carboxy-2-[4-(2-méthyl-quinolin-4-ylméthoxybenzènesulfonylamino]-éthyl}-pipérazine-1-carboxylate de benzyle (préparé comme décrit en 10.6), 115mg (82%) de 4-{(S)-1-hydroxycarbamoyl-2-[4-(2-méthyl-quinolin-4-ylméthoxy)-benzènesulfonylamino]-éthyl}-pipérazine-1-carboxylate de benzyle sont obtenus sous forme d'un solide blanc de point de fusion 162°C.

### 13.2 : (S)-N-hydroxy-3-[4-(2-méthyl-quinolin-4-ylméthoxy)-benzènesulfonylamino]-2-pipérazin-1-yl-propionamide

90mg (0,15 mmoles) de 4-{(S)-1-hydroxycarbamoyl-2-[4-(2-méthyl-quinolin-4-ylméthoxy)-benzènesulfonylamino]-éthyl}-pipérazine-1-carboxylate de benzyle sont placés en solution dans 5ml de dichlorométhane et 5ml d' acide trifluoroacétique. Le milieu réactionnel est ensuite agité à température ambiante pendant 96h. Après évaporation de l'acide trifluoroacétique, le résidu est repris 5ml de la solution aqueuse saturée d'hydrogénocarbonate de sodium et extrait au n-butanol. La phase organique est lavée à l'eau puis par une solution aqueuse saturée de chlorure de sodium, séchée sur sulfate de magnésium filtrée et concentrée sous vide. Le produit brut obtenu est repris dans un mélange heptane/acétate d'éthyle 50/50, agité pendant 1h puis filtré et séché sous vide. 50mg (70%) de (S)-N-hydroxy-3-[4-(2-méthyl-quinolin-4-ylméthoxy)-benzènesulfonylamino]-2-pipérazin-1-yl-propionamide sont obtenus sous forme d'un solide beige de point de fusion 225°C.
RMN ¹H (δ, DMSO) : 2,35-2,45 (m, 4H); 2,67 (s, 3H); 2,70 (m, 4H); 2,80-3,00 (m, 2H); 3,15 (s, 1H); 5,72 (s, 2H); 7,35 (d, J=8,6Hz, 2H); 7,70-7,80 (m, 3H); 7,98 (d, J=8,4Hz, 1 H); 8,12 (d, J=8,2Hz, 1 H).

### Exemple 14 : chlorhydrate de (S)-N-hydroxy-2-(4-méthanesulfonyl-pipérazin-1-yl)-3-[4-(2-méthyl-quinolin-4-ylméthoxy)-benzènesulfonylamino]-propionamide.

0,2ml (1,3 mmoles) d'une solution d'acide chlorhydrique dans l'isopropanol de concentration 5-6N sont ajoutés à une solution de 301 mg (0,5 mmoles) de (S)-N-hydroxy-2-(4-méthanesulfonyl-pipérazin-1-yl)-3-[4-(2-méthyl-quinolin-4-ylméthoxy)-benzènesulfonylamino]-propionamide (préparé comme décrit dans l'exemple 14) dans 10ml d'isopropanol. Après agitation à température ambiante pendant 1 h, le produit précipite. Par filtration, 927mg du chlorhydrate de (S)-N-hydroxy-2-(4-méthanesulfonyl-pipérazin-1-yl)-3-[4-(2-méthyl-quinolin-4-ylméthoxy)-benzènesulfonylamino]-propionamide sont obtenus sous forme d'une poudre blanche. Ce solide est recristallisé dans un mélange isopropanol-eau 30ml / 5ml. 176mg (52%) du chlorhydrate de (S)-N-hydroxy-2-(4-méthanesulfonyl-pipérazin-1-yl)-3-[4-(2-méthyl-quinolin-4-ylméthoxy)-benzènesulfonylamino]-propionamide sont obtenus sous forme d'une poudre blanche de point de fusion 209°C.
RMN ¹H (δ, DMSO) : 2,67 (m, 4H); 2,87 (s, 3H); 2,93 (s, 3H); 3,00-3,15 (m, 4H); 3,22 (m, 1H); 3,35-3,90 (m, 2H); 5,94 (s, 2H); 7,42 (d, J=8,7Hz, 2H); 7,64 (m, 1 H); 7,83-7,90 (m, 3H); 7,98 (m, 1 H); 8,05 (m, 1 H); 8,30 (d, J=7,6Hz; 1 H); 8,38 (d, J=8,5Hz, 1 H); 9,00 (m, 1 H); 10,75 (m, 1 H).

### Exemple 15 : 3-{4-[(S)-2-hydroxycarbamoyl-2-(4-méthanesulfonyl-pipérazin-1-yl)-éthylsulfamoyl]-phénoxyméthyl}-2-méthyl-indole-1-carboxylate de tert-butyle , di trifluoroacétate.

### 15.1 : 3-{4-[(S)-2-(4-méthanesulfonyl-pipérazin-1-yl)-2-méthoxycarbonyl-éthylsulfamoyl]-phénoxyméthyl}-2-méthyl-indole-1-carboxylate de tert-butyle

De manière analogue à l'exemple 11.1, à partir de 400mg (0,95 mmoles) de (S)-3-(4-hydroxy-benzènesulfonylamino)-2-(4-méthanesulfonyl-pipérazin-1-yl)-propanoate de méthyle (préparé comme décrit dans l'exemple 5.1) et de 248mg (0,95 mmoles) de 3-hydroxyméthyl-2-méthyl-indole-1-carboxylate de tert-butyle commercial, 326mg (52%) de 3-{4-[(S)-2-(4-méthanesulfonyl-pipérazin-1-yl)-2-méthoxycarbonyl-éthylsulfamoyl]-phénoxyméthyl}-2-méthyl-indole-1-carboxylate de tert-butyle sont obtenus sous forme d'une poudre beige.

### 15.2 : 3-{4-[(S)-2-carboxy-2-(4-méthanesulfonyl-pipérazin-1-yl)-éthylsulfamoyl]-phénoxymethyl}-2-méthyl-indole-1-carboxylate de tert-butyle

De manière analogue à l'exemple 3.7, à partir de 325mg (0,5 mmoles) de 3-{4-[(S)-2-(4-méthanesulfonyl-pipérazin-1-yl)-2-méthoxycarbonyl-éthylsulfamoyl]-phénoxyméthyl}-2-méthyl-indole-1-carboxylate de tert-butyle, 179mg (100%) de 3-{4-[(S)-2-carboxy-2-(4-méthanesulfonyl-pipérazin-1-yl)-éthylsulfamoyl]-phénoxyméthyl}-2-méthyl-indole-1-carboxylate de tert-butyle sont obtenus sous forme d'une poudre jaune.

### 15.3 : Ditrifluoroacétate de 3-{4-[(S)-2-hydroxycarbamoyl-2-(4-méthanesulfonyl-pipérazin-1-yl)-éthylsulfamoyl]-phénoxymethyl}-2-méthyl-indole-1-carboxylate de tert-butyle

45mg (0,3 mmoles) de O-tert-butyldiméthylsilylhydroxylamine en solution dans 1ml de diméthylformamide sont ajoutés à une solution de 179mg (0,3 mmoles) de 3-{4-[(S)-2-carboxy-2-(4-méthanesulfonyl-pipérazin-1-yl)-éthylsulfamoyl]-phénoxymethyl}-2-méthyl-indole-1-carboxylate de tert-butyle, 41 mg (0,3 mmoles) de 1-hydroxybenzotriazole et 58mg (0,3 mmoles) du chlorhydrate de 1-éthyl-3-(3-diméthylaminopropyl) carbodiimide dans 3ml de diméthylformamide. Le mélange réactionnel est agité 18h à température ambiante. Après addition d'eau puis extraction à l'acétate d'éthyle, les phases organiques sont rassemblées, lavées avec une solution saturée d'hydrogénocarbonate de sodium puis séchées sur sulfate de sodium, filtrées et évaporées. Le résidu est purifié par HPLC préparative (colonne Gemini C6 phenyl, 150x3mm, 3µm; détecteur UV : 190-420nm; débit : 0 ,3ml/mn; solvant A : CH₃CN + 0,02% acide trifluoroacétique; solvant B : eau + 0,02% acide trifluoroacétique).

| Gradient : | | |
|---|---|---|
| Temps | Composition | |
| 0.0 min | A = 5% | B = 95% |
| 20.0 min | A = 98% | B = 2% |
| 30.0 min | A = 98% | B = 2% |
| Temps de rétention : 14,6mn, M + 1 = 666,1. | | |

Après concentration des différentes fractions, 21 mg (10%) de ditrifluoroacétate de 3-{4-[(S)-2-hydroxycarbamoyl-2-(4-méthanesulfonyl-pipérazin-1-yl)-éthylsulfamoyl]-phénoxyméthyl}-2-méthyl-indole-1-carboxylate de tert-butyle sont obtenus.

### Exemple 16 : (S)-N-hydroxy-2-(4-méthanesulfonyl-pipérazin-1-yl)-3-[4-(quinolin-4-yl méthoxy)-benzènesulfonylamino]-propionamide.

### 16.1 (S)-2-(4-méthanesulfonyl-pipérazin-1-yl)-3-[4-(quinolin-4-ylméthoxy)-benzènesulfonylamino]-propanoate de méthyle

De manière analogue à l'exemple 5.2, à partir de 440mg (2,5 mmoles) de 4-chlorométhyl-quinoline et de 950mg (2,2 mmoles) de (S)-3-(4-hydroxy-benzènesulfonylamino)-2-(4-méthanesulfonyl-piperazin-1-yl)-propanoate de méthyle (préparé comme décrit en 5.1), 550mg (43%) de (S)-2-(4-méthanesulfonyl-pipérazin-1-yl)-3-[4-(quinolin-4-ylméthoxy)-benzènesulfonylamino]-propanoate de méthyle sont obtenus sous forme d'une huile incolore.

### 16.2 : acide (S)-2-(4-méthanesulfonyl-pipérazin-1-yl)-3-[4-(quinolin-4-ylméthoxy)-benzènesulfonylamino]-propanoïque

De manière analogue à l'exemple 3.7, à partir de 550mg (1,0 mmole) de (S)-2-(4-méthanesulfonyl-pipérazin-1-yl)-3-[4-(quinolin-4-ylméthoxy)-benzènesulfonylamino]-propanoate de méthyle, 450mg (83%) d' acide (S)-2-(4-méthanesulfonyl-pipérazin-1-yl)-3-[4-(quinolin-4-ylméthoxy)-benzènesulfonylamino]-propanoïque sont obtenus sous forme d'un solide blanc.

### 16.3 : (S)-N-hydroxy-2-(4-méthanesulfonyl-pipérazin-1-yl)-3-[4-(quinolin-4-ylméthoxy)-benzènesulfonylamino]-propionamide

De manière analogue à l'exemple 3.8, à partir de 450mg (0,8 mmoles) d' acide (S)-2-(4-méthanesulfonyl-pipérazin-1-yl)-3-[4-(quinolin-4-ylméthoxy)-benzènesulfonylamino]-propanoïque 260mg (56%) de (S)-N-hydroxy-2-(4-méthanesulfonyl-pipérazin-1-yl)-3-[4-(quinolin-4-ylméthoxy)-benzènesulfonylamino]-propionamide sont obtenus sous forme d'un solide blanc de point de fusion 180°C.
RMN ¹H (δ, DMSO) : 2,52-2,54 (m, 4H); 2,84 (s, 3H); 2,87 (m, 1 H); 2,97 (m, 1 H); 2,98-3,05 (m, 4H), 3,11 (t, J=7Hz, 1 H); 5,78 (s, 2H); 7,34 (d, J=8,8Hz, 2H); 7,52 (m, 1 H); 7,66-7,72 (m, 2H); 7,78-7,84 (m, 3H); 8,10 (d, J=8,3Hz, 1 H); 8,19 (d, J=8,2Hz, 1 H); 8,93 (s, 1 H); 8,94 (s, 1 H); 10,67 (s, 1 H).

### Exemple 17 : (S)-2-(4-benzyl-pipérazin-1-yl)-N-hydroxy-3-[4-(2-méthyl-quinolin-4-ylméthoxy)-benzènesulfonylamino]-propionamide.

### 17.1 : Sel de sodium de l'acide 4-(2-méthyl-quinolin-4-ylméthoxy)-benzènesulfonique

100g (438 mmoles) du chlorhydrate de 4-chlorométhyl-2-méthyl-quinoline sont additionnés sur une solution de 77g (395 mmoles) du sel de sodium de l'acide 4-hydroxy-benzènesulfonique et de 84ml (84 mmoles) d'une solution aqueuse d'hydroxyde de sodium de concentration 1M dans 800ml d'isopropanol. Le milieu réactionnel est chauffé à 70°C pendant 5h puis à 40°C pendant 18h.
Après évaporation de l'isopropanol, le produit obtenu est filtré, rincé à l'isopropanol et à l'éther diéthylique puis séché sous vide. 114g (75%) du sel de sodium de l'acide 4-(2-méthyl-quinolin-4-ylméthoxy)-benzènesulfonique sont obtenus sous forme d'un solide blanc.

### 17.2 : Chlorure de 4-(2-méthyl-quinolin-4-ylméthoxy)-benzènesulfonyle

76g (216 mmoles) du sel de sodium de l'acide 4-(2-méthyl-quinolin-4-ylméthoxy)-benzènesulfonique dans 500ml de diméthylformamide sont additionnés goutte à goutte à une solution de 55ml (649 mmoles) de chlorure d'oxalyle dans 100ml de dichlorométhane, préalablement refroidie à -10°C. Après addition, le milieu réactionnel est agité à température ambiante pendant 18h. Le milieu réactionnel est ensuite versé dans 11 de glace puis extrait à l'acétate d'éthyle. Les phases organiques sont rassemblées, lavées à l'eau puis avec une solution aqueuse saturée de chlorure de sodium, séchées de sulfate de magnésium, filtrées et concentrées sous vide. 77g (92%) du chlorhydrate de chlorure de 4-(2-méthyl-quinolin-4-ylméthoxy)-benzènesulfonyle sont obtenus sous forme d'un solide beige.

### 17.3 : benzyl-bis-(2-chloro-éthyl)-amine

21g (152 mmoles) de carbonate de potassium puis 8ml (67 mmoles) de bromure de benzyle sont ajoutés à une solution de 10g (56 mmoles) de chlorhydrate de bis-(2-chloro-éthyl)-amine dans 130ml d'acétonitrile puis le milieu réactionnel est chauffé à 60°C pendant 24h. Après filtration, le filtrat est concentré sous vide. Le résidu brut est purifié par chromatographie sur gel de silice élué avec un mélange heptane/acétate d'éthyle 90/10 pour donner 8,5g (65%) de benzyl-bis-(2-chloro-éthyl)-amine.

### 17.4 : (S)-2-(4-benzyl-pipérazin-1-yl)-3-tert-butoxycarbonylamino-propanoate de méthyle

Une solution de 5,9g (23 mmoles) du chlorhydrate de (S)-2-amino-3-tert-butoxycarbonylamino-propanoate de méthyle commercial et de 9,6g (23 mmoles) de benzyl-bis-(2-chloro-éthyl)-amine dans 50ml de N,N-diisopropyléthylamine est chauffée à 127°C pendant 3h30. Après évaporation de la N,N-diisopropyléthylamine, le milieu réactionnel est hydrolysé puis extrait par de l'acétate d'éthyle. La phase organique est lavée par une solution aqueuse d'hydroxyde de sodium de concentration 1 N, par de l'eau puis séchée sur sulfate de magnésium, filtrée et concentrée sous vide. Le produit brut obtenu est purifié par chromatographie sur gel de silice élué avec un mélange heptane/acétate d'éthyle 50/50. 8,9g (64%) de (S)-2-(4-benzyl-pipérazin-1-yl)-3-tert-butoxycarbonylamino-propanoate de méthyle sont obtenus sous forme d'une huile jaune.

### 17.5 : trichlorhydrate de (S)-3-amino-2-(4-benzyl-pipérazin-1-yl)-propanoate de méthyle

8,9g ( 23,5 mmoles) de (S)-2-(4-benzyl-pipérazin-1-yl)-3-tert-butoxycarbonylamino-propanoate de méthyle sont placés en solution dans 60ml de méthanol et dans 20ml d'acide chlorhydrique isopropanolique de concentration 5-6N . Le milieu réactionnel est agité à 40°C pendant 18h puis concentré sous vide. 9,0g (100%) du trichlorhydrate de (S)-3-amino-2-(4-benzyl-pipérazin-1-yl)-propanoate de méthyle sont obtenus sous forme d'un solide beige.

### 17.6 : (S)-2-(4-benzyl-pipérazin-1-yl)-3-[4-(2-méthyl-quinolin-4-ylméthoxy)-benzènesulfonylamino]-propanoate de méthyle.

De manière analogue à l'exemple 3.6, à partir de 1,0g (2,6 mmoles) du trichlorhydrate de (S)-3-amino-2-(4-benzyl-pipérazin-1-yl)-propanoate de méthyle et de 1,1g (2,8 mmoles) de chlorure de 4-(2-méthyl-quinolin-4-ylméthoxy)-benzènesulfonyle sous forme de chlorohydrate, 750mg (50%) de (S)-2-(4-benzyl-pipérazin-1-yl)-3-[4-(2-méthyl-quinolin-4-ylméthoxy)-benzènesulfonylamino]-propanoate de méthyle sont obtenus sous forme d'un solide beige.

### 17.7: acide (S)-2-(4-benzyl-pipérazin-1-yl)-3-[4-(2-méthyl-quinolin-4-ylméthoxy)-benzènesulfonylamino]-propanoïque.

De manière analogue à l'exemple 3.7, à partir de 750mg (1,3 mmoles) de (S)-2-(4-benzyl-pipérazin-1-yl)-3-[4-(2-méthyl-quinolin-4-ylméthoxy)-benzènesulfonylamino]-propanoate de méthyle, 680mg (93%) d'acide (S)-2-(4-benzyl-pipérazin-1-yl)-3-[4-(2-méthyl-quinolin-4-ylméthoxy)-benzènesulfonylamino]-propanoïque sont obtenus sous forme d'un solide blanc.

### 17.8 : (S)-2-(4-benzyl-pipérazin-1-yl)-N-hydroxy-3-[4-(2-méthyl-quinolin-4-ylméthoxy)-benzènesulfonylamino]-propionamide.

De manière analogue à l'exemple 3.8, à partir de 680mg (1,2 mmoles) d'acide (S)-2-(4-benzyl-pipérazin-1-yl)-3-[4-(2-méthyl-quinolin-4-ylméthoxy)-benzènesulfonylamino]-propanoique, 250mg (36%) de (S)-2-(4-benzyl-pipérazin-1-yl)-N-hydroxy-3-[4-(2-méthyl-quinolin-4-ylméthoxy)-benzènesulfonylamino]-propionamide sont obtenus sous forme d'un solide blanc de point de fusion 188°C.
RMN ¹H (δ, DMSO) : 2,33 (m, 4H); 2,49 (m, 4H); 2,73 (s, 3H); 2,80-2,90 (m, 1H); 3,00-3,10 (m, 2H); 2,46 (m, 2H); 5,77 (s, 2H); 7,25-7,40 (m, 7H); 7,50(m, 1 H); 7,61-7,67 (m, 2H); 7,78-7,85 (m, 3H); 8,04 (d, J=8Hz, 1 H); 8,17 (d, J=8,2Hz, 1 H); 8,95 (s, 1 H); 10,65 (s, 1 H).

### Exemple 18 : (S)-2-[4-(4-fluoro-benzyl)-pipérazin-1-yl]-N-hydroxy-3-[4-(2-méthyl-quinolin-4-ylméthoxy)-benzènesulfonylamino]-propionamide.

### 18.1 : bis-(2-chloro-éthyl)-(4-fluoro-benzyl)-amine

De manière analogue à l'exemple 17.3, à partir de 5g (28 mmoles) du chlorhydrate de bis-(2-chloro-éthyl)-amine et de 3,8ml (31 mmoles) de 1-bromométhyl-4-fluoro-benzène, 6,9g (98%) de bis-(2-chloro-éthyl)-(4-fluoro-benzyl)-amine sont obtenus.

### 18.2 : (S)-3-tert-butoxycarbonylamino-2-[4-(4-fluoro-benzyl)-pipérazin-1-yl]-propanoate de méthyle

De manière analogue à l'exemple 17.4, à partir de 7,1g (28 mmoles) du chlorhydrate de (S)-2-amino-3-tert-butoxycarbonylamino-propanoate de méthyle et de 6,9g (28 mmoles) de bis-(2-chloro-éthyl)-(4-fluoro-benzyl)-amine, 5,3g (48%) de (S)-3-tert-butoxycarbonylamino-2-[4-(4-fluoro-benzyl)-pipérazin-1-yl]-propanoate de méthyle sont obtenus sous forme d'une huile.

### 18.3 : trichlorhydrate de (S)-3-amino-2-[4-(4-fluoro-benzyl)-pipérazin-1-yl]-propanoate de méthyle

De manière analogue à l'exemple 17.5, à partir de 5,3g (13,4 mmoles) de (S)-3-tert-butoxycarbonylamino-2-[4-(4-fluoro-benzyl)-piperazin-1-yl]-propanoate de méthyle, 5,4g (100%) de trichlorhydrate de (S)-3-amino-2-[4-(4-fluoro-benzyl)-pipérazin-1-yl]-propanoate de méthyle sont obtenus sous forme d'un solide beige.

### 18.4 : (S)-2-[4-(4-fluoro-benzyl)-piperazin-1-yl]-3-[4-(2-méthyl-quinolin-4-ylméthoxy)-benzènesulfonylamino]-propanoate de méthyle

De manière analogue à l'exemple 3.6, à partir de 1,5g ( 3,7 mmoles) du trichlorhydrate de (S)-3-amino-2-[4-(4-fluoro-benzyl)-pipérazin-1-yl]-propanoate de méthyle et de 1,6g (4,1mmoles) de chlorure de 4-(2-méthyl-quinolin-4-ylméthoxy)-benzènesulfonyle chlorohydrate (préparé comme décrit en 17.2), 1,0g (46%) de (S)-2-[4-(4-fluoro-benzyl)-pipérazin-1-yl]-3-[4-(2-méthyl-quinolin-4-ylméthoxy)-benzènesulfonylamino]-propanoate de méthyle sont obtenus sous forme d'un solide blanc.

### 18.5 : acide (S)-2-[4-(4-fluoro-benzyl)-pipérazin-1-yl]-3-[4-(2-méthyl-quinolin-4-ylméthoxy)-benzènesulfonylamino]-propanoïque

De manière analogue à l'exemple 3.7, à partir de 1,1 g (1,7 mmoles) de (S)-2-[4-(4-fluoro-benzyl)-pipérazin-1-yl]-3-[4-(2-méthyl-quinolin-4-ylméthoxy)-benzènesulfonylamino]-propanoate de méthyle 1,0g (100%) d'acide (S)-2-[4-(4-fluoro-benzyl)-pipérazin-1-yl]-3-[4-(2-méthyl-quinolin-4-ylméthoxy)-benzènesulfonylamino]-propanoïque sont obtenus sous forme d'un solide blanc.

### 18.6 : (S)-2-[4-(4-fluoro-benzyl)-pipérazin-1-yl]-N-hydroxy-3-[4-(2-méthyl-quinolin-4-ylméthoxy)-benzènesulfonylamino]-propionamide

De manière analogue à l'exemple 3.8, 990mg (1,7 mmoles) d'acide (S)-2-[4-(4-fluoro-benzyl)-pipérazin-1-yl]-3-[4-(2-méthyl-quinolin-4-ylméthoxy)-benzènesulfonylamino]-propanoique, 330mg (33%) de (S)-2-[4-(4-fluoro-benzyl)-pipérazin-1-yl]-N-hydroxy-3-[4-(2-méthyl-quinolin-4-ylméthoxy)-benzènesulfonylamino]-propionamide sont obtenus sous forme d'un solide blanc de point de fusion 180°C.
RMN ¹H (δ, DMSO) : 2,20-2-30 (m, 4H); 2,35-2,45 (m, 4H); 2,66 (s, 3H); 2,72-2,80 (m, 1 H); 2,87-3,00 (m, 2H); 3,38 (s, 2H); 5,70 (s, 2H); 7,10 (t, J=8,8Hz, 2H); 7,26-7,33 (m, 4H); 7,56-7,60(m, 2H); 7,73-7,78 (m, 3H); 7,97 (d, J=8,4Hz, 1 H); 8,10 (d, J=8,2Hz, 1 H).

### Exemple 19 : (S)-2-(4-éthyl-pipérazin-1-yl)-N-hydroxy-3-[4-(2-méthyl-quinolin-4-yl-méthoxy)-benzènesulfonylamino]-propionamide.

### 19.1 : bis-(2-chloro-éthyl)-éthyl-amine

24ml (330 mmoles) de chlorure de thionyle sont additionnés goutte à goutte à une solution de 20g (150 mmoles) de 2-[éthyl-(2-hydroxy-éthyl)-amino]-éthanol dans 200ml de dichlorométhane préalablement refroidie à 0°C puis le milieu réactbnnel est agité à température ambiante pendant 20h. Après addition d'une solution aqueuse saturée d'hydrogénocarbonate de sodium, le produit est extrait au dichlorométhane. La phase organique obtenue est ensuite lavée à l'eau, séchée sur sulfate de magnésium, filtrée et concentrée sous vide. 19,5g (76%) de bis-(2-chloro-éthyl)-éthyl-amine sous la forme d'une huile.

### 19.2 : (S)-3-tert-butoxycarbonylamino-2-(4-éthyl-pipérazin-1-yl)-propanoate de méthyle

Une solution de 5,0g (19,6 mmoles) de chlorhydrate de (S)-2-amino-3-tert-butoxycarbonylamino-propanoate de méthyle commercial et 3,3g (19,6 mmoles) de bis-(2-chloro-éthyl)-éthyl-amine dans 50ml de N,N-diisopropyléthylamine est chauffée à 127°C pendant 5h. Après évaporation d'un maximum de diisopropyléthylamine, le milieu réactionnel est dilué par de l'acétate d'éthyle, lavé avec une solution aqueuse d'hydroxyde de sodium de concentration 1N. La phase organique obtenue est ensuite lavée à l'eau, séchée sur sulfate de magnésium, filtrée et concentrée sous vide. Le produit brut obtenu est purifié par chromatographie sur gel de silice élué avec un mélange heptane / acétate d'éthyle 30/70. 2,5g (40%) de (S)-3-tert-butoxycarbonylamino-2-(4-éthyl-pipérazin-1-yl)-propanoate de méthyle sont obtenus sous forme d'une huile.

### 19.3 : trichlorhydrate de (S)-3-amino-2-(4-éthyl-pipérazin-1-yl)-propanoate de méthyle

2,5g (7,9 mmoles) de (S)-3-tert-butoxycarbonylamino-2-(4-éthyl-pipérazin-1-yl)-propanoate de méthyle sont placés dans 20ml de méthanol et 10ml d'acide chlorhydrique isopropanolique de concentration 5-6N. Le milieu réactionnel est chauffé à 40°C pendant 3h puis évaporé à sec. Le résidu est repris dans 50ml d'éthanol, agité 1h à température ambiante puis filtré. 1,4g (54%) de trichlorhydrate de (S)-3-amino-2-(4-éthyl-pipérazin-1-yl)-propanoate de méthyle sont obtenus sous forme d'un solide beige.

### 19.4 : (S)-2-(4-éthyl-pipérazin-1-yl)-3-[4-(2-méthyl-quinolin-4-ylméthoxy)-benzènesulfonylamino]-propanoate de méthyle

De manière analogue à l'exemple 3.6, à partir de 700mg (2,1 mmoles) du trichlorhydrate de (S)-3-amino-2-(4-éthyl-pipérazin-1-yl)-propanoate de méthyle et de 900mg (2,3 mmoles) du chlorhydrate de chlorure de 4-(2-méthyl-quinolin-4-ylméthoxy)-benzènesulfonyle (préparé comme décrit en 17.2), 740mg (67%) de (S)-2-(4-éthyl-pipérazin-1-yl)-3-[4-(2-méthyl-quinolin-4-ylméthoxy)-benzènesulfonylamino]-propanoate de méthyle sont obtenus sous forme d'un solide blanc.

### 19.5 : acide ((S)-2-(4-éthyl-pipérazin-1-yl)-3-[4-(2-méthyl-quinolin-4-ylméthoxy)-benzènesulfonylamino]-propanoïque

De manière analogue à l'exemple 3.7, à partir 740mg (1,4 mmoles) de (S)-2-(4-éthyl-pipérazin-1-yl)-3-[4-(2-méthyl-quinolin-4-ylméthoxy)-benzènesulfonylamino]-propanoate de méthyle, 630mg (87%) d'acide ((S)-2-(4-éthyl-pipérazin-1-yl)-3-[4-(2-méthyl-quinolin-4-ylméthoxy)-benzènesulfonylamino]-propanoïque sont obtenus sous forme d'un solide blanc.

### 19.6 : (S)-2-(4-éthyl-pipérazin-1-yl)-N-hydroxy-3-[4-(2-méthyl-quinolin-4-ylméthoxy)-benzènesulfonylamino]-propionamide

De manière analogue à l'exemple 3.8, à partir de 630mg (1,2 mmoles) d'acide (S)-2-(4-éthyl-pipérazin-1-yl)-3-[4-(2-méthyl-quinolin-4-ylméthoxy)-benzènesulfonylamino]-propanoique, 60mg (8%) de (S)-2-(4-éthyl-pipérazin-1-yl)-N-hydroxy-3-[4-(2-méthyl-quinolin-4-ylméthoxy)-benzène-sulfonylamino]-propionamide sont obtenus sous forme d'un solide blanc de point de fusion 150°C.
RMN ¹H (δ, DMSO) : 2,49 (s, 3H); 2,55-2,65 (m, 2H); 2,69 (s, 3H); 2,70-2,90 (m, 6H); 2,90-3,00 (m, 2H); 3,13 (t, J=7,3Hz, 1 H); 3,20-3,35 (m, 2H); 3,36 (s, 2H); 5,72 (s, 2H); 7,35 (d, J=8,9Hz, 2H); 7,58-7,62 (m, 3H); 7,74-7,81 (m, 3H); 7,98 (d, J=7,9Hz, 1H); 8,12 (d, J=8,3Hz, 1H); 9,03 (s, 1H), 10,82 (s, 1 H).

### Exemple 20 : (S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide.

### 20.1 : bis-(2-chloro-éthyl)-(4-trifluorométhyl-benzyl)-amine

De manière analogue à l'exemple 32.3, à partir 5,0g (28 mmoles) du chlorhydrate bis-(2-chloro-éthyl)-amine et de 7,4g (31 mmoles) de 1-bromométhyl-4-trifluorométhyl-benzène, 5g (59%) d'un mélange de bis-(2-chloro-éthyl)-(4-trifluorométhyl-benzyl)-amine sont obtenus sous forme d'une huile incolore.

### 20.2 : (S)-3-tert-butoxycarbonylamino-2-[4-(4-trifluorométhyl-benzyl)-pipérazin-1-yl]-propanoate de méthyle

Une solution de 4,2g (16,5 mmoles) du chlorhydrate de (S)-2-amino-3-tert-butoxycarbonylamino-propanoate de méthyle et 4,95g (16,5 mmoles) de bis-(2-chloro-éthyl)-(4-trifluorométhyl-benzyl)-amine dans 25ml de N,N-Diisopropyléthylamine est chauffée à 127°C pendant 6h. Après évaporation d'un maximum de diisopropyléthylamine, le milieu réactionnel est dilué avec de l'acétate d'éthyle et lavé par une solution aqueuse d'hydroxyde de sodium de concentration 1 N. La phase organique obtenue est lavée à l'eau, séchée sur sulfate de magnésium, filtrée et concentrée sous vide. Le résidu brut est purifié par chromatographie sur gel de silice élué avec un mélange heptane / acétate d'éthyle 60/40. 4,0g (55%) de (S)-3-tert-butoxycarbonylamino-2-[4-(4-trifluorométhyl-benzyl)-pipérazin-1-yl]-propanoate de méthyle sont obtenus sous forme d'une huile.

### 20.3 : trichlorhydrate de (S)-3-amino-2-[4-(4-trifluorométhyl-benzyl)-pipérazin-1-yl]-propanoate de méthyle

De manière analogue à l'exemple 17.5, à partir de 4g (9,1 mmoles) de (S)-3-tert-butoxycarbonylamino-2-[4-(4-trifluorométhyl-benzyl)-pipérazin-1-yl]-propanoate de méthyle, 3,8g (93%) de trichlorhydrate de (S)-3-amino-2-[4-(4-trifluorométhyl-benzyl)-pipérazin-1-yl]-propanoate de méthyle sont obtenus sous la forme d'un solide beige.

### 20.4 : (S)-3-[4-(2-méthyl-quinolin-4-ylméthoxy)-benzènesulfonylamino]-2-[4-(4-trifluorométhyl-benzyl)-pipérazin-1-yl]-propanoate de méthyle

De manière analogue à l'exemple 3.6, à partir de 1,0g ( 2,2 mmoles) de trichlorhydrate de (S)-3-amino-2-[4-(4-trifluorométhyl-benzyl)-pipérazin-1-yl]-propanoate de méthyle et de 1,2g (3,1 mmoles) de chlorhydrate de chlorure de 4-(2-méthyl-quinolin-4-ylméthoxy)-benzènesulfonyle (préparé comme décrit dans l'exemple 17.2), 910mg (65%) de (S)-3-[4-(2-méthyl-quinolin-4-ylméthoxy)-benzènesulfonylamino]-2-[4-(4-trifluorométhyl-benzyl)-pipérazin-1-yl]-propanoate de méthyle sont obtenus sous forme d'un solide blanc.

### 20.5 : acide (S)-3-[4-(2-méthyl-quinolin-4-ylméthoxy)-benzènesulfonylamino]-2-[4-(4-trifluorométhyl-benzyl)-pipérazin-1-yl]propanoïque

De manière analogue à l'exemple 3.7, à partir de 910mg (1,4 mmoles) de (S)-3-[4-(2-méthyl-quinolin-4-ylméthoxy)-benzènesulfonylamino]-2-[4-(4-trifluorométhyl-benzyl)-pipérazin-1-yl]-propanoate de méthyle, 790mg (88%) d'acide (S)-3-[4-(2-méthyl-quinolin-4-ylméthoxy)-benzènesulfonylamino]-2-[4-(4-trifluorométhyl-benzyl)-pipérazin-1-yl]propanoïque sont obtenus sous forme d'un solide blanc.

### 20.6 : (S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide

De manière analogue à l'exemple 3.8, à partir de 790mg (1,2 mmoles) d'acide (S)-3-[4-(2-méthyl-quinolin-4-ylméthoxy)benzènesulfonylamino]-2-[4-(4-trifluorométhyl-benzyl)-pipérazin-1-yl]-propanoique, 550mg (68%) de (S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide sont obtenus sous forme d'un solide blanc de point de fusion 148°C.
RMN ¹H (δ, DMSO) : 2,21 (m, 4H); 2,38 (m, 4H); 2,58 (s, 3H); 2,69-2,75 (m, 1 H); 2,85-2,93 (m, 1 H); 2,93-2,98 (m, 1 H); 3,42 (s, 2H); 5,63 (s, 2H); 7,25 (d, J=9Hz, 2H); 7,40 (d, J=8Hz, 3H); 7,47-7,53 (m, 2H); 7,57 (d, J=8,1 Hz, 2H); 7,65-7,72 (m, 3H); 7,90 (d, J=7,9Hz, 1 H); 8,03 (d, J=7,8Hz, 1 H); 8,83 (s, 1 H), 10,56 (s, 1 H).

### Exemple 21 : (S)-N-hydroxy-2-[4-(4-méthyl-benzyl)-pipérazin-1-yl]-3-[4-(2-méthyl-quinolin-4-ylméthoxy)-benzènesulfonylamino]-propionamide.

### 21.1 : bis-(2-chloro-éthyl)-(4-méthyl-benzyl)-amine

De manière analogue à l'exemple 17.3, à partir de 5,0g (28 mmoles) du chlorhydrate de bis-(2-chloro-éthyl)-amine et de 5,7g (31 mmoles) de 1-bromométhyl-4-méthyl-benzène, 4,9g (71%) de bis-(2-chloro-éthyl)-(4-méthyl-benzyl)-amine sont obtenus.

### 21.2 : (S)-3-tert-butoxycarbonylamino-2-[4-(4-méthyl-benzyl)-pipérazin-1-yl]-propanoate de méthyle

De manière analogue à l'exemple 17.4, à partir de 5,1 g (20 mmoles) du chlorhydrate de (S)-2-amino-3-tert-butoxycarbonylamino-propanoate de méthyle commercial et de 4,9g (20 mmoles) de bis-(2-chloro-éthyl)-(4-méthyl-benzyl)-amine, 4,1g (53%) de (S)-3-tert-butoxycarbonylamino-2-[4-(4-méthyl-benzyl)-pipérazin-1-yl]-propanoate de méthyle sont obtenus sous forme d'une huile.

### 21.3 : trichlorhydrate de (S)-3-amino-2-[4-(4-méthyl-benzyl)-pipérazin-1-yl]-propanoate de méthyle

De manière analogue à l'exemple 19.3, à partir de 4,1g (10,5 mmoles) de (S)-3-tert-butoxycarbonylamino-2-[4-(4-méthyl-benzyl)-pipérazin-1-yl]-propanoate de méthyle, 3,95g (94%) de trichlorhydrate de (S)-3-amino-2-[4-(4-méthyl-benzyl)-pipérazin-1-yl]-propanoate de méthyle sont obtenus sous forme d'un solide crème.

### 21.4: (S)-3-[4-(2-méthyl-quinolin-4-ylméthoxy)-benzènesulfonylamino]-2-[4-(4-méthyl-benzyl)-pipérazin-1-yl]-propanoate de méthyle.

De manière analogue à l'exemple 3.6, à partir de 1,0g (2,5 mmoles) du trichlorhydrate de (S)-3-amino-2-[4-(4-méthyl-benzyl)-pipérazin-1-yl]-propanoate de méthyle et de 1,3g (3,5 mmoles) de chlorhydrate de chlorure de 4-(2-méthyl-quinolin-4-ylméthoxy)-benzènesulfonyle (préparé comme décrit dans l'exemple 17.2), 950mg (63%) de (S)-3-[4-(2-méthyl-quinolin-4-ylméthoxy)-benzènesulfonylamino]-2-[4-(4-méthyl-benzyl)-pipérazin-1-yl]-propanoate de méthyle sont obtenus sous forme d'un solide blanc.

### 21.5 : acide ((S)-2-[4-(4-méthyl-benzyl)-pipérazin-1-yl]-3-[4-(2-méthyl-quinolin-4-ylméthoxy)-benzènesulfonylamino]-propanoïque.

De manière analogue à l'exemple 3.7, à partir de 950mg (1,6 mmoles) de (S)-2-[4-(4-méthyl-benzyl)-pipérazin-1-yl]-3-[4-(2-méthyl-quinolin-4-ylméthoxy)-benzènesulfonylamino]- propanoate de méthyle, 880mg (95%) d'acide ((S)-2-[4-(4-méthyl-benzyl)-pipérazin-1-yl]-3-[4-(2-méthyl-quinolin-4-ylméthoxy)-benzènesulfonylamino]-propanoïque sont obtenus sous forme d'un solide crème.

### 21.6: (S)-N-hydroxy-3-[4-(2-méthyl-quinolin-4-ylméthoxy)-benzènesulfonylamino]-2-[4-(4-méthyl-benzyl)-pipérazin-1-yl]-propionamide.

De manière analogue à l'exemple 3.8, à partir de 880mg (1,5 mmoles) d'acide (S)-3-[4-(2-méthyl-quinolin-4-ylméthoxy)benzènesulfonylamino]-2-[4-(4-méthyl-benzyl)-pipérazin-1-yl]-propanoique, 150mg (17%) de (S)-N-hydroxy-3-[4-(2-méthyl-quinolin-4-ylméthoxy)-benzènesulfonylamino]-2-[4-(4-méthyl-benzyl)-pipérazin-1-yl]-propionamide sont obtenus sous forme d'un solide blanc de point de fusion 170°C.
RMN ¹H (δ, DMSO) : 2,25 (m, 4H); 2,25 (s, 3H); 2,43 (m, 4H); 2,67 (s, 3H); 2,80 (m, 1 H); 2,95-3,05 (m, 2H); 3,37 (m, 2H); 5,71 (s, 2H); 7,10 (q, J=8Hz, 4H); 7,33 (d, J=8,9Hz, 2H); 7,43 (m, 1H); 7,56-7,61 (m, 2H); 7,73-7,79 (m, 3H); 7,98 (d, J=8,3Hz, 1 H); 8,11 (d, J=8,2Hz, 1 H); 8,89 (s, 1 H); 10,59 (s, 1 H).

### Exemple 22 : (S)-3-[4-(benzoisoxazol-3-ylméthoxy)-benzènesulfonylamino]-N-hydroxy-2-(4-méthanesulfonyl-pipérazin-1-yl)-propionamide.

### 22.1 : Benzoisoxazol-3-yl-méthanol.

589mg (3,0 mmoles) de 1,2-benzoisoxazole-3-carboxylate d'éthyle en solution dans 10ml de tétrahydrofuranne sont ajoutés à une suspension de 129mg (3,5 mmoles) d'hydrure de lithium aluminium dans 5ml de tétrahydrofuranne. Le mélange réactionnel est agité une heure à 60°C puis traité par l'addition goutte à goutte de 2ml de méthanol, filtré sur célite, rincé à l'acétate d'éthyle. Les phases organiques sont rassemblées, séchées sur sulfate de sodium et évaporées. Le résidu obtenu est purifié par chromatographie sur gel de silice élué avec un mélange heptane/acétate d'éthyle 60/40. 180mg (39%) de benzoisoxazol-3-yl-méthanol sont obtenus sous forme d'un solide blanc.

### 22.2 : (S)-3-[4-(benzoisoxazol-3-ylméthoxy)-benzènesulfonylamino]-2-(4-méthanesulfonyl-pipérazin-1-yl)-propanoate de méthyle.

De manière analogue à l'exemple 11.1, à partir de 494mg (1,2 mmoles) de (S)-3-(4-hydroxy-benzènesulfonylamino)-2-(4-méthanesulfonyl-pipérazin-1-yl)-propanoate de méthyle (préparé comme décrit en 5.1 ) et de 175mg (1,2 mmoles) de benzoisoxazol-3-yl-méthanol, 459mg (71%) de (S)-3-[4-(benzoisoxazol-3-ylméthoxy)-benzènesulfonylamino]-2-(4-méthanesulfonyl-pipérazin-1-yl)-propanoate de méthyle sont obtenus sous forme d'une huile.

### 22.3 : acide (S)-3-[4-(benzoisoxazol-3-ylméthoxy)-benzènesulfonylamino]-2-(4-méthanesulfonyl-pipérazin-1-yl)-propanoique.

De manière analogue à l'exemple 3.7, à partir de 458mg (0,8 mmoles) de (S)-3-[4-(benzoisoxazol-3-ylméthoxy)-benzènesulfonylamino]-2-(4-méthanesulfonyl-pipérazin-1-yl)-propanoate de méthyle, 283mg (63%) d'acide (S)-3-[4-(benzoisoxazol-3-ylméthoxy)-benzènesulfonylamino]-2-(4-méthanesulfonyl-pipérazin-1-yl)-propanoique sont obtenus sous forme d'un solide blanc.

### 22.4 : (S)-3-[4-(benzoisoxazol-3-ylméthoxy)-benzènesulfonylamino]-N-hydroxy-2-(4-méthanesulfonyl-pipérazin-1-yl)-propionamide.

De manière analogue à l'exemple 3.8, à partir de 283mg (0,5 mmoles) d'acide (S)-3-[4-(benzoisoxazol-3-ylméthoxy)-benzènesulfonylamino]-2-(4-éthanesulfonyl-pipérazin-1-yl)-propanoique, 231 mg (80%) de (S)-3-[4-(benzoisoxazol-3-ylméthoxy)-benzènesulfonylamino]-N-hydroxy-2-(4-méthanesulfonyl-pipérazin-1-yl)-propionamide sont obtenus sous forme d'un solide beige de point de fusion 107°C.
RMN ¹H (δ, DMSO) : 2,53-2,55 (m, 4H); 2,88 (s, 3H); 2,90-2,93 (m, 2H); 3,00-3,10 (m, 4H); 3,13 (t, J=6,9Hz,1 H); 5,77 (s, 2H); 7,35 (d, J=8,8Hz, 2H); 7,49 (t, J=7,5Hz, 1 H); 7,57 (m, 1 H); 7,75 (t, J=7,4Hz, 1 H); 7,78-7,87 (m, 3H); 8,01 (d, J=8Hz, 1 H); 8,96 (m, 1H); 10,67 (m, 1H).

### Exemple 23 : (S)-N-hydroxy-2-(4-isobutyryl-pipérazin-1-yl)-3-[4-(2-méthyl-quinolin-4-ylméthoxy)-benzènesulfonylamino]-propionamide.

### 23.1 : (S)-3-tert-butoxycarbonylamino-2-pipérazin-1-yl-propanoate de méthyle.

2g (25% massique) de palladium sur charbon 10% sont additionnés à une solution de 8g (21 mmoles) de (S)-2-(4-benzyl-pipérazin-1-yl)-3-tert-butoxycarbonylamino-propanoate de méthyle (préparé comme décrit dans l'exemple 17.4) dans 120ml d'éthanol, préalablement dégazée sous flux d'azote. Le milieu réactionnel est ensuite placé sous une pression atmosphérique d'hydrogène pendant 24h puis filtré sur célite et abondamment rincé au dichlorométhane. Après concentration sous vide, 6,1g (100%) de (S)-3-tert-butoxycarbonylamino-2-pipérazin-1-yl-propanoate de méthyle sont obtenus.

### 23.2 : (S)-3-tert-butoxycarbonylamino-2-(4-isobutyryl-pipérazin-1-yl)-propanoate de méthyle.

1,2ml ( 8,3 mmoles) de triéthylamine puis 0,8ml (7,6 mmoles) de chlorure d'isobutyryle sont additionnés à une solution de 2,0g ( 6,9 mmoles) de (S)-3-tert-butoxycarbonylamino-2-pipérazin-1-yl-propanoate de méthyle dans 20ml de dichlorométhane, préalablement refroidie à 0°C. Après agitation à température ambiante pendant 1h30, de l'eau est additionnée. Le milieu réactionnel est extrait au dichlorométhane. La phase organique est lavée à l'eau, séchée sur sulfate de magnésium, filtrée et concentrée sous vide. Le résidu brut obtenu est purifié par chromatographie sur gel de silice élué avec un mélange heptane / acétate d'éthyle 50/50. 2,0g (81%) de ((S)-3-tert-butoxycarbonylamino-2-(4-isobutyryl-pipérazin-1-yl)-propanoate de méthyle sont obtenus sous forme d'une huile incolore.

### 23.3 : acide (S)-3-tert-butoxycarbonylamino-2-(4-isobutyryl-pipérazin-1-yl)-propanoique.

10ml (10 mmoles) d'une solution aqueuse d'hydroxyde de lithium de concentration 1N sont additionnés à une solution de 2,0g (5,6 mmoles) de (S)-3-tert-butoxycarbonylamino-2-(4-isobutyryl-pipérazin-1-yl)-propanoate de méthyle dans 40ml de tétrahydrofuranne et 8ml d'eau puis le milieu réactionnel est agité à température ambiante pendant 20h. Après addition d'une solution aqueuse d'acide acétique de concentration 1N, le produit est extrait au n-butanol. La phase organique est séchée sur sulfate de magnésium, filtrée, concentrée sous vide. 1,5g (78%) d'acide (S)-3-tert-butoxycarbonylamino-2-(4-isobutyryl-pipérazin-1-yl)propanoïque sont obtenus sous forme d'un solide blanc.

### 23.4 : (S)-2-allyloxycarbamoyl-2-(4-isobutyryl-pipérazin-1-yl)-éthyl]-carbamate de tert-butyle.

1,4g (4,4 mmoles) de O-(benzotriazol-1-yl)-N,N, N',N'-tétraméthyluroniumtétrafluoroborate puis 2,3ml (13,1 mmoles) de diisopropyléthylamine sont ajoutés à une solution de 1,5g (4,4 mmoles) d'acide (S)-3-tert-butoxycarbonylamino-2-(4-isobutyryl-pipérazin-1-yl)-propanoïque dans 20ml de diméthylformamide. Après agitation à température ambiante pendant 15min, une solution de 500mg (4,6 mmoles) de chlorhydrate de O-allyl-hydroxylamine et de 0,8ml (4,6 mmoles) de diisopropyléthylamine dans 10ml de diméthylformamide est ajoutée. Le milieu réactionnel est agité à température ambiante pendant 20h, hydrolysé par une solution aqueuse saturée d'hydrogénocarbonate de sodium puis dilué par de l'acétate d'éthyle. La phase organique est lavée par une solution aqueuse saturée de chlorure de sodium, séchée sur sulfate de magnésium, filtrée et concentrée sous vide. 1,45g (83%) de [(S)-2-allyloxycarbamoyl-2-(4-isobutyryl-pipérazin-1-yl)-éthyl]-carbamate de tert-butyle sont obtenus sous forme d'une huile incolore.

### 23.5 : dichlorhydrate de (S)-N-allyloxy-3-amino-2-(4-isobutyryl-pipérazin-1-yl)-propionamide.

De manière analogue à l'exemple 19.3, à partir de 1,45g (3,6 mmoles) de [(S)-2-allyloxycarbamoyl-2-(4-isobutyryl-pipérazin-1-yl)-éthyl]-carbamate de tert-butyle, 1,4g (100%) de dichlorhydrate de (S)-N-allyloxy-3-amino-2-(4-isobutyryl-pipérazin-1-yl)-propionamide sont obtenus sous forme d'un solide blanc.

### 23.6 : (S)-N-allyloxy-2-(4-isobutyryl-pipérazin-1-yl)-3-[4-(2-méthyl-quinolin-4-ylméthoxy)-benzènesulfonylamino]-propionamide.

1,9g (5,1 mmoles) du chlorhydrate de chlorure de 4-(2-méthyl-quinolin-4-ylméthoxy)-benzènesulfonyle chlorohydrate (préparé comme décrit en 32.2) sont ajoutés à une solution de 1,3g (3,6 mmoles) de dichlorhydrate de (S)-N-allyloxy-3-amino-2-(4-isobutyryl-pipérazin-1-yl)-propionamide, 2,0ml (14,5 mmoles) de triéthylamine dans 15ml de dichlorométhane et 15ml de diméthylformamide , préalablement refroidie à 0°C. Le milieu réactionnel est ensuite agité de 0°C à température ambiante pendant 3h. Après addition d'eau, le milieu réactionnel est extrait au dichlorométhane. La phase organique est lavée par une solution aqueuse d'hydrogénocarbonate de sodium saturée, par de l'eau, séchée sur sulfate de magnésium, filtrée et concentrée.
Le résidu brut obtenu est purifié par chromatographie sur colonne de silice élué avec un mélange dichlorométhane/ méthanol 97/3. 900mg (41%) de (S)-N-allyloxy-2-(4-isobutyryl-pipérazin-1-yl)-3-[4-(2-méthyl-quinolin-4-ylméthoxy)-benzènesulfonylamino]-propionamide sont obtenus sous forme d'un solide blanc.

### 23.7: (S)-N-hydroxy-2-(4-isobutyryl-pipérazin-1-yl)-3-[4-(2-méthyl-quinolin-4-ylméthoxy)-benzènesulfonylamino]-propionamide.

33mg (0,06 mmoles) de palladium tétrakis triphénylphosphine puis 920mg (6,6 mmoles) de carbonate de potassium sont additionnés à une solution de 670mg (1,1 mmoles) de (S)-N-allyloxy-2-(4-isobutyryl-pipérazin-1-yl)-3-[4-(2-méthyl-quinolin-4-ylméthoxy)-benzènesulfonylamino]-propionamide dans 15ml de méthanol puis le milieu réactionnel est chauffé à reflux pendant 8h. Après addition d'acétate d'éthyle, le milieu réactionnel est lavé par une solution aqueuse d'hydrogénocarbonate de sodium saturée. La phase organique est ensuite lavée par de l'eau, séchée sur sulfate de magnésium, filtrée et concentrée. Le produit brut est repris dans 6ml d'éthanol et 12 ml d'eau puis chauffé à 80°C jusqu'à solubilisation. Après refroidissement, la cristallisation est amorcée par évaporation d'un minimum d'éthanol. 120mg de produit sont obtenus par filtration et purifiés par chromatographie sur couche mince de silice préparative, élués avec un mélange dichlorométhane / méthanol 97/3. 20mg (3%) de (S)-N-hydroxy-2-(4-isobutyryl-pipérazin-1-yl)-3-[4-(2-méthyl-quinolin-4-ylméthoxy)-benzènesulfonylamino]-propionamide sont finalement obtenus sous forme d'un solide beige.
RMN ¹H (δ, DMSO) : 0,84 (s, 3H); 0,85 (s, 3H); 2,30-2,44 (m, 2H); 2,52 (m, 2H); 2,67 (s, 3H); 2,77 (m, 1 H); 2,85 (m, 2H); 2,95 (m, 1 H); 3,35 (m, 4H); 5,71 (s, 2H); 7,33 (d, J=8,9Hz, 2H); 7,43 (m, 1 H); 7,55-7,62 (m, 2H); 7,72-7,82 (m, 3H); 7,98 (d, J=8,4Hz, 1 H); 8,11 (d, J=8,2Hz, 1 H); 8,96 (m, 1H); 10,67 (m, 1 H).

### Exemple 24 : (S)-N-hydroxy-2-[4-(2-méthyl-propane-1-sulfonyl)-pipérazin-1-I]-3-[4-(2-méthyl-quinolin-4-ylméthoxy)-benzènesulfonylamino]-propionamide.

### 24.1 : (S)-3-tert-butoxycarbonylamino-2-[4-(2-méthyl-propane-1-sulfonyl)-pipérazin-1-yl]-propanoate de méthyle.

479mg (3,0 mmoles) de chlorure de 2-méthyl-propane-1-sulfonyle sont additionnés à une solution de 800mg (2,8 mmoles) de (S)-3-tert-butoxycarbonylamino-2-pipérazin-1-yl-propanoate de méthyle (préparé comme décrit dans l'exemple 23.1), 775µl (5,5 mmoles) de triéthylamine dans 8ml de dichlorométhane, préalablement refroidie à 0°C. Le milieu réactionnel est agité à température ambiante pendant 18h puis de l'eau est additionnée et le milieu est extrait au dichlorométhane. La phase organique est lavée à l'eau, séchée sur sulfate de magnésium, filtrée et concentrée. Le résidu obtenu est purifié par chromatographie sur gel de silice élué avec un mélange heptane / acétate d'éthyle 5/5. 785mg (71%) de (S)-3-tert-butoxycarbonylamino-2-[4-(2-méthyl-propane-1-sulfonyl)-pipérazin-1-yl]-propanoate de méthyle sont obtenus sous forme d'une huile incolore.

### 24.2 : dichlorhydrate de (S)-3-amino-2-[4-(2-méthyl-propane-1-sulfonyl)-pipérazin-1-yl]-propanoate de méthyle.

De manière analogue à l'exemple 3.3, à partir de 785mg (1,9 mmoles) de (S)-3-tert-butoxycarbonylamino-2-[4-(2-méthyl-propane-1-sulfonyl)-pipérazin-1-yl]-propanoate de méthyle, 621 mg (85%) de dichlorhydrate de (S)-3-amino-2-[4-(2-méthyl-propane-1-sulfonyl)-pipérazin-1-yl]-propanoate de méthyle sont obtenus sous forme d'un solide.

### 24.3 : (S)-2-[4-(2-méthyl-propane-1-sulfonyl)-pipérazin-1-yl]-3-[4-(2-méthyl-quinolin-4-ylméthoxy)-benzènesulfonylamino]-propanoate de méthyle.

De manière analogue à l'exemple 3.6, à partir de 621 mg (1,6 mmoles) de dichlorhydrate de (S)-3-amino-2-[4-(2-méthyl-propane-1-sulfonyl)-pipérazin-1-yl]-propanoate de méthyle et de 876mg (2,3 mmoles) de chlorhydrate de chlorure de 4-(2-méthyl-quinolin-4-ylméthoxy)-benzènesulfonyle (préparé comme décrit dans l'exemple 17.2), 643mg (64%) de (S)-2-[4-(2-méthyl-propane-1-sulfonyl)-pipérazin-1-yl]-3-[4-(2-méthyl-quinolin-4-ylméthoxy)-benzènesulfonylamino]-propanoate de méthyle sont obtenus sous forme d'une huile.

### 24.4 : acide (S)-2-[4-(2-méthyl-propane-1-sulfonyl)-pipérazin-1-yl]-3-[4-(2-méthyl-quinolin-4-ylméthoxy)-benzènesulfonylaminol-propanoique.

De manière analogue à l'exemple 3.7, à partir de 643mg (1,0 mmole) de (S)-2-[4-(2-méthyl-propane-1-sulfonyl)-pipérazin-1-yl]-3-[4-(2-méthyl-quinolin-4-ylméthoxy)-benzènesulfonylamino]-propanoate de méthyle, 395mg (63%) d' acide (S)-2-[4-(2-méthyl-propane-1-sulfonyl)-pipérazin-1-yl]-3-[4-(2-méthyl-quinolin-4-ylméthoxy)-benzènesulfonylamino]-propanoique sont obtenus sous forme d'un solide blanc.

### 24.5: (S)-N-hydroxy-2-[4-(2-méthyl-propane-1-sulfonyl)-pipérazin-1-yl]-3-[4-(2-méthyl-quinolin-4-ylméthoxy)-benzènesulfonylamino]-propionamide.

De manière analogue à l'exemple 3.8, à partir de 390mg (0,6 mmoles) d'acide (S)-2-[4-(2-méthyl-propane-1-sulfonyl)-pipérazin-1-yl]-3-[4-(2-méthyl-quinolin-4-ylméthoxy)-benzènesulfonylamino]-propanoique, 12mg (3%) de (S)-N-hydroxy-2-[4-(2-méthyl-propane-1-sulfonyl)-pipérazin-1-yl]-3-[4-(2-méthyl-quinolin-4-ylméthoxy)-benzènesulfonylamino]-propionamide sont obtenus sous forme d'un solide blanc.
RMN ¹H (δ, DMSO) : 1,01 (d, J=6,7Hz, 6H); 2,05 (m, 1 H); 2,49 (m, 4H); 2,67 (s, 3H); 2,86 (d, J=6,6Hz, 2H); 3,00-3,10 (m, 6H); 3,31 (m, 1H); 5,71 (s, 2H); 7,34 (d, J=8,9Hz, 2H); 7,52 (m, 1H); 7,57 (m, 2H); 7,76-7,80 (m, 3H); 7,98 (d, J=8,2Hz, 1H); 8,10 (m, 1 H); 8,93 (s, 1H); 10,66 (s, 1H).

### Exemple 25 : (S)-N-hydroxy-2-(4-méthanesulfonyl-pipérazin-1-yl)-3-[4-(2-trifluorométhyl-pyrazolo[1,5-α]pyridin-3-ylméthoxy)-benzènesulfonylamino]-propionamide.

### 25.1 : 2-trifluorométhyl-pyrazolo[1,5-α]pyridine-3-carboxylate d'éthyle.

Une solution de 2,1g (38 mmoles) de KOH dans 20ml d'eau puis 6,7g (30 mmoles) d'iodure de 1-amino-pyridinium sont ajoutés à une solution de 2,5g (15 mmoles) de 4,4,4-trifluoro-but-2-ynoate d'éthyle dans 25ml de dichlorométhane. Après agitation à température ambiante pendant 5h, de l'eau est additionnée et le milieu réactionnel est extrait au dichlorométhane. La phase organique est lavée à l'eau, séchée sur sulfate de magnésium, filtrée et concentrée. Le résidu obtenu est purifié par chromatographie sur gel de silice élué avec un mélange heptane / acétate d'éthyle 8/2. 2,8g (73%) de 2-trifluorométhyl-pyrazolo[1,5-a]pyridine-3-carboxylate d'éthyle sont obtenus sous forme d'un solide jaune.

### 25.2: (2-trifluorométhyl-pyrazolo[1,5-α]pyridin-3-yl)-méthanol.

Une solution de 2,8g (11 mmoles) de 2-trifluorométhyl-pyrazolo[1,5-α]pyridine-3-carboxylate d'éthyle dans 50ml de tétrahydrofuranne est additionnée goutte à goutte à une suspension de 0,5g (12 mmoles) d'hydrure d'aluminium et de lithium dans 45ml de tétrahydrofuranne. Le milieu réactionnel est ensuite agité à 70°C pendant 3h. Après addition goutte à goutte de 2,5ml de méthanol puis de 1,8ml d'une solution aqueuse d'hydroxyde de sodium de concentration 2N, le milieu réactionnel est agité 20mn à température ambiante puis filtré. Le filtrat est séché sur sulfate de magnésium, filtré et concentré sous vide. 2,3g (100%) de (2-trifluorométhyl-pyrazolo[1,5-α]pyridin-3-yl)-méthanol sont obtenus sous forme d'un solide.

### 25.3 : (S)-2-(4-méthanesulfonyl-pipérazin-1-yl)-3-[4-(2-trifluorométhyl-pyrazolo[1,5-a]pyridin-3-ylméthoxy)-benzènesulfonylamino]-propanoate de méthyle.

De manière analogue à l'exemple 11.1, à partir de 800mg (1,9 mmoles) de (S)-3-(4-hydroxy-benzènesulfonylamino)-2-(4-méthanesulfonyl-pipérazin-1-yl)-propanoate de méthyle (préparé comme décrit en 5.1) et de 540mg (2,5 mmoles) de (2-trifluorométhyl-pyrazolo[1,5-α]pyridin-3-yl)-méthanol, 380mg (32%) de (S)-2-(4-méthanesulfonyl-pipérazin-1-yl)-3-[4-(2-trifluorométhyl-pyrazolo[1,5-a]pyridin-3-ylméthoxy)-benzènesulfonylamino]-propanoate de méthyle sont obtenus sous forme d'un solide blanc.

### 25.4 : acide (S)-2-(4-méthanesulfonyl-pipérazin-1-yl)-3-[4-(2-trifluorométhyl-pyrazolo[1,5-α]pyridin-3-ylméthoxy)-benzènesulfonylaminol-propanoique.

De manière analogue à l'exemple 3.7, à partir de 380mg (0,6 mmoles) de (S)-2-(4-méthanesulfonyl-pipérazin-1-yl)-3-[4-(2-trifluorométhyl-pyrazolo[1,5-α]pyridin-3-ylméthoxy)-benzènesulfonylamino]-propanoate de méthyle, 237mg (64%) d' acide (S)-2-(4-méthanesulfonyl-pipérazin-1-yl)-3-[4-(2-trifluorométhyl-pyrazolo[1,5-α]pyridin-3-ylméthoxy)-benzènesulfonylamino]-propanoique sont obtenus sous forme d'un solide blanc.

### 25.5: (S)-N-hydroxy-2-(4-méthanesulfonyl-pipérazin-1-yl)-3--[4-(2-trifluorométhyl-pyrazolo[1,5-α]pyridin-3-ylméthoxy)-benzènesulfonylamino]-propionamide.

De manière analogue à l'exemple 3.8, à partir de 230mg (0,4 mmoles) d'acide (S)-2-(4-méthanesulfonyl-pipérazin-1-yl)-3-[4-(2-trifluorométhyl-pyrazolo[1,5-α]pyridin-3-ylméthoxy)-benzènesulfonylamino]-propanoique, 9mg (4%) de (S)-N-hydroxy-2-(4-méthanesulfonyl-pipérazin-1-yl)-3-[4-(2-trifluorométhyl-pyrazolo[1,5-α]pyridin-3-ylméthoxy)-benzènesulfonylamino]-propionamide sous forme d'un solide blanc.
RMN ¹H (δ, DMSO) : 2,51-2,54 (m, 4H); 2,84 (s, 3H); 2,95 (m, 1H); 2,97-3,04 (m, 4H); 3,10 (m, 1H); 3,32 (m, 1 H); 5,45 (s, 2H); 7,20-7,25 (m, 3H); 7,49-7,51 (m, 2H); 7,76 (d, J=8,8Hz, 2H); 8,04 (m, 1 H); 8,87 (d, J=7Hz, 2H); 8,90 (m, 1 H).

### Exemple 26 : (S)-N-hydroxy-3-[4-(2-méthyl-quinolin-4-ylméthoxy)-benzènesulfonylamino]-2-[4-(propane-2-sulfonyl)-pipérazin-1-yl]-propionamide.

### 26.1 : (S)-3-tertbutoxycarbonylamino-2-[4-(propane-2-sulfonyl)-pipérazin-1-yl]-propanoate de méthyle

De manière analogue à l'exemple 20.2, à partir de 800mg (2,8 mmoles) de (S)-3-tertbutoxycarbonylamino-2-pipérazin-1-yl-propanoate de méthyle (préparé comme décrit dans l'exemple 23.1) et de 342µl (3,1 mmoles) de chlorure de propane-2-sulfonyle, 700mg (64%) de (S)-3-tertbutoxycarbonylamino-2-[4-(propane-2-sulfonyl)-pipérazin-1-yl]-propanoate de méthyle sont obtenus sous forme d'une huile.

### 26.2 : dichlorhydrate de (S)-3-amino-2-[4-(propane-2-sulfonyl)-pipérazin-1-yl]-propanoate de méthyle

De manière analogue à l'exemple 3.3, à partir de 700mg (1,8 mmoles) (S)-3-tertbutoxycarbonylamino-2-[4-(propane-2-sulfonyl)-pipérazin-1-yl]-propanoate de méthyle, 620mg (86%) du dichlorhydrate de (S)-3-amino-2-[4-(propane-2-sulfonyl)-pipérazin-1-yl]-propanoate de méthyle sont obtenus sous forme d'une huile.

### 26.3 : (S)-3-[4-(2-méthyl-quinolin-4-ylméthoxy)-benzènesulfonylamino]-2-[4-(propane-2-sulfonyl)-pipérazin-1-yl]-propanoate de méthyle

De manière analogue à l'exemple 17.6, à partir de 620mg (1,5 mmoles) de dichlorhydrate de (S)-3-amino-2-[4-(propane-2-sulfonyl)-pipérazin-1-yl]-propanoate de méthyle et de 830mg (2,1 mmoles) de chlorhydrate de chlorure de 4-(2-méthyl-quinolin-4-ylméthoxy)-benzènesulfonyle (préparé comme décrit dans l'exemple 17.2), 505mg (54%) de (S)-3-[4-(2-méthyl-quinolin-4-ylméthoxy)-benzènesulfonylamino]-2-[4-(propane-2-sulfonyl)-pipérazin-1-yl]-propanoate de méthyle sont obtenus sous forme d'un solide blanc.

### 26.4 : acide (S)-3-[4-(2-méthyl-quinolin-4-ylméthoxy)-benzènesulfonylamino]-2-[4-(propane-2-sulfonyl)-pipérazin-1-yl]-propanoique

De manière analogue à l'exemple 3.7, à partir de 505mg (0,8 mmoles) de (S)-3-[4-(2-méthyl-quinolin-4-ylméthoxy)-benzènesulfonylamino]-2-[4-(propane-2-sulfonyl)-pipérazin-1-yl]-propanoate de méthyle, 135mg (27%) d'acide (S)-3-[4-(2-méthyl-quinolin-4-ylméthoxy)-benzènesulfonylamino]-2-[4-(propane-2-sulfonyl)-pipérazin-1-yl]-propanoique sont obtenus sous forme d'un solide blanc.

### 26.5 : (S)-N-hydroxy-3-[4-(2-méthyl-quinolin-4-ylméthoxy)-benzènesulfonylamino]-2-[4-(propane-2-sulfonyl)-pipérazin-1-yl]-propionamide

De manière analogue à l'exemple 3.8, à partir de 135mg (0,2 mmoles) d'acide (S)-3-[4-(2-méthyl-quinolin-4-ylméthoxy)-benzènesulfonylamino]-2-[4-(propane-2-sulfonyl)-pipérazin-1-yl]-propanoique, 24mg (17%) de (S)-N-hydroxy-3-[4-(2-méthyl-quinolin-4-ylméthoxy)-benzènesulfonylamino]-2-[4-(propane-2-sulfonyl)-pipérazin-1-yl]-propionamide sont obtenus sous forme d'un solide blanc.
RMN ¹H (δ, DMSO) : 1,19 (d, J=6,8Hz, 6H); 2,45 (m, 4H); 2,68 (s, 3H); 2,80-2,90 (m, 1H); 2,95-3,15 (m, 6H); 3,29 (m, 1 H); 5,72 (s, 2H); 7,34 (d, J=8,9Hz, 2H); 7,52 (m, 1 H); 7,57 (m, 2H); 7,76-7,80 (m, 3H); 7,98 (d, J=8,2Hz, 1 H); 8,10 (d, J=8,1 Hz, 1 H); 8,93 (s, 1 H); 10,66 (s, 1 H).

### Exemple 27 : (S)-2-(4-benzyl-pipérazin-1-yl)-N-hydroxy -3-[4-(2-trifluorométhyl-pyrazolo[1,5-α]pyridin-3-ylméthoxy)-benzènesulfonylamino]-propionamide.

### 27.1 : chlorure de 4-hydroxy-benzènesulfonyle

Une solution de 7g (30 mmoles) du sel de sodium de l'acide 4-hydroxy-benzènesulfonique dihydrate dans 40ml de diméthylformamide est additionnée goutte à goutte à une solution de 15,5ml (181 mmoles) de chlorure d'oxalyle dans 120ml de dichlorométhane refroidie à -30°C. Le milieu réactionnel est lentement ramené à température ambiante puis agité à température ambiante pendant 18h. Après addition de 200ml de glace, le milieu réactionnel est extrait à l'acétate d'éthyle. La phase organique est lavée par de l'eau, par une solution aqueuse saturée de chlorure de sodium, séchée sur sulfate de magnésium, filtrée et concentrée. 6,2g (100%) de chlorure de 4-hydroxy-benzènesulfonyle sont obtenus sous forme d'une huile incolore.

### 27.2 : (S)-2-(4-benzyl-pipérazin-1-yl)-3-(4-hydroxy-benzènesulfonylamino)-propanoate de méthyle

De manière analogue à l'exemple 3.6, à partir de 5,8g (30 mmoles) de chlorure de 4-hydroxy-benzènesulfonyle et de 7,7g (20 mmoles) de trichlorhydrate de (S)-3-amino-2-(4-benzyl-pipérazin-1-yl)-propanoate de méthyle (préparé comme décrit dans l'exemple 17.5), 2,25g (27%) de (S)-2-(4-benzyl-pipérazin-1-yl)-3-(4-hydroxy-benzènesulfonylamino)-propanoate de méthyle sont obtenus sous forme d'un solide blanc.

### 27.3 : (S)-2-(4-benzyl-pipérazin-1-yl)-3-[4-(2-trifluorométhyl-pyrazolo[1,5-α]pyridin-3-ylméthoxy)-benzènesulfonylamino]-propanoate de méthyle

De manière analogue à l'exemple 11.1, à partir de 500mg (1,1 mmoles) de (S)-2-(4-benzyl-pipérazin-1-yl)-3-(4-hydroxy-benzènesulfonylamino)-propanoate de méthyle, et de 370mg (1,7 mmoles) de (2-trifluorométhyl-pyrazolo[1,5-α]pyridin-3-yl)-méthanol (préparé comme décrit dans l'exemple 25.2), 350mg (50%) de (S)-2-(4-benzyl-pipérazin-1-yl)-3-[4-(2-trifluorométhyl-pyrazolo[1,5-α]pyridin-3-ylméthoxy)-benzènesulfonylamino]-propanoate de méthyle sont obtenus sous forme d'une huile incolore.

### 27.4 : acide (S)-2-(4-benzyl-pipérazin-1-yl)-3-[4-(2-trifluorométhyl-pyrazolo[1,5-α]pyridin-3-ylméthoxy)-benzènesulfonylamino]-propanoïque

De manière analogue à l'exemple 3.7, à partir de 350mg (0,5 mmoles) de (S)-2-(4-benzyl-pipérazin-1-yl)-3-[4-(2-trifluorométhyl-pyrazolo[1,5-α]pyridin-3-ylméthoxy)-benzènesulfonylamino]- propanoate de méthyle, 165mg (48%) d' acide (S)-2-(4-benzyl-pipérazin-1-yl)-3-[4-(2-trifluorométhyl-pyrazolo[1,5-α]pyridin-3-ylméthoxy)-benzènesulfonylamino]-propanoïque sont obtenus sous forme d'un solide blanc.

### 27.5 : (S)-2-(4-benzyl-pipérazin-1-yl)-N-hydroxy-3-[4-(2-trifluorométhyl-pyrazolo[1,5-α]pyridin-3-ylméthoxy)-benzènesulfonylamino]-propionamide

De manière analogue à l'exemple 3.8, à partir de 165mg (0,3 mmoles) d'acide (S)-2-(4-benzyl-pipérazin-1-yl)-3-[4-(2-trifluorométhyl-pyrazolo[1,5-a]pyridin-3-ylméthoxy)-benzènesulfonylamino]-propanoique, 50mg (29%) de (S)-2-(4-benzyl-pipérazin-1-yl)-N-hydroxy-3-[4-(2-trifluorométhyl-pyrazolo[1,5-α]pyridin-3-ylméthoxy)-benzènesulfonylamino]-propionamide sont obtenus sous forme d'un solide blanc de point de fusion 138°C.
RMN ¹H (δ, DMSO) : 2,20 (m, 4H); 2,38 (m, 4H); 2,65-2,75 (m, 1 H); 2,86-2,98 (m, 2H); 3,35 (m, 2H); 5,37 (s, 2H); 7,10-7,25 (m, 8H); 7,35-7,44 (m, 2H); 7,68 (d, J=8,9Hz, 2H); 7,98 (d, J=9Hz, 1H); 8,81 (m, 2H); 10,52 (s, 1H).

### Exemple 28 : Test enzymatique d'inhibition de TACE.

### Description du test

Les produits sont solubilisés dans du DMSO à une concentration de 10 mM. Une dilution sérielle de raison 3 sur 10 points est effectuée de façon à avoir une gamme de concentration allant de 10 µM à 0,5 nM final.
L'enzyme TACE est une production interne (réalisée selon la publication « protein Eng Des Sel 2006, 19.,155-161 ») et, est ajouté de façon à avoir un signal équivalent à 6 fois le bruit de fond en 2h à 37°C. La réaction s'effectue en milieu tamponné Tris 50 mM, 4% de glycérol, pH7,4. Le substrat fluorescent est MCA-Pro-Leu-Ala-Val-(Dpa)-Arg-Ser-Ser-Arg-NH2 (R&D system référence :ES003). Le substrat est clivé par l'enzyme entre l'alanine et la valine libérant ainsi un peptide fluorescent (excitation : 320nm, émission : 420nm). Le substrat est utilisé à 40µM. la réaction est réalisée dans un volume final de 10µl (4µl inhibiteur, 4µl substrat, 2µl enzyme) dans une plaque de 384 puits low volume (Corning référence : 3676). La plaque est incubée 2h à température ambiante, puis lue en fluorescence au Pherastar (BMG labtech). L'IC₅₀ est déterminée en utilisant un logiciel de traitement mathématique (XLfit).

### Test des produits

| n° exemple | % inhibition TACE à 10 µM | IC50 - TACE (rM) |
|---|---|---|
| ex1 | 100 | 87 |
| ex2 | 100 | 32 |
| ex4 | 95 | 497 |
| ex5 | 99 | 21 |
| ex6 | 99 | 52 |
| ex8 | 100 | 127 |
| ex9 | 100 | 147 |
| ex10 | 93 | 47 |
| ex11 | 93 | 24 |
| ex13 | 96 | 108 |
| ex14 | 98 | 64 |
| ex16 | 96 | 168 |
| ex17 | 91 | 62 |
| ex18 | 90 | 67 |
| ex19 | 92 | 41 |
| ex21 | 97 | 63 |
| ex23 | 97 | 53 |
| ex24 | 98 | 86 |
| ex26 | 98 | 33 |

Sur la base des résultats obtenus dans le test enzymatique TACE décrit ci-dessus, les composés revendiqués dans la présente invention sont des inhibiteurs de l'enzyme TNF-alpha converting enzyme (TACE) et de ce fait peuvent être des principes actifs potentiels pour le traitement de pathologies pour lesquelles diminuer la production de TNF alpha serait d'un grand intérêt.

### Exemple 29 : test de sélectivité

### Principe du test :

Les molécules sont testées en dose réponse sur les enzymes suivants MMP1, MMP3, MMP9, ADAM9 et ADAM10 suivant le même protocole que celui décrit pour l'enzyme TACE dans l'exemple 28 mais avec des substrats différents (MMP R&D system référence :P126-990 et ADAM R&D system référence :ES003).
Les enzymes sont achetées chez Calbiochem

### Test des produits :

| | IC50 (nM) | | | | | |
|---|---|---|---|---|---|---|
| Exemple | MMP1 | MMP3 | MMP9 | ADAM9 | ADAM10 | TACE |
| **5** | 5100 | 3200 | >10000 | >10000 | >10000 | 21 |
| **18** | 670 | 849 | >10000 | 9254 | >10000 | 67 |
| **19** | 2303 | 1770 | >10000 | 3054 | >10000 | 41 |
| **20** | 3935 | 4775 | >10000 | >10000 | >10000 | 140 |
| **21** | 1166 | 887 | >10000 | >10000 | >10000 | 63 |
| **23** | 2221 | 1065 | >10000 | >10000 | >10000 | 53 |
| **24** | 2059 | 1878 | >10000 | >10000 | >10000 | 86 |
| **26** | 969 | 574 | >10000 | >10000 | >10000 | 16 |
| Apratastat | 145 | 10 | 82 | 85 | 71 | 5 |

Sur la base des résultats obtenus dans le test de sélectivité décrit ci-dessus, ces composés sont également très sélectifs de TACE par rapport aux autres ADAMs et MMPs, c'est-à-dire qu'ils présentent des IC₅₀ pour d'autres ADAMs ou MMPs au moins 10 fois supérieures à celle obtenue pour TACE, et plus avantageusement au moins 100 fois supérieures.
Or, dans la mesure où il est connu que l'inhibition non sélective de ces familles d'enzymes induit des effets secondaires indésirables observés *In Vivo,* l'inhibition sélective de TACE par rapport à ces autres enzymes devrait permettre de réduire des effets secondaires indésirables lors de l'administration de ces molécules pour le traitement de pathologies pour lesquelles diminuer la production de TNF alpha serait d'un grand intérêt.

### CARACTERES DE L'APPLICATION PARENT:

**1.** Composé de formule générale (I) suivante : 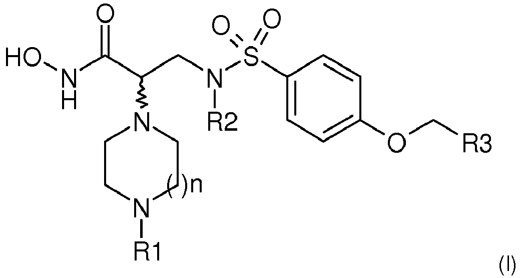 dans laquelle
   R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical-C(O)-R₄, un radical -SO₂-R₄, un radical C(O)OR4, R₄ ayant les significations données ci-après;
   R₂est un atome d'hydrogène ou un radical alkyle inférieur;
   R₃ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué, un radical hétérocyclique, un radical hétérocyclique substitué, un radical cycloalkyle, un radical cycloalkyle substitué, un radical hétéroaryle, un radical hétéroaryle substitué, un radical hétéroaralkyle, un radical hétéroaralkyle substitué;
   R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
   n peut prendre les valeurs de 0, 1, 2 ou 3;
   ainsi que les sels d'addition des composés de formule générale (I) avec un acide pharmaceutiquement acceptable, les sels d'addition des composés de formule générale (I) avec une base pharmaceutiquement acceptable et les énantiomères des composés de formule générale (I).
2. Sel d'addition avec un acide pharmaceutiquement acceptable d'un composé selon le caractère 1 caractérisé en ce que l'acide pharmaceutiquement acceptable est choisi dans la liste constituée par l'acide chlorhydrique, l'acide bromhydrique, l'acide sulfurique, l'acide nitrique, l'acide phosphorique, l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide methanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.
3. Sel d'addition avec une base pharmaceutiquement acceptable d'un composé selon le caractère 1 caractérisé en ce que la base pharmaceutiquement acceptable est choisie dans la liste constituée par l'hydroxyde de potassium, l'hydroxyde de sodium, l'hydroxyde de lithium, l'hydroxyde de calcium, la methylamine, l'ethylamine, l'éthanolamine, la propylamine, l'isopropylamine, les 4 isomères de la butylamine, la diméthylamine, diethylamine, la diethanolamine, la dipropylamine, la diisopropylamine, la di n-butylamine, la pyrrolidine, la piperidine, la morpholine, la diethanol phenylamine, la trimethylamine, la triethylamine, la tripropylamine, la quinuclidine, la pyridine, la quinoline, l'isoquinoline, la lysine, l'arginine et l'ornithine.
4. Composé selon l'une des caractères 1 à 3 caractérisé en ce que
   R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical - C(O)-R₄, un radical -SO₂-R₄, un radical C(O)OR4, R₄ ayant les significations données ci-après;
   R₂est un atome d'hydrogène ou un radical alkyle inférieur;
   R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
   R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
   n peut prendre les valeurs de 0, 1, 2;
   ainsi que leurs sels d'addition avec un acide pharmaceutiquement acceptable, leurs sels d'addition avec une base pharmaceutiquement acceptable et les énantiomères desdits composés.
5. Composé selon l'une des caractères 1 à 3 caractérisé en ce que
   R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical -C(O)-R₄, un radical -SO₂-R₄, R₄ ayant les significations données ci-après;
   R₂est un atome d'hydrogène ou un radical alkyle inférieur;
   R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
   R₄ est un radical alkyle, un radical alkyle substitué, un radical aryle, un radical aryle substitué, un radical aralkyle, un radical aralkyle substitué;
   n peut prendre les valeurs de 1 ou 2;
   ainsi que leurs sels d'addition avec un acide pharmaceutiquement acceptable, leurs sels d'addition avec une base pharmaceutiquement acceptable et les énantiomères desdits composés.
6. Composé selon l'une des caractères 1 à 3 caractérisé en ce que
   R₁ représente un radical alkyle, un radical alkyle substitué, un radical aralkyle, un radical aralkyle substitué, un radical -C(O)-R₄, un radical -SO₂-R₄, R₄ ayant les significations données ci-après; R₂est un atome d'hydrogène;
   R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
   R₄ est un radical alkyle, un radical alkyle substitué, un radical aryle, un radical aryle substitué, un radical aralkyle, un radical aralkyle substitué;
   n prend la valeur de 1 ;
   ainsi que leurs sels d'addition avec un acide pharmaceutiquement acceptable, leurs sels d'addition avec une base pharmaceutiquement acceptable et les énantiomères desdits composés.
7. Composé selon l'une des caractères 1 à 3 caractérisé en ce
   R₁ représente un radical alkyle, un radical alkyle substitué, un radical aralkyle, un radical aralkyle substitué, un radical -C(O)-R₄, un radical -SO₂-R₄, R₄ ayant les significations données ci-après;
   R₂ est un atome d'hydrogène;
   R₃ est un radical hétérocyclique, un radical hétérocyclique substitué, un radical hétéroaryle, un radical hétéroaryle substitué, un radical hétéroaralkyle, un radical hétéroaralkyle substitué;
   R₄ est un radical alkyle, un radical alkyle substitué, un radical aryle, un radical aryle substitué, un radical aralkyle, un radical aralkyle substitué;
   n prend la valeur de 1 ;
   ainsi que leurs sels d'addition avec un acide pharmaceutiquement acceptable, leurs sels d'addition avec une base pharmaceutiquement acceptable et les énantiomères desdits composés.
8. Composé selon l'une des caractères 1 à 3 caractérisé en ce que
   R₁ représente un radical alkyle, un radical alkyle substitué, un radical aralkyle, un radical aralkyle substitué, un radical -C(O)-R₄, un radical -SO₂-R₄, R₄ ayant les significations données ci-après;
   R₂est un atome d'hydrogène;
   R₃ est un radical hétéroaryle, un radical hétéroaryle substitué;
   R₄ est un radical alkyle, un radical alkyle substitué, un radical aryle, un radical aryle substitué, un radical aralkyle, un radical aralkyle substitué;
   n prend la valeur de 1 ;
   ainsi que leurs sels d'addition avec un acide pharmaceutiquement acceptable, leurs sels d'addition avec une base pharmaceutiquement acceptable et les énantiomères desdits composés.
9. Composé selon l'une des caractères 1 à 8 choisi dans la liste constituée par les composés suivants :
   1) 3-[(4-but-2-ynyloxy-benzènesulfonyl)-méthyl-amino]-N-hydroxy-2-(4-méthanesulfonyl-pipérazin-1-yl)-propionamide
   2) ((S)-3-(4-but-2-ynyloxy-benzènesulfonylamino)-N-hydroxy-2-(4-méthanesulfonyl-pipérazin-1-yl)-propionamide
   3) ((S)-3-(4-benzyloxy-benzènesulfonylamino)-N-hydroxy-2-(4-méthanesulfonyl-pipérazin-1-yl)-propionamide
   4) ((S)-3-[(4-benzyloxy-benzènesulfonyl)-méthyl-amino]-N-hydroxy-2-(4-méthanesulfonyl-pipérazin-1-yl)-propionamide
   5) ((S)-N-hydroxy-2-(4-méthanesulfonyl-pipérazin-1-yl)-3-[4-(2-méthyl-quinolin-4-ylméthoxy)-benzènesulfonylamino]-propionamide
   6) ((S)-N-hydroxy-2-(4-méthanesulfonyl-pipérazin-1-yl)-3-[4-(naphthalen-1-ylméthoxy)-benzènesulfonylamino]-propionamide
   7) ((S)-N-hydroxy-2-(4-méthanesulfonyl-pipérazin-1-yl)-3-(4-propoxy-benzènesulfonylamino)-propionamide
   8) ((S)-3-[4-(3-cyano-benzyloxy)-benzènesulfonylamino]-N-hydroxy-2-(4-méthanesulfonyl-pipérazin-1-yl)-propionamide
   9) ((S)-3-[4-(4-cyano-benzyloxy)-benzènesulfonylamino]-N-hydroxy-2-(4-méthanesulfonyl-pipérazin-1-yl)-propionamide
   10) 4-{(S)-1-hydroxycarbamoyl-2-[4-(2-méthyl-quinolin-4-ylméthoxy)-benzènesulfonylamino]-éthyl}-pipérazine-1-carboxylate de benzyle
   11) ((S)-N-hydroxy-2-(4-méthanesulfonyl-pipérazin-1-yl)-3-[4-(2-phényl-pyridin-4-ylméthoxy)-benzène sulfonylamino]-propionamide
   12) ((R)-N-hydroxy-2-(4-méthanesulfonyl-pipérazin-1-yl)-3-[4-(2-méthyl-quinolin-4-ylméthoxy)-benzène sulfonylamino]-propionamide
   13) ((S)-N-hydroxy-3-[4-(2-méthyl-quinolin-4-ylméthoxy)-benzènesulfonylamino]-2-pipérazin-1-yl-propionamide
   14) Chlorhydrate de (S)-N-hydroxy-2-(4-méthanesulfonyl-pipérazin-1-yl)-3-[4-(2-méthyl-quinolin-4-ylméthoxy)-benzène sulfonylamino]-propionamide
   15) Di trifluoro acétate de 3-{4-[(S)-2-hydroxycarbamoyl-2-(4-méthanesulfonyl-pipérazin-1-yl)-éthylsulfamoyl]-phénoxyméthyl}-2-méthyl-indole-1-carboxylate de terbutyle
   16) ((S)-N-hydroxy-2-(4-méthanesulfonyl-pipérazin-1-yl)-3-[4-(quinolin-4-ylméthoxy)-benzènesulfonylamino]-propionamide
   17) ((S)-2-(4-benzyl-pipérazin-1-yl)-N-hydroxy-3-[4-(2-méthyl-quinolin-4-ylméthoxy)-benzènesulfonylamino]-propionamide
   18) (S)-2-[4-(4-fluoro-benzyl)-pipérazin-1-yl]-N-hydroxy-3-[4-(2-méthyl-quinolin-4-ylméthoxy)-benzène sulfonylamino]-propionamide
   19) ((S)-2-(4-éthyl-pipérazin-1-yl)-N-hydroxy-3-[4-(2-méthyl-quinolin-4-ylméthoxy)-benzènesulfonylamino]-propionamide
   20) ((S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide
   21) ((S)-N-hydroxy-2-[4-(4-méthyl-benzyl)-pipérazin-1-yl]-3-[4-(2-méthyl-quinolin-4-ylméthoxy)-benzène sulfonylamino]-propionamide
   22) ((S)-3-[4-(benzo-isoxazol-3-ylméthoxy)-benzènesulfonylamino]-N-hydroxy-2-(4-méthanesulfonyl-pipérazin-1-yl)-propionamide
   23) ((S)-N-hydroxy-2-(4-isobutyryl-pipérazin-1-yl)-3-[4-(2-méthyl-quinolin-4-ylméthoxy)-benzènesulfonylamino]-propionamide
   24) ((S)-N-hydroxy-2-[4-(2-méthyl-propane-1-sulfonyl)-pipérazin-1-yl]-3-[4-(2-méthyl-quinolin-4-ylméthoxy)-benzènesulfonylamino]-propionamide
   25) ((S)-N-hydroxy-2-(4-méthanesulfonyl-pipérazin-1-yl)-3-[4-(2-trifluorométhyl-pyrazolo[1,5-a]pyridin-3-ylméthoxy)-benzènesulfonylamino]-propionamide
   26) ((S)-N-hydroxy-3-[4-(2-méthyl-quinolin-4-ylméthoxy)-benzènesulfonylamino]-2-[4-(propane-2-sulfonyl)-pipérazin-1-yl]-propionamide
   27) ((S)-2-(4-benzyl-pipérazin-1-yl)-N-hydroxy -3-[4-(2-trifluorométhyl-pyrazolo[1,5-a]pyridin-3-ylméthoxy)-benzènesulfonylamino]-propionamide
   28) ((S)-2-(4-acétyl-pipérazin-1-yl)-N-hydroxy-3-[4-(2-méthyl-quinolin-4-ylméthoxy)-benzènesulfonylamino]-propionamide
   29) ((S)-N-hydroxy-2-(4-méthanesulfonyl-pipérazin-1-yl)-3-{propyl-[4-(quinolin-4-ylméthoxy)-benzènesulfonyl]-amino}-propionamide
   30) ((S)-2-(4-benzènesulfonyl-pipérazin-1-yl)-N-hydroxy-3-[4-(pyrazolo[1,5-a]pyridin-3-ylméthoxy)-benzènesulfonylamino]-propionamide
   31) ((S)-2-(4-benzyl-pipérazin-1-yl)-N-hydroxy-3-[4-(1-méthyl-pipéridin-4-ylméthoxy)-benzènesulfonylamino]-propionamide
   32) ((S)-2-[4-(4-fluoro-benzoyl)-pipérazin-1-yl]-N-hydroxy-3-[4-(3-m-tolyl-propoxy)-benzènesulfonylamino]-propionamide
   33) ((S)-N-hydroxy-3-[4-(2-méthyl-naphthalen-1-ylméthoxy)-benzènesulfonylamino]-2-(4-propionyl-pipérazin-1-yl)-propionamide
   34) ((S)-N-hydroxy-3-[4-(4-méthyl-pentyloxy)-benzènesulfonylamino]-2-(4-phénylacétyl-pipérazin-1-yl)-propionamide
   35) ((S)-N-hydroxy-2-(4-méthanesulfonyl-pipérazin-1-yl)-3-[4-(2-méthyl-pyridin-4-ylméthoxy)-benzènesulfonylamino]-propionamide
   36) ((S)-2-(3-acétyl-imidazolidin-1-yl)-N-hydroxy-3-[4-(2-méthyl-quinolin-4-ylméthoxy)-benzènesulfonylamino]-propionamide
   37) ((S)-3-[4-(3,5-diméthyl-benzyloxy)-benzènesulfonylamino]-N-hydroxy-2-imidazolidin-1-yl-propionamide
   38) (S)-N-hydroxy-2-(4-méthanesulfonyl-[1,4]-diazepan-1-yl)-3-[4-(2-méthyl-quinolin-4-ylméthoxy)-benzènesulfonylamino]-propionamide
   39) ((S)-2-(4-benzyl-[1,4]-diazepan-1-yl)-N-hydroxy-3-[4-(2-méthyl-quinolin-4-ylméthoxy)-benzènesulfonylamino]-propionamide
   40) ((S)-2-[1,4]-diazocan-1-yl-N-hydroxy-3-[4-(2-méthyl-quinolin-4-ylméthoxy)-benzènesulfonylamino]-propionamide
   41) ((S)-N-hydroxy-3-[4-(2-méthyl-benzofuran-3-ylméthoxy)-benzènesulfonylamino]-2-[4-(propane-2-sulfonyl)-pipérazin-1-yl]-propionamide et
   42) ((S)-2-(4-benzyl-pipérazin-1-yl)-N-hydroxy-3-[4-(2-isopropyl-1 H-indol-3-ylméthoxy)-benzènesulfonylamino]-propionamide.
10. Composé selon l'une des caractères 1 à 9 en tant que médicament.
11. Composé selon l'une des caractères 1 à 9 pour le traitement de pathologies ou de désordres liés à une libération de TNFα.
12. Composé selon l'une des caractères 1 à 9 en tant qu'inhibiteur de la production de TNFα.
13. Composé selon l'une des caractères 1 à 9 en tant qu'inhibiteurs de l'enzyme TNFα-converting enzyme (TACE).
14. Composé selon l'une des caractères 1 à 9 pour le traitement ou l'amélioration des désordres ou pathologies médiés par TACE.
15. Composé selon l'une des caractères 1 à 9 pour le traitement de maladies ou désordres impliquant une production de TNFα choisies parmi le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'attaque ischémique, le rejet de greffe, le cancer, l'athérosclérose, l'obésité, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD) et l'inflammation occulaire.
16. Composé selon l'une des caractères 1 à 9 pour le traitement des maladies inflammatoires de la peau, du psoriasis, de la dermatite atopique et du rhumatisme psoriasique.
17. Composé selon l'une des caractères 1 à 9 pour le traitement des pathologies neurologiques à caractère inflammatoire impliquant une production de TNFα choisies parmi la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'oedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale.

## Revendications

1. Composé de formule générale (I) suivante : 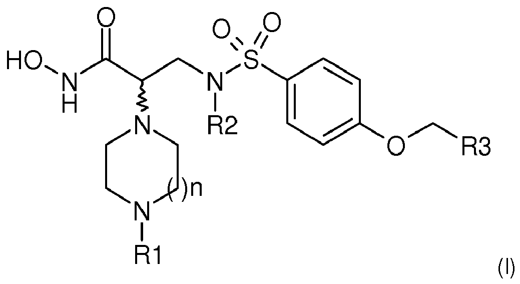 dans laquelle
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical - C(O)-R₄, un radical -SO₂-R₄, un radical C(O)OR4, R₄ ayant les significations données ci-après;
R₂est un atome d'hydrogène ou un radical alkyle inférieur;
R₃ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué, un radical hétérocyclique, un radical hétérocyclique substitué, un radical cycloalkyle, un radical cycloalkyle substitué, un radical hétéroaryle, un radical hétéroaryle substitué, un radical hétéroaralkyle, un radical hétéroaralkyle substitué;
R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
n peut prendre les valeurs de 0, 1, 2 ou 3;
ainsi que les sels d'addition des composés de formule générale (I) avec un acide pharmaceutiquement acceptable, les sels d'addition des composés de formule générale (I) avec une base pharmaceutiquement acceptable et les énantiomères des composés de formule générale (I).

2. Sel d'addition avec un acide pharmaceutiquement acceptable d'un composé selon la revendication 1 **caractérisé en ce que** l'acide pharmaceutiquement acceptable est choisi dans la liste constituée par l'acide chlorhydrique, l'acide bromhydrique, l'acide sulfurique, l'acide nitrique, l'acide phosphorique, l'acide acétique, l'acide trifluoroacétique, l'acide trichloroacétique, l'acide propionique, l'acide glycolique, l'acide pyruvique, l'acide succinique, l'acide benzoïque, l'acide cinnamique, l'acide mandélique, l'acide methanesulfonique, l'acide para-toluène sulfonique, l'acide salicylique, l'acide picrique, l'acide citrique, l'acide oxalique, l'acide tartrique, l'acide malonique, l'acide maléique, l'acide camphorsulfonique et l'acide fumarique.

3. Sel d'addition avec une base pharmaceutiquement acceptable d'un composé selon la revendication 1 **caractérisé en ce que** la base pharmaceutiquement acceptable est choisie dans la liste constituée par l'hydroxyde de potassium, l'hydroxyde de sodium, l'hydroxyde de lithium, l'hydroxyde de calcium, la methylamine, l'ethylamine, l'éthanolamine, la propylamine, l'isopropylamine, les 4 isomères de la butylamine, la diméthylamine, diethylamine, la diethanolamine, la dipropylamine, la diisopropylamine, la di n-butylamine, la pyrrolidine, la piperidine, la morpholine, la diethanol phenylamine, la trimethylamine, la triethylamine, la tripropylamine, la quinuclidine, la pyridine, la quinoline, l'isoquinoline, la lysine, l'arginine et l'ornithine.

4. Composé selon l'une des revendications 1 à 3 **caractérisé en ce que**
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical hétéroaralkyle, un radical hétéroaralkyle substitué, un radical - C(O)-R₄, un radical -SO₂-R₄, un radical C(O)OR4, R₄ ayant les significations données ci-après;
R₂est un atome d'hydrogène ou un radical alkyle inférieur;
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical alkenyle, un radical alkenyle substitué, un radical alkynyle, un radical alkynyle substitué, un radical aryle, un radical aryle substitué, un radical aralkyle, un radical aralkyle substitué;
n peut prendre les valeurs de 0, 1, 2;
ainsi que leurs sels d'addition avec un acide pharmaceutiquement acceptable, leurs sels d'addition avec une base pharmaceutiquement acceptable et les énantiomères desdits composés.

5. Composé selon l'une des revendications 1 à 3 **caractérisé en ce que**
R₁ représente un hydrogène, un radical alkyle, un radical alkyle substitué, un radical alkenyle, un radical alkenyle substitué, un radical alkynyle, un radical alkynyle substitué, un radical aralkyle, un radical aralkyle substitué, un radical -C(O)-R₄, un radical -SO₂-R₄, R₄ ayant les significations données ci-après;
R₂est un atome d'hydrogène ou un radical alkyle inférieur;
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical aryle, un radical aryle substitué, un radical aralkyle, un radical aralkyle substitué;
n peut prendre les valeurs de 1 ou 2;
ainsi que leurs sels d'addition avec un acide pharmaceutiquement acceptable, leurs sels d'addition avec une base pharmaceutiquement acceptable et les énantiomères desdits composés.

6. Composé selon l'une des revendications 1 à 3 **caractérisé en ce que**
R₁ représente un radical alkyle, un radical alkyle substitué, un radical aralkyle, un radical aralkyle substitué, un radical -C(O)-R₄, un radical -SO₂-R₄, R₄ ayant les significations données ci-après; R₂est un atome d'hydrogène;
R₃ est un radical aryle, un radical aryle substitué, un radical aralkyle, un radical aralkyle substitué, un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

7. Composé selon l'une des revendications 1 à 3 caractérisé en ce
R₁ représente un radical alkyle, un radical alkyle substitué, un radical aralkyle, un radical aralkyle substitué, un radical -C(O)-R₄, un radical -SO₂-R₄, R₄ ayant les significations données ci-après;
R₂ est un atome d'hydrogène;
R₃ est un radical hétérocyclique, un radical hétérocyclique substitué, un radical hétéroaryle, un radical hétéroaryle substitué, un radical hétéroaralkyle, un radical hétéroaralkyle substitué;
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

8. Composé selon l'une des revendications 1 à 3 **caractérisé en ce que**
R₁ représente un radical alkyle, un radical alkyle substitué, un radical aralkyle, un radical aralkyle substitué, un radical -C(O)-R₄, un radical -SO₂-R₄, R₄ ayant les significations données ci-après; R₂est un atome d'hydrogène;
R₃ est un radical hétéroaryle, un radical hétéroaryle substitué;
R₄ est un radical alkyle, un radical alkyle substitué, un radical aryle, un radical aryle substitué, un radical aralkyle, un radical aralkyle substitué;
n prend la valeur de 1 ;
ainsi que leurs sels d'addition avec un acide pharmaceutiquement acceptable, leurs sels d'addition avec une base pharmaceutiquement acceptable et les énantiomères desdits composés.

9. Composé selon l'une des revendications 1 à 8 choisi dans la liste constituée par les composés suivants:
1) 3-[(4-but-2-ynyloxy-benzènesulfonyl)-méthyl-amino]-N-hydroxy-2-(4-méthanesulfonyl-pipérazin-1-yl)-propionamide
2) ((S)-3-(4-but-2-ynyloxy-benzènesulfonylamino)-N-hydroxy-2-(4-méthanesulfonyl-pipérazin-1-yl)-propionamide
3) ((S)-3-(4-benzyloxy-benzènesulfonylamino)-N-hydroxy-2-(4-méthanesulfonyl-pipérazin-1-yl)-propionamide
4) ((S)-3-[(4-benzyloxy-benzènesulfonyl)-méthyl-amino]-N-hydroxy-2-(4-méthanesulfonyl-pipérazin-1-yl)-propionamide
5) ((S)-N-hydroxy-2-(4-méthanesulfonyl-pipérazin-1-yl)-3-[4-(2-méthyl-quinolin-4-ylméthoxy)-benzènesulfonylamino]-propionamide
6) ((S)-N-hydroxy-2-(4-méthanesulfonyl-pipérazin-1-yl)-3-[4-(naphthalen-1-ylméthoxy)-benzènesulfonylamino]-propionamide
7) ((S)-N-hydroxy-2-(4-méthanesulfonyl-pipérazin-1-yl)-3-(4-propoxy-benzènesulfonylamino)-propionamide
8) ((S)-3-[4-(3-cyano-benzyloxy)-benzènesulfonylamino]-N-hydroxy-2-(4-méthanesulfonyl-pipérazin-1-yl)-propionamide
9) ((S)-3-[4-(4-cyano-benzyloxy)-benzènesulfonylamino]-N-hydroxy-2-(4-méthanesulfonyl-pipérazin-1-yl)-propionamide
10) 4-{(S)-1-hydroxycarbamoyl-2-[4-(2-méthyl-quinolin-4-ylméthoxy)-benzènesulfonylamino]-éthyl}-pipérazine-1-carboxylate de benzyle
11) ((S)-N-hydroxy-2-(4-méthanesulfonyl-pipérazin-1-yl)-3-[4-(2-phényl-pyridin-4-ylméthoxy)-benzène sulfonylamino]-propionamide
12) ((R)-N-hydroxy-2-(4-méthanesulfonyl-pipérazin-1-yl)-3-[4-(2-méthyl-quinolin-4-ylméthoxy)-benzène sulfonylamino]-propionamide
13) ((S)-N-hydroxy-3-[4-(2-méthyl-quinolin-4-ylméthoxy)-benzènesulfonylamino]-2-pipérazin-1-yl-propionamide
14) Chlorhydrate de (S)-N-hydroxy-2-(4-méthanesulfonyl-pipérazin-1-yl)-3-[4-(2-méthyl-quinolin-4-ylméthoxy)-benzène sulfonylamino]-propionamide
15) Di trifluoro acétate de 3-{4-[(S)-2-hydroxycarbamoyl-2-(4-méthanesulfonyl-pipérazin-1-yl)-éthylsulfamoyl]-phénoxyméthyl}-2-méthyl-indole-1-carboxylate de terbutyle
16) ((S)-N-hydroxy-2-(4-méthanesulfonyl-pipérazin-1-yl)-3-[4-(quinolin-4-ylméthoxy)-benzènesulfonylamino]-propionamide
17) ((S)-2-(4-benzyl-pipérazin-1-yl)-N-hydroxy-3-[4-(2-méthyl-quinolin-4-ylméthoxy)-benzènesulfonylamino]-propionamide
18) (S)-2-[4-(4-fluoro-benzyl)-pipérazin-1-yl]-N-hydroxy-3-[4-(2-méthyl-quinolin-4-ylméthoxy)-benzène sulfonylamino]-propionamide
19) ((S)-2-(4-éthyl-pipérazin-1-yl)-N-hydroxy-3-[4-(2-méthyl-quinolin-4-ylméthoxy)-benzènesulfonylamino]-propionamide
20) ((S)-N-hydroxy-3-[4-(2-méthyl-quinolin-4-ylméthoxy)-benzènesulfonylamino]-2-[4-(4-trifluorométhyl-benzyl)-pipérazin-1-yl]-propionamide
21) ((S)-N-hydroxy-2-[4-(4-méthyl-benzyl)-pipérazin-1-yl]-3-[4-(2-méthyl-quinolin-4-ylméthoxy)-benzène sulfonylamino]-propionamide
22) ((S)-3-[4-(benzo-isoxazol-3-ylméthoxy)-benzènesulfonylamino]-N-hydroxy-2-(4-méthanesulfonyl-pipérazin-1-yl)-propionamide
23) ((S)-N-hydroxy-2-(4-isobutyryl-pipérazin-1-yl)-3-[4-(2-méthyl-quinolin-4-ylméthoxy)-benzènesulfonylamino]-propionamide
24) ((S)-N-hydroxy-2-[4-(2-méthyl-propane-1-sulfonyl)-pipérazin-1-yl]-3-[4-(2-méthyl-quinolin-4-ylméthoxy)-benzènesulfonylamino]-propionamide
25) ((S)-N-hydroxy-2-(4-méthanesulfonyl-pipérazin-1-yl)-3-[4-(2-trifluorométhyl-pyrazolo[1,5-a]pyridin-3-ylméthoxy)-benzènesulfonylamino]-propionamide
26) ((S)-N-hydroxy-3-[4-(2-méthyl-quinolin-4-ylméthoxy)-benzènesulfonylamino]-2-[4-(propane-2-sulfonyl)-pipérazin-1-yl]-propionamide
27) ((S)-2-(4-benzyl-pipérazin-1-yl)-N-hydroxy -3-[4-(2-trifluorométhyl-pyrazolo[1,5-a]pyridin-3-ylméthoxy)-benzènesulfonylamino]-propionamide
28) ((S)-2-(4-acétyl-pipérazin-1-yl)-N-hydroxy-3-[4-(2-méthyl-quinolin-4-ylméthoxy)-benzènesulfonylamino]-propionamide
29) ((S)-N-hydroxy-2-(4-méthanesulfonyl-pipérazin-1-yl)-3-{propyl-[4-(quinolin-4-ylméthoxy)-benzènesulfonyl]-amino}-propionamide
30) ((S)-2-(4-benzènesulfonyl-pipérazin-1-yl)-N-hydroxy-3-[4-(pyrazolo[1,5-a]pyridin-3-ylméthoxy)-benzènesulfonylamino]-propionamide
31) ((S)-2-(4-benzyl-pipérazin-1-yl)-N-hydroxy-3-[4-(1-méthyl-pipéridin-4-ylméthoxy)-benzènesulfonylamino]-propionamide
32) ((S)-2-[4-(4-fluoro-benzoyl)-pipérazin-1-yl]-N-hydroxy-3-[4-(3-m-tolyl-propoxy)-benzènesulfonylamino]-propionamide
33) ((S)-N-hydroxy-3-[4-(2-méthyl-naphthalen-1-ylméthoxy)-benzènesulfonylamino]-2-(4-propionyl-pipérazin-1-yl)-propionamide
34) ((S)-N-hydroxy-3-[4-(4-méthyl-pentyloxy)-benzènesulfonylamino]-2-(4-phénylacétyl-pipérazin-1-yl)-propionamide
35) ((S)-N-hydroxy-2-(4-méthanesulfonyl-pipérazin-1-yl)-3-[4-(2-méthyl-pyridin-4-ylméthoxy)-benzènesulfonylamino]-propionamide
36) ((S)-2-(3-acétyl-imidazolidin-1-yl)-N-hydroxy-3-[4-(2-méthyl-quinolin-4-ylméthoxy)-benzènesulfonylamino]-propionamide
37) ((S)-3-[4-(3,5-diméthyl-benzyloxy)-benzènesulfonylamino]-N-hydroxy-2-imidazolidin-1-yl-propionamide
38) (S)-N-hydroxy-2-(4-méthanesulfonyl-[1,4]-diazepan-1-yl)-3-[4-(2-méthyl-quinolin-4-ylméthoxy)-benzènesulfonylamino]-propionamide
39) ((S)-2-(4-benzyl-[1,4]-diazepan-1-yl)-N-hydroxy-3-[4-(2-méthyl-quinolin-4-ylméthoxy)-benzènesulfonylamino]-propionamide
40) ((S)-2-[1,4]-diazocan-1-yl-N-hydroxy-3-[4-(2-méthyl-quinolin-4-ylméthoxy)-benzènesulfonylamino]-propionamide
41) ((S)-N-hydroxy-3-[4-(2-méthyl-benzofuran-3-ylméthoxy)-benzènesulfonylamino]-2-[4-(propane-2-sulfonyl)-pipérazin-1-yl]-propionamide et
42) ((S)-2-(4-benzyl-pipérazin-1-yl)-N-hydroxy-3-[4-(2-isopropyl-1 H-indol-3-ylméthoxy)-benzènesulfonylamino]-propionamide.

10. Composé selon l'une des revendications 1 à 9 en tant que médicament.

11. Composé selon l'une des revendications 1 à 9 pour son utilisation dans le traitement de maladies inflammatoires ou désordres impliquant une production de TNFα choisies parmi le choc septique, le choc hémodynamique, la malaria, les maladies intestinales inflammatoires (IBD) comme la maladie de Crohn et les colites ulcératives, les maladies osseuses inflammatoires, les infections mycobactériennes, la méningite, les maladies fibrotiques, les maladies cardiaques, l'attaque ischémique, le rejet de greffe, le cancer, l'athérosclérose, l'obésité, les maladies impliquant des phénomènes d'angiogénèse, les maladies auto-immunes, l'ostéoarthrite, l'arthrite rhumatoïde, la spondylarthrite ankylosante, l'arthrite chronique juvénile, la sclérose multiple, le HIV, le diabète melitus non insulino dépendant, les maladies allergiques, l'asthme, la maladie d'obstruction pulmonaire chronique (COPD) et l'inflammation occulaire.

12. Composé selon l'une des revendications 1 à 9 pour son utilisation dans le traitement des maladies inflammatoires de la peau, du psoriasis, de la dermatite atopique et du rhumatisme psoriasique.

13. Composé selon l'une des revendications 1 à 9 pour son utilisation dans le traitement des pathologies neurologiques à caractère inflammatoire impliquant une production de TNFα choisies parmi la maladie d'Alzheimer, la maladie de Parkinson, les désordres parkinsoniens, la sclérose amyotrophique latérale, les maladies autoimmunes du système nerveux, les maladies autonomiques du système nerveux, les douleurs dorsales, l'oedème cérébral, les désordres cérébrovasculaires, la démence, les maladies autoimmunes de démyelination des fibres nerveuses du système nerveux, les neuropathies diabétiques, l'encéphalite, l'encéphalomyélite, l'épilepsie, le syndrome chronique de fatigue, l'artérite des cellules géantes, le syndrome Guillain-Barre, les maux de tête, la sclérose multiple, la neuralgie, les maladies du système nerveux périphérique, les polyneuropathies, la polyradiculoneuropathie, la radiculopathie, les paralysies respiratoires, les maladies des cordes spinales, le syndrome de Tourette, la vasculite du système nerveux centreux, les maladies d'Huntington et l'attaque cérébrale.

14. Composition pharmaceutique **caractérisée par le fait qu'**elle comprend, dans un support pharmaceutiquement acceptable et compatible avec le mode d'administration retenu pour cette composition, au moins un composé selon l'une des revendications 1 à 9, ledit composé pouvant également se présenter sous une de ses formes énantiomériques ou sous la forme d'un de ses sels pharmaceutiquement acceptable.
